# FASCICULE DE BREVET EUROPEEN

(11) **EP 0 708 774 B1**
(45) Date de publication et mention de la délivrance du brevet: **17.03.1999**
(21) Numéro de dépôt: 94922904.1
(22) Date de dépôt: 11.07.1994
(51) Int. Cl.: C07D 487/04, C07D 487/10, A61K 31/495

(54) **DERIVES D'IMIDAZO[1,2-a]PYRAZIN-4-ONE ANTAGONISTES DES RECEPTEURS AMPA ET NMDA**
IMIDAZO[1,2-a]PYRAZIN-4-ON-DERIVATE ANTAGONISTEN DER AMPA UND NMDA REZEPTOREN
IMIDAZO[1,2-a]PYRAZIN-4-ONE DERIVATIVES ANTAGONISTS OF AMPA AND NMDA RECEPTORS

(30) Priorité: 16.07.1993 FR 9308754
(43) Date de publication de la demande: 01.05.1996
(73) Titulaire: RHONE-POULENC RORER S.A., 92160 Antony (FR)
(72) Inventeur: ALOUP, Jean-Claude, F-94290 Villeneuve-le-Roi (FR); AUDIAU, François, F-94220 Charenton-le-Pont (FR); DAMOUR, Dominique, F-75014 Paris (FR); GENEVOIS-BORELLA, Arielle, F-94320 Thiais (FR); JIMONET, Patrick, F-78450 Villepreux (FR); MIGNANI, Serge, F-92290 Chatenay-Malabry (FR); RIBEILL, Yves, F-91360 Villemoisson-sur-Orge (FR)
(74) Mandataire: Savina, Jacques
(86) Numéro de dépôt international: FR9400866
(87) Numéro de publication internationale: WO9502601

(56) Documents cités:
- EP-A- 0 518 530
- WO-A-92/11245
- WO-A-94/07893

## Description

La présente invention concerne les composés de formule : 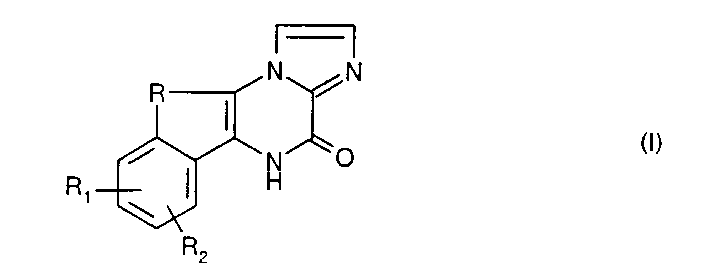 leurs sels, leur préparation et les médicaments les contenant.

Dans la formule (I),
soit R représente un radical C=R₃, C(R₄)R₅ ou CH-R₆,
- R₁ et R₂, identiques ou différents, représentent des atomes d'hydrogène ou d'halogène ou des radicaux alkyle, alcoxy, amino, acylamino, -NH-CO-NH-Ar, -N=CH-N(alk)alk', nitro, cyano, phényle, imidazolyle ou SO₃H,
- R₃ représente un atome d'oxygène ou un radical NOH, NO-alk-COOX ou CH-R₇,
- R₄ représente un radical alkyle, -alk-Het ou -alk-Ar,
- R₅ représente un radical alkyle (1-11C en chaîne droite ou ramifiée), -alk-Het ou -alk-Ar,
ou bien R₄ et R₅ forment ensemble avec l'atome de carbone auquel ils sont rattachés un radical cycloalkyle,
- R₆ représente un radical hydroxy, alkyle (1-11C en chaîne droite ou ramifiée), -NR₈R₉, -alk-OH, -alk-NR₈R₉, -alk-Ar ou -alk-Het,
- R₇ représente un radical hydroxy, alkyle, phényle, -alk-Ar, -alk-Het, -NR₁₀R₁₁ ou un hétérocycle mono ou polycyclique saturé ou insaturé contenant 4 à 9 atomes de carbone et un ou plusieurs hétéroatomes (O, S, N),
- R₈ et R₉, identiques ou différents, représentent chacun un radical alkyle, ou bien R₈ représente un atome d'hydrogène et R₉ représente un atome d'hydrogène ou un radical alkyle, -COR₁₂, -CSR₃₀ ou -SO₂R₁₃,
- R₁₀ et R₁₁, identiques ou différents, représentent chacun un radical alkyle ou cycloalkyle,
- R₁₂ représente un radical alkyle, cycloalkyle, phényle, -COO-alk, -CH₂-COOX, -CH₂-NH₂, -NH-alk, -NH₂, -NH-Ar ou -NH-Het,
- R₁₃ représente un radical alkyle ou phényle,
- R₃₀ représente un radical -NH-alk, -NH-Ar, -NH₂ ou -NH-Het,
- alk représente un radical alkyle ou alkylène,
- alk' représente un radical alkyle,
- X représente un atome d'hydrogène ou un radical alkyle,
- Ar représente un radical phényle et
- Het représente un hétérocycle mono ou polycyclique saturé ou insaturé contenant 4 à 9 atomes de carbone et un ou plusieurs hétéroatomes (O, S, N);
soit R représente un radical CH-R₆ et R₆ représente un radical 2-imidazolylméthyle et R₁ et R₂ représentent chacun un atome d'hydrogène.

Sauf mention contraire, dans les définitions qui précédent et celles qui suivent, les radicaux et portions alkyle, alkylène contiennent 1 à 4 atomes de carbone en chaîne droite ou ramifiée, les portions acyle contiennent 2 à 4 atomes de carbone, les radicaux cycloalkyle contiennent 3 à 6 atomes de carbone et les atomes d'halogène sont choisis parmi le fluor, le chlore, le brome et l'iode.

Les hétérocycles mentionnés dans les définitions précédentes sont de préférence les cycles pyridyle, furyle, quinolyle, pyrazinyle et pipéridyle.

Les composés de formule (I) pour lesquels R₃ représente un radical NOH, NO-alk-COOX ou CH-R₇ présentent des formes isomères (E et Z). Ces isomères et leurs mélanges font partie de l'invention.

Les énantiomères et diastéréoisomères des composés de formule (I) pour lesquels R représente un radical C(R₄)R₅ dans lequel R₄ est différent de R₅ ou CH-R₆ font également partie de l'invention.

Les composés de formule (I) pour lesquels R représente un radical C=R₃ dans lequel R₃ représente un atome d'oxygène peuvent être préparés par hydrolyse des composés de formule (I) correspondants pour lesquels R représente un radical C=R₃ et R₃ représente un radical NOH.

Cette réaction s'effectue généralement au moyen d'un acide, en milieu aqueux, à la température d'ébullition du milieu réactionnel. Comme acide, on utilise de préférence l'acide chlorhydrique.

Les composés de formule (I) pour lesquels R représente un radical C=R₃ et R₃ représente un radical NOH peuvent être préparés par action d'un nitrite d'alkyle sur un dérivé de formule : 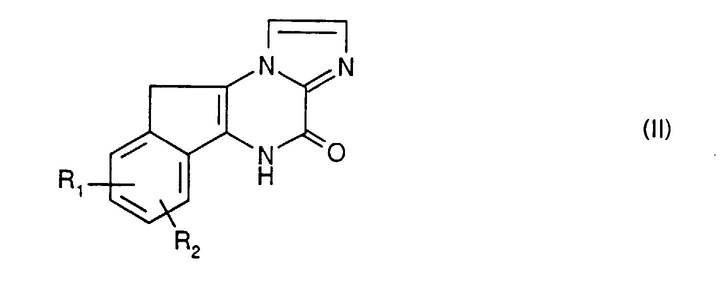 dans laquelle R₁ et R₂ ont les mêmes significations que dans la formule (I).

Cette réaction s'effectue de préférence au sein d'un solvant inerte tel que le diméthylsulfoxyde, en présence d'un hydrure de métal alcalin tel que l'hydrure de sodium, à une température voisine de 20°C. De préférence, on utilise le nitrite d'isoamyle.

Les dérivés de formule (Il) peuvent être préparés par désalkylation et désalification des dérivés de formule : 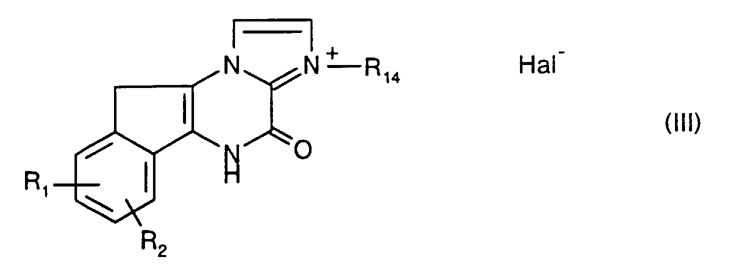 dans laquelle R₁ et R₂ ont les mêmes significations que dans la formule (I), R₁₄ représente un radical alkyle et Hal représente un atome d'halogène et, de préférence, un atome de brome.

Cette réaction s'effectue, de préférence, en présence d'imidazole, à une température comprise entre 100 et 200°C.

Les dérivés de formule (III) peuvent être obtenus par action des dérivés de formule : 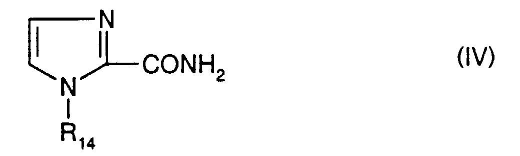 dans laquelle R₁₄ a les mêmes significations que dans la formule (III), sur une 2-halogènoindanone de formule : 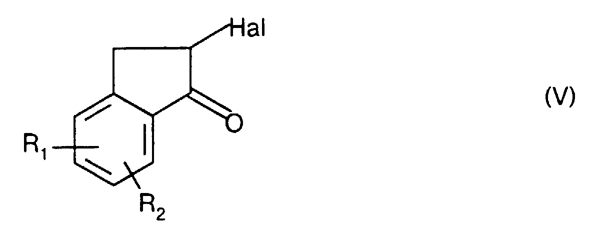 dans laquelle R₁ et R₂ ont les mêmes significations que dans la formule (I) et Hal représente un atome d'halogène.

Cette réaction s'effectue généralement au sein d'un solvant inerte tel que le diméthylformamide, à une température comprise entre 50 et 150°C et, de préférence à 115°C.

Les dérivés de formule (IV) peuvent être obtenus par adaptation ou application de la méthode décrite par D. D. DAVEY, J. Org. Chem., 52, 4379 (1987).

Les 2-halogénoindanones de formule (V) peuvent être obtenues par application ou adaptation de la méthode décrite par M. OLIVIER et coll., Bull. Soc. Chim. France, 3092 (1973) et dans le brevet allemand 2 640 358.

Les dérivés de formule (Il) peuvent également être obtenus par cyclisation d'un dérivé de formule : 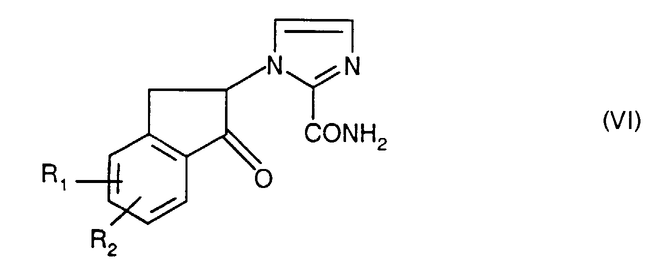 dans laquelle R₁ et R₂ ont les mêmes significations que dans la formule (I).

Cette cyclisation s'effectue généralement au moyen d'un acide tel que l'acide chlorhydrique en solution aqueuse ou l'acide acétique, à une température comprise entre 20°C et la température d'ébullition du milieu réactionnel.

Les dérivés de formule (VI) peuvent être obtenus par action d'ammoniac sur un dérivé de formule : 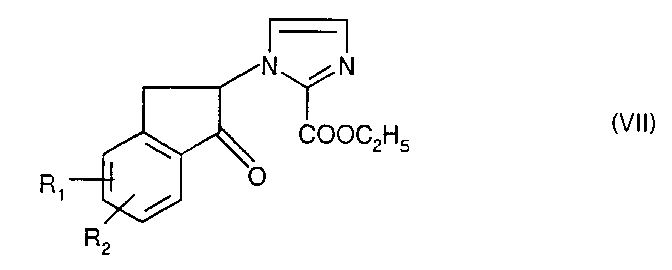 dans laquelle R₁ et R₂ ont les mêmes significations que dans la formule (I).

Cette réaction s'effectue généralement au sein d'un solvant inerte tel qu'un alcool, à une température comprise entre 20°C et la température d'ébullition du milieu réactionnel.

Les dérivés de formule (VII) peuvent être obtenus par condensation d'imidazole-2-carboxylate d'éthyle sur un dérivé de formule (V) dans laquelle R₁ et R₂ ont les mêmes significations que dans la formule (I) et Hal représente un atome d'halogène et, de préférence, un atome de brome.

Cette réaction s'effectue, soit par fusion du milieu réactionnel, soit au sein d'un solvant inerte tel qu'un alcool (méthanol, éthanol par exemple), un hydrocarbure aromatique tel que le toluène ou le diméthylformamide, éventuellement en présence d'une base telle qu'un hydrure de métal alcalin (hydrure de sodium par exemple), à une température comprise entre 20°C et la température d'ébullition du milieu réactionnel.

L'imidazole-2-carboxylate d'éthyle peut être obtenu selon la méthode décrite dans le brevet US 3600399.

Les composés de formule (I) pour lesquels R représente un radical C=R₃ et R₃ représente un radical NO-alk-COOX peuvent être préparés par action d'un composé de formule (I) correspondant pour lequel R représente un radical C=R₃ et R₃ représente un radical NOH sur un halogénure Hal-alk-COOX pour lequel Hal représente un atome d'halogène, alk représente un radical alkyle et X représente un radical alkyle, suivie éventuellement de la libération de la fonction carboxy par action de l'acide trifluoroacétique.

Cette réaction s'effectue de préférence en présence d'une base telle qu'un hydrure de métal alcalin comme l'hydrure de sodium, au sein d'un solvant inerte tel que le diméthylsulfoxyde, à une température voisine de 20°C. Le traitement avec l'acide trifluoroacétique s'effectue à une température comprise entre 5°C et la température d'ébullition du milieu réactionnel.

Les composés de formule (I) pour lesquels R représente un radical C=R₃ et R₃ représente un radical CH-R₇ dans lequel R₇ représente un radical hydroxy peuvent être préparés par hydrolyse des composés de formule (I) correspondants pour lesquels R₇ représente un radical -NR₁₀R₁₁.

Cette réaction s'effectue de préférence au moyen d'un acide tel que l'acide chlorhydrique, en milieu aqueux, à une température comprise entre 20 et 40°C.

Les composés de formule (I) pour lesquels R représente un radical C=R₃, R₃ représente un radical CH-R₇ et R₇ représente un radical -NR₁₀R₁₁ peuvent être préparés par action d'un dérivé de formule (II) dans laquelle R₁ et R₂ ont les mêmes significations que dans la formule (I) sur un dérivé de formule : 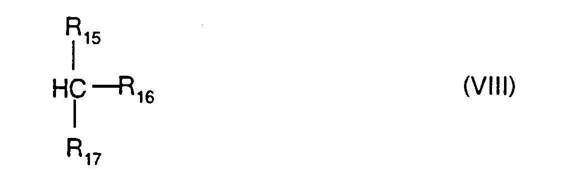 dans laquelle soit R₁₅ et R₁₇, identiques ou différents, représentent chacun un radical -NR₁₀R₁₁ et R₁₆ représente un radical alcoxy tel que tert-butoxy, soit R₁₅, R₁₆ et R₁₇, identiques, représentent chacun un radical -NR₁₀R₁₁.

Cette réaction s'effectue généralement au sein d'un solvant inerte tel que le diméthylformamide, à une température comprise entre 20 et 40°C.

Les dérivés de formule (VIII) peuvent être obtenus par application ou adaptation de la méthode décrite par H. BREDERECK, Liebigs Ann. Chem., 762, 62 (1972).

Les composés de formule (I) pour lesquels R représente un radical C=R₃, R₃ représente un radical CH-R₇ et R₇ représente un radical alkyle, phényle, -alk-Het, -alk-Ar ou un hétérocycle peuvent être préparés par action d'un dérivé de formule (Il) dans laquelle R₁ et R₂ ont les mêmes significations que dans la formule (I) sur un aldéhyde de formule :

OHC-R₁₈ (IX)

dans laquelle R₁₈ représente un radical alkyle, phényle, -alk-Het, -alk-Ar ou Het.

Cette réaction s'effectue généralement soit au sein d'un solvant inerte tel que le diméthylformamide, le 1,2-diméthoxyéthane, un alcool (méthanol, éthanol par exemple) ou un mélange de ces solvants, en présence d'une base telle que la soude, la potasse, une base organique forte telle que le 1,8-diazabicyclol[5,4,0]undec-7-ène, à une température comprise entre 20 et 100°C soit au sein du diméthylsulfoxyde, en présence d'un hydrure alcalin tel que l'hydrure de sodium, à une température voisine de 20°C soit en présence de bromure de tétrabutylammonium et d'une base telle qu'un hydroxyde de métal alcalin (soude, potasse par exemple), au sein du diméthylsulfoxyde, à une température comprise entre 20°C et la température d'ébullition du milieu réactionnel.

Les dérivés de formule OHC-R₁₈ sont commercialisés ou peuvent être obtenus par application ou adaptation des méthodes décrites par H. RUTNER et coll., J. Org. Chem., 28, 1898 (1963). Ils peuvent être aussi obtenus (a) par oxydation des alcools correspondants (à l'aide de K₂Cr₂O₇, en milieu sulfurique; de CrO₃ au sein de la pyridine ou de MnO₂ au sein d'un solvant chloré (dichlorométhane par exemple), à une température voisine de 20°C ou à l'aide du diméthylsulfoxyde et de ClCO-COCl par adaptation ou application de la méthode décrite par D. SWERN et coll., J. Org. Chem., 44, 4148 (1979)); (b) par réduction des acides carboxyliques correspondants (à l'aidé de l'hydrure de lithium-aluminium ou de AlH₃, dans un solvant inerte tel que le tétrahydrofuranne, à une température comprise entre 0 et 25°C); (c) par réduction des esters correspondants (à l'aide d'hydrure de diisobutylaluminium, au sein d'un solvant inerte tel que le toluène, à une température comprise entre -70°C et 25°C ou d'hydrure de lithium-aluminium, au sein d'un solvant inerte tel que le tétrahydrofuranne, à une température comprise entre 0 et 25°C).

Les alcools correspondants HO-alk-Het ou HO-alk-Ar sont commercialisés ou peuvent être obtenus à partir des composés organométalliques correspondants par application ou adaptation des méthodes décrites par N.S. NARASIMHAN et coll., Tetrahedron Lett., 22 (29), 2797 (1981); L. ESTEL et coll., J. Het. Chem., 26, 105 (1989); N.S. NARASIMHAN et coll., Synthesis, 957 (1983) et F. MARSAIS et coll., J. Heterocyclic Chem., 25, 81 (1988). De préférence, on fait réagir l'organolithien ou l'organomagnésien de l'hétérocycle ou du benzène sur le formol, un aldéhyde, une cétone, un époxyde ou Hal-alk-OP où P est un groupe protecteur (méthyléther, tétrahydropyranyléther, benzyléther ou triéthylsilyléther par exemple) puis libération de la fonction alcool par application ou adaptation des méthodes décrites par W. GREENE et coll., Protecting Groups in Organic Synthesis, second edition, 1991, John Wiley and Sons.

Les alcools correspondants HO-alk-Het ou HO-alk-Ar peuvent également être obtenus par réduction des acides carboxyliques ou des esters correspondants, au moyen d'hydrure de lithium-aluminium, au sein d'un solvant inerte tel que le tétrahydrofuranne ou le diéthyléther, à la température d'ébullition du milieu réactionnel.

Les alcools HO-(alk)ₙ-Het peuvent aussi être obtenus par application ou adaptation de la méthode décrite par J.Th. MEYER et coll., Helv. Chem. Acta, 65, 1868 (1982) à partir des dérivés Hal-(alk)ₙ₋₁-Het qui sont eux-mêmes obtenus par action d'un agent d'halogénation (dérivé halogéné du phosphore ou chlorure de thionyle) sur un dérivé HO-(alk)ₙ₋₁-Het correspondant, éventuellement au sein d'un solvant inerte tel que le dichlorométhane, à une température comprise entre 20 et 40°C.

Les acides carboxyliques correspondants HOOC-Het, HOOC-alk-Het, HOOC-alk-Ar sont commercialisés ou peuvent être obtenus à partir des hétérocycles correspondants et du chlorobenzène, bromobenzène ou iodobenzène par application ou adaptation des méthodes décrites par L. ESTEL et coll., J. Heterocyclic Chem., 26, 105 (1989); N.S. NARASIMHAN et coll., Synthesis, 957 (1983); A. TURCK et coll., Synthesis, 881 (1988); A.J. CLARKE et coll., Tetrahedron Lett, 27, 2373 (1974); A.R. KATRITZKY et coll., Org. Perp. Procedure Int., 20 (6), 585 (1988); N. FURUKAWA et coll., Tetrahedron Let., 28 (47), 5845 (1987); H.W. GSCHWEND et coll., Organic Reactions, 26, 1 (1979) et V. SNIECKUS, Chem. Rev., 90, 879 (1990). De préférence, on prépare le dérivé organométallique correspondant de l'hétérocycle ou du benzène (organolithien, organomagnésien par exemple), on le fait réagir soit sur du CO₂ soit sur un dérivé Hal-alk-COOalk dans lequel Hal représente un atome d'halogène et alk représente un radical alkyle et éventuellement on hydrolyse le produit ainsi obtenu. Cette hydrolyse s'effectue par toute méthode d'hydrolyse connue de l'homme du métier et qui ne touche pas au reste de la molécule. De préférence, on opère soit au moyen d'un acide tel que l'acide chlorhydrique, au sein de l'acide acétique, à une température comprise entre 20 et 50°C, soit au moyen d'acide trifluoroacétique, à une température comprise entre 5°C et la température d'ébullition du milieu réactionnel, soit en présence d'un hydroxyde de métal alcalin (soude, potasse, baryte par exemple), au sein d'un alcool tel que le méthanol ou l'éthanol, à une température comprise entre 20°C et la température d'ébullition du milieu réactionnel. Les esters correspondants sont commercialisés ou peuvent être obtenus à partir des acides par action d'un acide organique tel que l'acide chlorhydrique ou l'acide sulfurique, au sein de l'alcool servant également d'agent d'estérification, à la température d'ébullition du milieu réactionnel. Les dérivés Hal-alk-COOalk sont commercialisés ou préparés par action de Hal-alk-Hal dans lequel Hal représente un atome d'halogène et alk un radical alkyle sur un cyanure alcalin tel que le cyanure de sodium ou de potassium au sein d'un mélange eau-alcool, à une température comprise entre 0°C et la température d'ébullition du milieu réactionnel, suivie de l'action d'un acide tel que l'acide chlorhydrique, en présence d'un alcool, à une température comprise entre 0°C et la température d'ébullition du milieu réactionnel.

Les composés de formule (I) pour lesquels R représente un radical C(R₄)R₅, R₄ représente un radical alkyle, -alk-Het ou -alk-Ar et R₅ est identique à R₄ peuvent être préparés par action d'un dérivé de formule (II) dans laquelle R₁ et R₂ ont les mêmes significations que dans la formule (I) sur un halogénure de formule :

Hal-R₁₉ (X)

dans laquelle R₁₉ représente un radical alkyle, -alk-Het ou -alk-Ar.

Cette réaction s'effectue de préférence au sein d'un solvant inerte tel que le diméthylsulfoxyde, le diméthylformamide, le tétrahydrofuranne, le dioxanne, en présence d'une base tel qu'un hydroxyde de métal alcalin (soude, potasse par exemple), éventuellement en présence de bromure de tétrabutylammonium au sein du diméthylsulfoxyde ou en présence d'un hydrure de métal alcalin (hydrure de sodium par exemple), à une température comprise entre 20°C et la température d'ébullition du milieu réactionnel.

Les halogénures Hal-R₁₉ sont commercialisés ou peuvent être obtenus à partir des alcools correspondants par application ou adaptation des méthodes décrites par R.C. LAROCK, "Comprehensive Organic Transformations", Ed. VCH, page 353 (1989).

Les composés de formule (I) pour lesquels R représente un radical C(R₄)R₅, R₄ représente un radical alkyle, -alk-Het ou -alk-Ar et R₅ représente un radical alkyle (1-11C en chaîne droite ou ramifiée), -alk-Het ou -alk-Ar, peuvent être préparés par action d'un dérivé de formule : 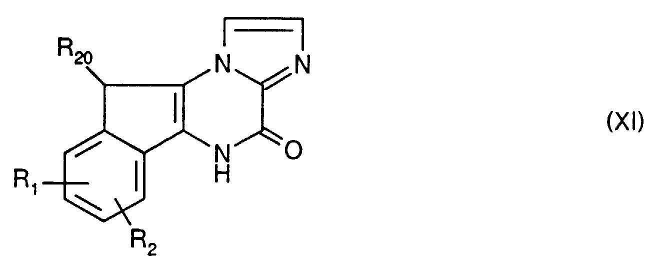 dans laquelle R₁ et R₂ ont les mêmes significations que dans la formule (I) et R₂₀ représente un radical alkyle, -alk-Het ou -alk-Ar ou le dérivé silylé de ce dérivé sur un halogénure de formule :

Hal-R₂₁ (XII)

dans laquelle R₂₁ représente un radical alkyle (1-11C en chaîne droite ou ramifiée), -alk-Het ou -alk-Ar.

Cette réaction s'effectue de préférence au sein d'un solvant inerte tel que le diméthylsulfoxyde, le diméthylformamide, le tétrahydrofuranne, le dioxanne, en présence d'une base tel qu'un hydroxyde de métal alcalin (soude, potasse par exemple), éventuellement en présence de bromure de tétrabutylammonium au sein du diméthylsulfoxyde ou en présence d'un hydrure de métal alcalin (hydrure de sodium par exemple), à une température comprise entre 20°C et la température d'ébullition du milieu réactionnel. Lorsque l'on utilise le dérivé silylé, on prépare ce dernier par action du triméthylchlorosilane sur le dérivé de formule (XI), de préférence au sein d'un solvant inerte tel que le diméthylformamide, en présence d'un hydrure de métal alcalin tel que l'hydrure de sodium, à une température comprise entre 5 et 30°C et l'on fait réagir le dérivé de formule (XII) dans ce milieu, à une température comprise entre 5 et 30°C.

Les composés de formule (I) pour lesquels R représente un radical C(R₄)R₅, R₄ représente un radical -alk-Het dans lequel alk est un radical éthyle et Het est un radical 4-pyridyl ou 2-pyridyl et R₅ représente un radical alkyle (1-11C en chaîne droite ou ramifiée), -alk-Het ou -alk-Ar peuvent être également préparés par action d'un dérivé de formule (XI) dans laquelle R₁ et R₂ ont les mêmes significations que dans la formule (I) et R₂₀ représente un radical alkyle (1-11C en chaîne droite ou ramifiée), -alk-Het ou -alk-Ar sous sa forme silylée, sur la 2- ou 4-vinylpyridine.

Le dérivé silylé est généralement obtenu par action de triméthylchlorosilane sur le dérivé de formule (XI), au sein d'un solvant inerte tel que le diméthylformamide, en présence d'un excès (4 à 5 équivalents environ) d'un hydrure de métal alcalin tel que l'hydrure de sodium, à une température comprise entre 5 et 30°C et l'on fait réagir le vinyl dans ce milieu à la même température.

Les composés de formule (I) pour lesquels R représente un radical C(R₄)R₅ et R₄ et R₅ forment avec l'atome de carbone auquel ils sont rattachés un radical cycloalkyle peuvent être préparés par action d'un dérivé de formule (II) dans laquelle R₁ et R₂ ont les mêmes significations que dans la formule (I) sur un dérivé de formule :

Hal-alk-Hal (XIII)

dans laquelle Hal représente un atome d'halogène et alk représente un radical alkyle (2-5C).

Cette réaction s'effectue généralement en présence de bromure de tétrabutylammonium et d'une base telle qu'un hydroxyde de métal alcalin (soude, potasse par exemple) au sein du diméthylsulfoxyde, à une température comprise entre 20°C et la température d'ébullition du milieu réactionnel.

Les dérivés de formule (XIII) sont commercialisés ou peuvent être obtenus à partir des dialcools correspondants par application ou adaptation des méthodes décrites par C. LAROCK, "Comprehensive Organic Transformations", Ed. VHC, page 353 (1989).

Les composés de formule (I) pour lesquels R représente un radical CH-R₆ dans lequel R₆ représente un radical hydroxy peuvent être préparés par réduction des composés de formule (I) correspondants pour lesquels R représente un radical C=R₃ et R₃ représente un atome d'oxygène.

Cette réaction s'effectue de préférence au sein d'un solvant inerte tel qu'un alcool (méthanol, éthanol par exemple), en présence de borohydrure de sodium, à une température comprise entre 15 et 40°C.

Les composés de formule (I) pour lesquels R représente un radical CH-R₆ dans lequel R₆ représente un radical alkyle (2-11C), -alk-Ar, -alk-Het ou 2-imidazolylméthyle peuvent être préparés par hydrogénation d'un dérivé de formule : 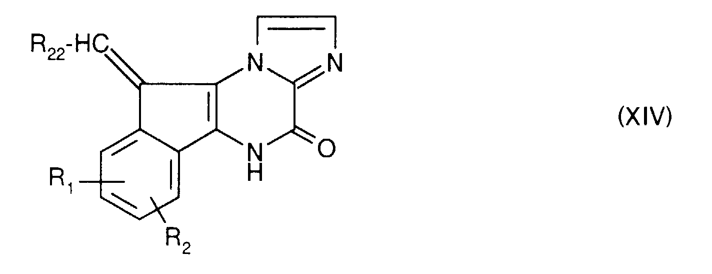 dans laquelle R₁ et R₂ ont les mêmes significations que dans la formule (I), R₂₂ représente un radical 2-imidazolyle, alkyle en chaîne droite ou ramifiée contenant 1 à 10 atomes de carbone, phényle, -alk-Ar, -alk-Het dans lesquels la portion alkyle est en chaîne droite ou ramifiée et contient 1 à 3 atomes de carbone ou un hétérocycle mono ou polycyclique saturé ou insaturé contenant 4 à 9 atomes de carbone et un ou plusieurs hétéroatomes (O, S, N).

Cette réduction s'effectue de préférence au moyen d'hydrogène, sous une pression de 1 à 20 bar, au sein d'un solvant inerte tel que le diméthylformamide, l'acide acétique, l'acétate d'éthyle, un alcool (méthanol, éthanol par exemple) ou un mélange de ces solvants, en présence d'un catalyseur d'hydrogénation tel que le charbon palladié, l'hydroxyde de palladium ou le palladium (N. RICO et coll., Nouveau Journal de Chimie, 10, 1, 25 (1986)), à une température comprise entre 20°C et 60°C ou par adaptation de la méthode décrite par L.M. STRAWN et coll., J. Med. Chem., 32, 2104 (1989) qui consiste à faire réagir le dérivé à réduire sur le sulfate d'hydroxylamine et H₂NOSO₃H, en milieu aqueux, à un pH compris entre 6 et 7, à une température de 10°C.

Les dérivés de formule (XIV) pour lesquels R₂₂ représente un radical alkyle en chaîne droite ou ramifiée, contenant 5 à 10 atomes de carbone ou 2-imidazolyle peuvent être préparés comme décrit précédemment pour leurs homologues inférieurs (composés de formule (I) pour lesquels R représente un radical C=R₃, R₃ représente un radical -CH-R₇ et R₇ représente un radical alkyle).

Les composés de formule (I) pour lesquels R représente un radical CH-R₆ dans lequel R₆ représente un radical méthyle peuvent être préparés par réduction des composés de formule (I) correspondants pour lesquels R représente un radical C=R₃, R₃ représente un radical CH-R₇ et R₇ représente un radical hydroxy ou -NR₁₀R₁₁.

Cette réduction s'effectue dans les conditions décrites précédemment pour la réduction des composés de formule (XIV) pour lesquels R₂₂ représente un radical alkyle.

Les composés de formule (I) pour lesquels R représente un radical CH-R₆ et R₆ représente un radical -alk(1C)-OH peuvent être préparés par réduction des composés de formule (I) correspondants pour lesquels R représente un radical C=R₃, R₃ représente un radical CH-R₇ et R₇ représente un radical hydroxy.

Cette réduction s'effectue généralement à l'aide d'un agent réducteur tel que le borohydrure de sodium, au sein d'un solvant inerte tel qu'un alcool (méthanol, éthanol par exemple), à une température voisine de 20°C.

Les composés de formule (I) pour lesquels R représente un radical CH-R₆ et R₆ représente un radical -alk(2-4C)-OH peuvent être préparés par réduction des dérivés de formule (XIV) correspondants pour lesquels R₁ et R₂ ont les mêmes significations que dans la formule (I) et R₂₂ représente un radical -alk(1-3C)-O-CH₂-Ar.

Cette réduction s'effectue dans les conditions décrites précédemment pour la réduction des composés de formule (XIV) pour lesquels R₂₂ représente un radical alkyle.

Les dérivés de formule (XIV) pour lesquels R₁ et R₂ ont les mêmes significations que dans la formule (I) et R₂₂ représente un radical -alk(1-3C)-O-CH₂-Ar peuvent être obtenus par action d'un dérivé de formule (II) sur un aldéhyde OHC-alk(1-3C)-O-CH₂-Ar.

Cette réaction s'effectue dans les mêmes conditions que celles mentionnées précédemment pour la réaction des dérivés de formule (II) avec les aldéhydes de formule (IX).

Les aldéhydes OHC-alk(1-3C)-O-CH₂-Ar peuvent être obtenus par application ou adaptation des méthodes décrites par P. SCHORIGIN et coll., Chem. Ber., 68, 838 (1935) et A. GAIFFE et coll., C. R. Acad. Sc. Paris, Ser. C, 266, 1379 (1968).

Les composés de formule (I) pour lesquels R représente un radical CH-R₆ et R₆ représente un radical -alk(2-4C)-OH peuvent également être préparés par action de (COCl)₂ sur un dérivé de formule (XI) correspondant pour lequel R₁ et R₂ ont les mêmes significations que dans la formule (I) et R₂₀ représente un radical -alk-COOH suivie d'une réduction.

Cette réaction s'effectue au sein d'un solvant inerte tel que le dioxanne ou le chloroforme. La réduction s'effectue de préférence, au moyen de borohydrure de sodium, au sein d'un solvant inerte tel que le diméthylformamide, à une température comprise entre 10 et 20°C.

Les dérivés de formule (XI) pour lesquels R₁ et R₂ ont les mêmes significations que dans la formule (I) et R₂₀ représente un radical -alk-COOH peuvent être obtenus par réduction d'un dérivé de formule (XIV) dans laquelle R₁ et R₂ ont les mêmes significations que dans la formule (I) et R₂₂ représente un radical -alk(1-3C)-COOalk ou -COOalk dans lesquels alk représente un radical alkyle, suivie d'une hydrolyse de l'ester ainsi obtenu.

Cette réduction s'effectue dans les conditions décrites précédemment pour la réduction des composés de formule (XIV) pour lesquels R₂₂ représente un radical alkyle. L'hydrolyse s'effectue par toute méthode connue et, de préférence, au moyen d'un acide tel que l'acide chlorhydrique, au sein d'un solvant inerte tel qu'un mélange de diméthylsulfoxyde et d'un éther (éther éthylique par exemple), à une température comprise entre 10 et 30°C.

Les dérivés de formule (XIV) dans laquelle R₁ et R₂ ont les mêmes significations que dans la formule (I) et R₂₂ représente un radical -alk(1-3C)-COOalk peuvent être obtenus de manière analogue à la préparation des dérivés de formule (XIV) pour lesquels R₂₂ représente un radical alkyle, à partir du dérivé OHC-alk(1-3C)-COOalk.

Les dérivés de formule (XIV) dans laquelle R₁ et R₂ ont les mêmes significations que dans la formule (I) et R₂₂ représente un radical -COOalk peuvent être obtenus par action d'un dérivé de formule (II) dans laquelle R₁ et R₂ ont les mêmes significations que dans la formule (I) sur un glyoxalate d'alkyle, au sein d'un solvant inerte tel que le diméthylsulfoxyde, en présence d'un hydrure de métal alcalin, à une température voisine de 20°C.

Les composés de formule (I) pour lesquels R représente un radical CH-R₆ dans lequel R₆ représente un radical -NR₈R₉, R₈ et R₉ représentent chacun un atome d'hydrogène peuvent être préparés par hydrolyse d'un composé de formule (I) correspondant pour lequel R représente un radical CH-R₆ dans lequel R₆ représente un radical -NR₈R₉, R₈ représente un atome d'hydrogène et R₉ représente un radical -COR₁₂ et R₁₂ représente un radical alkyle.

Cette hydrolyse s'effectue généralement au moyen d'un acide tel que l'acide chlorhydrique, en milieu aqueux, à la température d'ébullition du milieu réactionnel.

Les composés de formule (I) pour lesquels R représente un radical CH-R₆ dans lequel R₆ représente un radical -NR₈R₉, R₈ et R₉ représentent chacun un atome d'hydrogène peuvent également être préparés par réduction d'un composé de formule (I) correspondant pour lequel R représente un radical C=R₃ et R₃ représente un radical NOH.

Cette réduction s'effectue généralement au moyen de zinc, en présence d'acétate d'ammonium et d'ammoniaque à 28%, au sein d'un alcool tel que l'éthanol, à la température d'ébullition du milieu réactionnel.

Les composés de formule (I) pour lesquels R représente un radical CH-R₆ dans lequel R₆ représente un radical -NR₈R₉, R₈ représente un atome d'hydrogène et R₉ représente un radical -COR₁₂ et R₁₂ représente un radical alkyle peuvent être préparés par action d'un acide de formule :

R₂₄-COOH (XV)

dans laquelle R₂₄ représente un radical alkyle (1-3C) sur un composé de formule (I) correspondant pour lequel R représente un radical C=R₃ et R₃ représente un radical NOH, en présence d'un agent réducteur.

Cette réaction s'effectue généralement à une température comprise entre 50 et 100°C. Comme agent réducteur on utilise de préférence le zinc.

Les composés de formule (I) pour lesquels R représente un radical CH-R₆ et R₆ représente un radical -NR₈R₉ ou -alk-NR₈R₉, dans lesquels R₈ et R₉, identiques ou différents, représentent chacun un radical alkyle ou bien R₈ représente un atome d'hydrogène et R₉ représente un radical alkyle, -COR₁₂ ou -SO₂R₁₃ et R₁₂ représente un radical alkyle, cycloalkyle, phényle, -COO-alk ou -CH₂-COOX peuvent être préparés par action d'un composé de formule (I) correspondant pour lequel R₈ et R₉ représentent chacun un atome d'hydrogène ou bien R₈ représente un atome d'hydrogène et R₉ représente un radical alkyle, sur un halogénure de formule :

Hal-R₂₇ (XVI)

dans laquelle R₂₇ représente un radical alkyle, -COR₁₂ ou -SO₂R₁₃ et R₁₂ représente un radical alkyle, cycloalkyle, phényle, -COO-alk ou -CH₂-COOX et R₁₃ a les mêmes significations que dans la formule (I).

Cette réaction s'effectue de préférence au sein d'un solvant inerte tel que le diméthylformamide, le tétrahydrofuranne ou le diméthylsulfoxyde, en présence d'une base telle qu'une amine tertiaire (la triéthylamine par exemple) ou aromatique (pyridine par exemple) ou une base minérale telle qu'un hydroxyde de métal alcalin (soude, potasse par exemple), à une température comprise entre 20°C et la température d'ébullition du milieu réactionnel.

Les dérivés Hal-R₂₇ sont commercialisés ou ceux pour lesquels R₂₇ représente un radical -COR₁₂ peuvent être obtenus à partir des acides carboxyliques correspondants par application ou adaptation des méthodes décrites par B. HELFERICH et coll., Organic Synth., I, 147 et J. GASON, Organic Synth., III, 169 et ceux pour lesquels R₂₇ représente un radical -SO₂R₁₃ peuvent être obtenus par application ou adaptation des méthodes décrites dans HOUBEN-WEYL, volume 9, pages 390 et 564 (1955).

Les composés de formule (I) pour lesquels R représente un radical CH-R₆ et R₆ représente un radical -NR₈R₉ ou -alk-NR₈R₉ dans lesquels R₈ représente un atome d'hydrogène, R₉ représente un radical -COR₁₂ ou -CSR₃₀ et R₁₂ et R₃₀ représentent un radical -NH-alk, -NH₂, -NH-Ar ou -NH-Het peuvent être préparés par action d'un composé de formule (I) correspondant pour lequel R₈ et R₉ représentent chacun un atome d'hydrogène sur un dérivé R₂₈-N=C=R₃₁ pour lequel R₂₈ représente un radical triméthylsilyle, benzoyle, alkyle, phényle ou un hétérocycle mono ou polycyclique saturé ou insaturé contenant 4 à 9 atomes de carbone et un ou plusieurs hétéroatomes (O, S, N) et R₃₁ représente un atome d'oxygène ou de soufre, suivie éventuellement par une hydrolyse.

Cette réaction s'effectue de préférence au sein d'un solvant inerte tel que le diméthylformamide, le tétrahydrofuranne ou le dioxanne, à une température comprise entre 20°C et la température d'ébullition du milieu réactionnel. Pour les composés pour lesquels R₁₂ et R₃₀ sont des radicaux NH₂, cette réaction est suivie d'une hydrolyse du dérivé silylé ou du dérivé benzoylé précédemment obtenu soit au moyen d'eau, soit au moyen d'une solution aqueuse d'une base minérale telle qu'un hydroxyde de métal alcalin (soude, potasse par exemple), à une température comprise entre 20°C et la température d'ébullition du milieu réactionnel.

Les dérivés R₂₈-N=C=R₃₁ sont commercialisés ou peuvent être obtenus à partir des amines primaires correspondantes par action du phosgène ou de thiophosgène par application ou adaptation des méthodes décrites par R.L. SHRINER et coll., Organic Synth., II, 453 et G.M. DYSON, Organic Synth., I, 165; R.J. SLOCOMPIE et col., J. Am. Chem. Soc., 72, 1888 (1950) et S. PATAI, "The chemistry of cyanates and their thio derivatives", Ed. John Wiley and Sons, pages 619 et 819 (1977). Les amines primaires correspondantes sont commercialisées ou celles pour lesquelles R₂₈ représente un radical Het peuvent être obtenues par application ou adaptation des méthodes décrites par B.A. TERTOV et coll., Khim. Geterotsikl. Soedin, II, 1552 (1972) et R.C. LAROCK, "Comprehensive Organic Transformations", Ed. VCH, page 399, qui consiste à faire réagir l'organolithien ou l'organomagnésien de l'hétarocycle considéré sur PhN₃, en présence d'acide acétique, de (PhO)₂PON₃, de NH₂OCH₃ ou de N₃CH₂Si(CH₃)₃. Les organolithiens ou organomagnésiens peuvent être obtenus par application ou adaptation des méthodes décrites par D.L. COMINS et coll., J. Org. Chem., 52, 104 (1987); N. FURUKANA et coll., Tetrahedron Lett., 28 (47), 5845 (1987); A.R. KATRITZKY et coll., Org. Prep. Procedure Int., 20 (6), 585 (1988); A.J. CLARKE et coll., Tetrahedron Lett., 27, 2373 (1974) et A.W. GSCHWEN et coll., Organic Reaction, 26, 1 (1979).

Les composés de formule (I) pour lesquels R représente un radical CH-R₆ et R₆ représente un radical -NR₈R₉ ou -alk-NR₈R₉ dans lesquels R₈ représente un atome d'hydrogène, R₉ représente un radical -COR₁₂ et R₁₂ représente un radical -CH₂-NH₂ peuvent être préparés par action d'un composé de formule (I) correspondant pour lequel R₈ et R₉ représentent chacun un atome d'hydrogène sur un acide HOOC-CH₂-NH-R₂₉ dans lequel R₂₉ représente un groupe protecteur de la fonction amine tel que tert-butoxycarbonyle suivie d'une hydrolyse.

Cette réaction s'effectue, de préférence, au sein d'un solvant inerte tel que le diméthylformamide, en présence d'hydroxybenzotriazole, de 1-(3-diméthylaminopropyl)-3éthylcarbodiimide et d'une base organique telle qu'une trialkyamine (triéthylamine par exemple), à une température comprise entre 0 et 5°C. L'hydrolyse s'effectue généralement au moyen d'acide trifluoroacétique, à une température voisine de 20°C.

Les composés de formule (I) pour lesquels R représente un radical CH-R₆ dans lequel R₆ représente un radical -alk-Ar ou -alk-Het peuvent également être préparés par action d'un dérivé de formule (Il) sur un halogènure de formule :

Hal-R₂₅ (XVII)

dans laquelle R₂₅ représente un radical -alk-Ar ou -alk-Het.

Cette réaction s'effectue de préférence au sein d'un solvant inerte tel que le diméthylsulfoxyde, le diméthylformamide, le tétrahydrofuranne, le dioxanne, l'éther diéthylique, en présence d'une base tel qu'un hydroxyde de métal alcalin (soude, potasse par exemple), éventuellement en présence de bromure de tétrabutylammonium ou un hydrure de métal alcalin (hydrure de sodium par exemple), à une température voisine de 20°C.

Les composés de formule (I) pour lesquels R représente un radical CH-R₆, R₆ représente un radical -alk-NR₈R₉, R₈ et R₉ représentent chacun un atome d'hydrogène peuvent être préparés par action de brome et de soude sur un dérivé de formule : 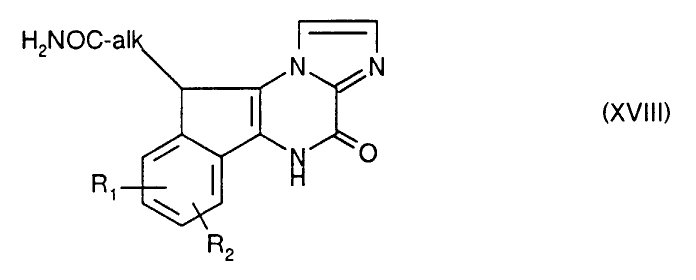 dans laquelle R₁ et R₂ ont les mêmes significations que dans la formule (I) et alk représente un radical alkyle.

Cette réaction s'effectue généralement en milieu aqueux, à une température comprise entre 20 et 70°C.

Les dérivés de formule (XVIII) peuvent être obtenus par action d'ammoniac sur un dérivé de formule : 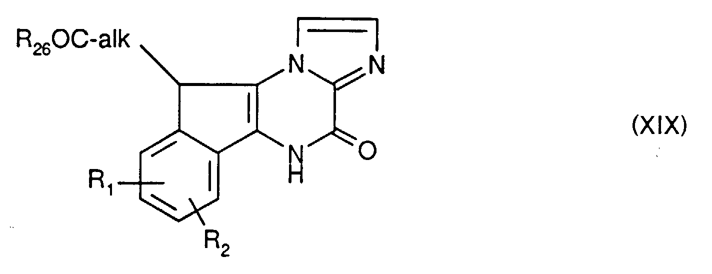 dans laquelle R₁ et R₂ ont les mêmes significations que dans la formule (I) et R₂₆ représente un radical alcoxy.

Cette réaction s'effectue généralement au sein d'un solvant inerte tel qu'un alcool, à une température voisine de 20°C.

Les dérivés de formule (XIX) peuvent être obtenus par hydrogénation des dérivés de formule : 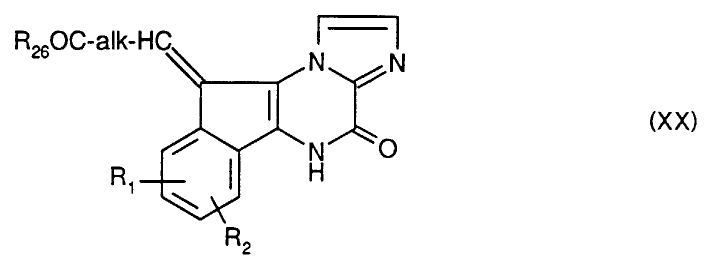 dans laquelle R₁ et R₂ ont les mêmes significations que dans la formule (I), R₂₆ représente un radical alcoxy et alk représente un radical alkyle (1-3C).

Cette réduction s'effectue dans les conditions décrites précédemment pour la réduction des composés de formule (XIV) pour lesquels R₂₂ représente un radical alkyle.

Les dérivés de formule (XX) peuvent être obtenus par action d'un dérivé de formule (Il) sur un aldéhyde de formule :

OHC-alk-COR₂₆ (XXI)

dans laquelle R₂₆ représente un radical alcoxy et alk représente un radical alkyle (1-3C).

Cette réaction s'effectue généralement soit au sein d'un solvant inerte tel que le diméthylformamide, le diméthylsulfoxyde, le 1,2-diméthoxyéthane, un alcool (méthanol, éthanol par exemple) ou un mélange de ces solvants, en présence d'une base telle que la soude, la potasse, une base organique forte telle que le 1,8-diazabicyclo[5,4,0]undec-7-ène, à une température comprise entre 20 et 100°C soit au sein du diméthylsulfoxyde, en présence d'un hydrure alcalin tel que l'hydrure de sodium, à une température voisine de 20°C soit en présence de bromure de tétrabutylammonium et d'une base telle qu'un hydroxyde de métal alcalin (soude, potasse par exemple), au sein du diméthylsulfoxyde, à une température comprise entre 10 et 100°C.

Les dérivés de formule (XXI) sont commercialisés ou peuvent être obtenus à partir des alcools correspondants par application ou adaptation des méthodes décrites par R.C. LAROCK, "Comprehensive Organic Transformations", Ed. VCH, page 612 (1989). Les alcools correspondants pauvent être obtenus par application ou adaptation de la méthode décrite par H.C. BROWN, J. Org. Chem., 31, 485 (1966).

Les composés de formule (I) pour lesquels R représente un radical CH-R₆, R₆ représente un radical -NR₈R₉ ou -alk-NR₈R₉, R₈ représente un atome d'hydrogène et R₉ représente un radical alkyle peuvent être préparés par réduction d'un dérivé de formule (XI) correspondant pour lequel R₁ et R₂ ont les mêmes significations que dans la formule (I) et R₂₀ représente un radical -NR₈R₉ ou -alk-NR₈R₉, R₈ représente un atome d'hydrogène et R₉ représente un radical -CHO ou -COR₁₂ dans lequel R₁₂ représente un radical alkyle ou cycloalkyle.

Cette réduction s'effectue généralement au sein d'un solvant inerte tel que le tétrahydrofuranne, en présence de B₂H₆, à la température d'ébullition du milieu réactionnel.

Les dérivés de formule (XI) pour lesquels R₁ et R₂ ont les mêmes significations que dans la formule (I) et R₂₀ représente un radical -NR₈R₉ ou -alk-NR₈R₉, R₈ représente un atome d'hydrogène et R₉ représente un radical -CHO peuvent être obtenus par action d'un composé de formule (I) correspondant pour lequel R représente un radical CH-R₆, R₆ représente un radical -NR₈R₉ ou -alk-NR₈R₉, R₈ représenté un atome d'hydrogène et R₉ représente un atome d'hydrogène sur le H₃C-COOCHO, à une température comprise entre 0 et 60°C, éventuellement en présence d'acétate de sodium anhydre.

Les composés de formule (I) pour lesquels soit R représente un radical C(R₄)R₅ dans lequel R₄ et/ou R₅ représente un radical -alk-Het, soit R représente un radical CH-R₆, R₆ représente un radical -alk-Het dans lesquels Het est un radical pipéridyle peuvent être également préparés par hydrogénation des composés de formule (I) correspondants pour lesquels soit R représente un radical C(R₄)R₅ dans lequel R₄ et/ou R₅ représente un radical -alk-Het, soit R représente un radical CH-R₆, R₆ représente un radical -alk-Het dans lesquels Het est un radical pyridyle.

Cette réduction s'effectue généralement au moyen d'hydrogène, sous une pression comprise entre 1 et 100 bar, au sein d'un solvant inerte tel que le diméthylformamide, l'acide acétique, l'acétate d'éthyle, un alcool (méthanol, éthanol par exemple) ou un mélange de ces solvants, en présence d'un catalyseur d'hydrogénation tel que l'oxyde de platine ou le nickel de Raney, à une température comprise entre 25 et 100°C.

Il est entendu pour l'homme du métier que, pour la mise en oeuvre des procédés selon l'invention décrits précédemment, il peut être nécessaire d'introduire des groupes protecteurs par exemple des fonctions amino afin d'éviter des réactions secondaires. Ces groupes sont ceux qui permettent d'être éliminés sans toucher au reste de la molécule. Comme exemples de groupes protecteurs de la fonction amino on peut citer les carbamates de tert-butyle ou de méthyle qui peuvent être régénérées au moyen d'iodotriméthylsilane. D'autres groupes protecteurs utilisables sont décrits par W. GREENE et coll., Protecting Groups in Organic Synthesis, second edition, 1991, Jonh Wiley & Sons.

Les composés de formule (I) peuvent être purifiés par les méthodes connues habituelles, par exemple par cristallisation, chromatographie ou extraction.

Les énantiomères des composés de formule (I) pour lesquels R représente un radical C(R₄)R₅ dans lequel R₄ est différent de R₅ ou CH-R₆ peuvent être obtenus par dédoublement des racémiques par exemple par chromatographie sur colonne chirale selon W.H. PIRCKLE et coll., asymetric synthesis, vol. 1, Academic Press (1983) ou par synthèse à partir des précurseurs chiraux.

Les diastéréoisomères des composés de formule (I) pour lesquels R représente un radical C(R₄)R₅ dans lequel R₄ est différent de R₅ ou CH-R₆ comportant un ou plusieurs carbones chiraux et les différents isomères E et Z des composés de formule (I) peuvent être séparés par les méthodes connues habituelles, par exemple par cristallisation ou chromatographie.

Les composés de formule (I) comportant un reste basique peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) comportant un reste acide peuvent éventuellement être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'une amine ou d'un sel d'une amine sur un composé de formule (I), dans un solvant. Le sel formé est séparé par les méthodes habituelles.

Ces sels font également partie de l'invention.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalino-terreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthyl glucamine).

Les composés de formule (I) présentent des propriétés pharmacologiques intéressantes. Ces composés sont des antagonistes du récepteur de l'acide α-amino-3-hydroxy-5-méthyl-4-isoxazolepropionique (AMPA), connu aussi sous le nom de récepteur du quisqualate.

Par ailleurs, les composés de formule (I) sont des antagonistes non compétitifs du récepteur N-méthyl-D-aspartate (NMDA) et, plus particulièrement, ce sont des ligands pour les sites modulateurs de la glycine du récepteur NMDA.

Ces composés sont donc utiles pour traiter ou prévenir toutes les ischémies (telles l'ischémie focale ou globale) consécutives à des accidents vasculaires cérébraux, un arrêt cardiaque, une hypotension artérielle, une intervention chirurgicale cardiaque ou pulmonaire ou une hypoglycémie sévère. Ils sont également utiles dans le traitement des effets dus à une anoxie, qu'elle soit périnatale ou consécutive à une noyade ou à des lésions cérébrospinales. Ces composés peuvent également être utilisés pour traiter ou prévenir l'évolution de maladies neurodégénératives, de la chorée d'HUNTINGTON, de la maladie d'ALZHEIMER, de la sclérose latérale amyotrophique, de l'atrophie olivo-pontocérébelleuse et de la maladie de PARKINSON. Ces composés peuvent aussi être utilisés vis-à-vis des manifestations épileptogènes et/ou convulsives, pour le traitement des traumatismes cérébraux ou spinaux, des traumatismes liés à la dégénérescence de l'oreille interne (R. PUJOL et coll., Neuroreport, 3, 299-302 (1992) ou de la rétine (J.L. MONSINGER et coll., Exp. Neurol., 113, 10-17 (1991), de l'anxiété (KEHNE et coll., Eur. J. Pharmacol., 193, 283 (1991)), de la dépression (TRULLAS et coll.,Eur. J. Pharmacol., 185, 1 (1990)), de la schizophrénie (REYNOLDS, TIPS, 13, 116 (1992)), du syndrome de TOURETTE, des encéphalopathies hépatiques, en tant qu'analgésiques (DICKENSON et coll., Neurosc. Letters, 121, 263 (1991)), antiinflammatoires (SLUTA et coll., Neurosci. Letters, 149, 99-102 (1993)) antianorexiques (SORRELS et coll., Brain Res., 572, 265 (1992)), antimigraineux, antiémétiques et pour traiter les empoisonnements par des neurotoxines ou d'autres substances agonistes du récepteur NMDA, ainsi que les troubles neurologiques associés aux maladies virales telles que le sida (LIPTON et coll., Neuron, 7, 111 (1991)), la rage, la rougeole et le tétanos (BAGETTA et coll., Br. J. Pharmacol., 101, 776 (1990)). Ces composés sont aussi utiles pour la prévention des symptômes d'abstinence aux drogues et à l'alcool et de l'inhibition de l'accoutumance et de la dépendance aux opiacés. Ils peuvent également être utilisés dans le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, l'hydroxybutirique-aminoacidurie, l'encéphalopathie de LEAD et la déficience en sulfite oxydase.

L'affinité des composés de formule (I) vis-à-vis du récepteur AMPA a été déterminée en étudiant l'antagonisme de la fixation spécifique du [³H]-AMPA sur des membranes de cortex cérébral de rat (HONORE et coll., Neuroscience letters, 54, 27 (1985)). Le [³H]-AMPA est mis à incuber en présence de 0,2 mg de protéines à 4°C pendant 30 minutes dans du tampon KH₂PO₄ 10mM, KSCN 100mM, pH7,5. La fixation non spécifique est déterminée en présence de L-glutamate 1mM. La radioactivité liée est séparée par filtration sur filtres PHARMACIA (Printed Filtermate A). L'activité inhibitrice de ces produits est inférieure ou égale à 100 µM.

L'affinité des composés de formule (I) pour le site glycine lié au récepteur NMDA a été déterminée en étudiant l'antagonisme de la fixation spécifique du [³H]-DCKA sur des membranes de cortex cérébral de rat selon la méthode décrite par T. CANTON et coll., J. Pharm. Pharmacol., 44, 812 (1992). Le [³H]-DCKA (20nM) est mis à incuber en présence de 0,1 mg de protéines à 4°C pendant 30 minutes dans du tampon HEPES 50 mM, pH7,5. La fixation non spécifique est déterminée en présence de glycine 1mM. La radioactivité liée est séparée par filtration sur filtres Whatman GF/B. L'activité inhibitrice de ces produits est inférieure ou égale à 100 µM.

Les composés de formule (I) présentent une toxicité faible. Leur DL50 est supérieure à 50 mg/kg par voie IP chez la souris.

Sont particulièrement intéressants les composés de formule (I) pour lesquels soit R représente un radical C=R₃, C(R₄)R₅ ou CH-R₆, R₁ et R₂, représentent des atomes d'hydrogène ou d'halogène, R₃ représente un atome d'oxygène ou un radical NO-alk-COOX ou CH-R₇, R₄ représente un radical alkyle ou -alk-Ar, R₅ représente un radical alkyle (1-11C en chaîne droite ou ramifiée), -alk-Het ou -alk-Ar ou bien R₄ et R₅ forment ensemble avec l'atome de carbone auquel ils sont rattachés un radical cycloalkyle, R₆ représente un radical hydroxy, alkyle (1-11C en chaîne droite ou ramifiée), -NR₈R₉, -alk-NR₈R_{9,} -alk-OH, -alk-Ar ou -alk-Het, R₇ représente un radical hydroxy, -NR₁₀R₁₁ ou un hétérocycle mono ou polycyclique saturé ou insaturé contenant 4 à 9 atomes de carbone et un ou plusieurs hétéroatomes (O, S, N), R₈ représente un atome d'hydrogène et R₉ représente un atome d'hydrogène ou un radical alkyle, -COR₁₂ ou -SO₂R₁₃, R₁₀ et R₁₁, identiques ou différents, représentent chacun un radical alkyle, R₁₂ représente un radical alkyle, -NH-Ar, -NH-alk ou phényle, R₁₃ représente un radical alkyle ou phényle, alk représente un radical alkyle ou alkylène, X représente un radical alkyle et Het représente un hétérocycle mono ou polycyclique saturé ou insaturé contenant 4 à 9 atomes de carbone et un ou plusieurs hétéroatomes (O, S, N) ou bien R représente un radical CH-R₆ et R₆ représente un radical 2-imidazolylméthyle et R₁, R₂ sont des atomes d'hydrogène.

D'un intérêt particulier sont les composés suivants :
- 10-hydroxy-5H,10H-imidazo[1,2-a]indéno[1,2-e]pyrazine-4-one,
- 10-acétamido-5H,10H-imidazo[1,2-a]indéno[1,2-e]pyrazine-4-one,
- 10-amino-5H,10H-imidazo[1,2-a]indéno[1,2-e]pyrazine-4-one,
- 10-(E-diméthylaminométhylène)-5H,10H-imidazo[1,2-a]indéno[1,2-e] pyrazine-4-one,
- 10-hydroxyméthylène-5H,10H-imidazo[1,2-a]indéno[1,2-e]pyrazine-4-one,
- 10,10-diméthyl-5H,10H-imidazo[1,2-a]indéno[1,2-e]pyrazine-4-one,
- spiro[5H,10H-imidazo[1,2-a]indéno[1,2-e]pyrazine-10:1'-cyclopropane]-4-one,
- spiro[5H,10H-imidazo[1,2-a]indéno[1,2-e]pyrazine-10:1'-cyclopentane]-4-one,
- 10-méthyl-5H,10H-imidazo[1,2-a]indéno[1,2-e]pyrazine-4-one,
- 10,10-dibenzyl-5H,10H-imidazo[1,2-a]indéno[1,2-e]pyrazine-4-one,
- 10-hydroxyméthyl-5H,10H-imidazo[1,2-a]indéno[1,2-e]pyrazine-4-one,
- 10-(2-furylméthyl)-5H,10H-imidazo[1,2-a]indéno[1,2-e]pyrazine-4-one,
- 10-(4-pyridylméthylène)-5H,10H-imidazo[1,2-a]indéno[1,2-e]pyrazine-4-one,
- 10-(4-pyridylméthyl)-5H,10H-imidazo[1,2-a]indéno[1,2-e]pyrazine-4-one,
- 10-(phénylpropyl)-5H,10H-imidazo[1,2-a]indéno[1,2-e]pyrazine-4-one,
- 10-(3-pyridylméthylène)-5H,10H-imidazo[1,2-a]indéno[1,2-e]pyrazine-4-one,
- 10-(3-pyridylméthyl)-5H,10H-imidazo[1,2-a]indéno[1,2-e]pyrazine-4-one,
- 10-(2-pyridylméthyl)-5H,10H-imidazo[1,2-a]indéno[1,2-e]pyrazine-4-one,
- 10-(2-imidazolylméthyl)-5H,10H-imidaio[1,2-a]indéno[1,2-e]pyrazine-4-one,
- (4-oxo-5H,10H-imidazo[1,2-a]indéno[1,2-e]pyrazine-10-ylidène)aminooxyacétate de tert-butyle
- 10-isobutyl-5H,10H-imidazo[1,2-a]indéno[1,2-e]pyrazine-4-one,
- acide (4-oxo-5H,10H-imidazo[1,2-a]indéno[1,2-e]pyrazine-10-ylidène)aminooxyacétique,
- 10-propionamido-5H,10H-imidazo[1,2-a]indéno[1,2-e]pyrazine-4-one,
- 10-amino-8-fluoro-5H,10H-imidazo[1,2-a]indéno[1,2-e]pyrazine-4-one,
- 10-(4-quinolinylméthylène)-5H,10H-imidazo[1,2-a]indéno[1,2-e]pyrazine-4-one,
- 10-(4-quinolinylméthyl)-5H,10H-imidazo[1,2-a]indéno[1,2-e]pyrazine-4-one,
- 10-(3-phényluréido)-5H,10H-imidazo[1,2-a]indéno[1,2-e]pyrazine-4-one,
- 10-(3-méthyluréido)-5H,10H-imidazo[1,2-a]indéno[1,2-e]pyrazine-4-one,
- 10-isobutyramido-5H,10H-imidazo[1,2-a]indéno[1,2-e]pyrazine-4-one,
- 10-amino-7-chloro-5H,10H-imidazo[1,2-a]indéno[1,2-e]pyrazine-4-one,
- 10-benzènesulfonylamido-5H,10H-imidazo[1,2-a]indéno[1,2-e]pyrazine-4-one,
- 10-méthylamino-5H,10H-imidazo[1,2-a]indéno[1,2-e]pyrazine-4-one,
- 10-(2-pyrazinylméthylène)-5H,10H-imidazo[1,2-a]indéno[1,2-e]pyrazine-4-one,
- 10-(2-pyrazinylméthyl)-5H,10H-imidazo[1,2-a]indéno[1,2-e]pyrazine-4-one,
- 10-benzyl-7-chloro-5H,10H-imidazo[1,2-a]indéno[1,2-e]pyrazine-4-one,
- 7-chloro-10-(3-phényluréido)-5H,10H-imidazo[1,2-a]indéno[1,2-e]pyrazine-4-one,
- 10-(2-hydroxyéthyl)-5H,10H-imidazo[1,2-a]indéno[1,2-e]pyrazine-4-one,
- 7-chloro-10-méthylamino-5H,10H-imidazo[1,2-a]indéno[1,2-e]pyrazine-4-one,
- 10-benzyl-10-méthyl-5H,10H-imidazo[1,2-a]indéno[1,2-e]pyrazine-4-one,
- 10-méthyl-10-(4-pyridylméthyl)-5H,10H-imidazo[1,2-a]indéno[1,2-e]pyrazine-4-one,
- 10-(4-pipéridylméthyl)-5H,10H-imidazo[1,2-a]indéno[1,2-e]pyrazine-4-one,
- 5H,10H-imidazo[1,2-a]indéno[1,2-e]pyrazine-4,10-dione,
- 10-benzyl-5H,10H-imidazo[1,2-a]indéno[1,2-elpyrazine-4-one,
- 10-hexyl-5H,10H-imidazo[1,2-a]indéno[1,2-e]pyrazine-4-one,
- 10-benzamido-5H,10H-imidazo[1,2-a]indéno[1,2-e]pyrazine-4-one,
- 10-méthyl-10-12-(pyridine-4-yl)éthyl]-5H,10H-imidazo[1,2-a]indéno[1,2-e] pyrazine-4-one.

Sont particulièrement intéressants vis-à-vis du récepteur AMPA les composés suivants :
- 10-hydroxy-5H,10H-imidazo[1,2-a]indéno[1,2-e]pyrazine-4-one,
- 10-acétamido-5H,10H-imidazo[1,2-a]indéno[1,2-e]pyrazine-4-one,
- 10-amino-5H,10H-imidazo[1,2-a]indéno[1,2-e]pyrazine-4-one,
- 10-(E-diméthylam inométhylène)-5H,10H-imidazo[1,2-a]indéno[1,2-e]pyrazine-4-one,
- 10-hydroxyméthylène-5H,10H-imidazo[1,2-a]indéno[1,2-e]pyrazine-4-one,
- 10,10-diméthyl-5H,10H-imidazo[1,2-a]indéno[1,2-e]pyrazine-4-one,
- spiro[5H,10H-imidazo[1,2-a]indéno[1,2-e]pyrazine-10:1'-cyclopropane]-4-one,
- spiro[5H,10H-imidazo[1,2-a]indéno[1,2-e]pyrazine-10:1'-cyclopentane]-4-one,
- 10-méthyl-5H,10H-imidazo[1,2-a]indéno[1,2-e]pyrazine-4-one,
- 10-hydroxyméthyl-5H,10H-imidazo[1,2-a]indéno[1,2-e]pyrazine-4-one,
- 10-(2-furylméthyl)-5H,10H-imidazo[1,2-a]indéno[1,2-e]pyrazine-4-one,
- 10-(4-pyridylméthylène)-5H,10H-imidazo[1,2-a]indéno[1,2-e]pyrazine-4-one,
- 10-(4-pyridylméthyl)-5H,10H-imidazo[1,2-a]indéno[1,2-e]pyrazine-4-one,
- 10-(phénylpropyl)-5H,10H-imidazo[1,2-a]indéno[1,2-e]pyrazine-4-one,
- 10-(3-pyridylméthylène)-5H,10H-imidazo[1,2-a]indéno[1,2-e]pyrazine-4-one,
- 10-(3-pyridylméthyl)-5H,10H-imidazo[1,2-a]indéno[1,2-e]pyrazine-4-one,
- 10-(2-pyridylméthyl)-5H,10H-imidazo[1,2-a]indéno[1,2-e]pyrazine-4-one,
- 10-(2-imidazolylméthyl)-5H,10H-imidazo[1,2-a]indéno[1.2-e]pyrazine-4-one,
- 10-isobutyl-5H,10H-imidazo[1,2-a]indéno[1,2-e]pyrazine-4-one,
- acide (4-oxo-5H,10H-imidazo[1,2-a]indéno[1,2-e]pyrazine-10-ylidène)aminooxyacétique,
- 10-propionamido-5H,10H-imidazo[1,2-a]indéno[1,2-e]pyrazine-4-one,
- 10-amino-8-fluoro-5H,10H-imidazo[1,2-a]indéno[1,2-e]pyrazine-4-one,
- 10-(3-méthyluréido)-5H,10H-imidazo[1,2-a]indéno[1,2-e]pyrazine-4-one,
- 10-isobutyramido-5H,10H-imidazo[1,2-a]indéno[1,2-e]pyrazine-4-one,
- 10-amino-7-chloro-5H,10H-imidazo[1,2-a]indéno[1,2-e]pyrazine-4-one,
- 10-benzènesulfonylamido-5H,10H-imidazo[1,2-a]indéno[1,2-e]pyrazine-4-one,
- 10-méthylamino-5H,10H-imidazo[1,2-a]indéno[1,2-e]pyrazine-4-one,
- 10-(2-pyrazinylméthyléne)-5H,10H-imidazo[1,2-a]indéno[1,2-e]pyrazine-4-one,
- 10-(2-pyrazinylméthyl)-5H,10H-imidazo[1,2-a]indéno[1,2-e]pyrazine-4-one,
- 10-(2-hydroxyéthyl)-5H,10H-imidazo[1,2-a]indéno[1,2-e]pyrazine-4-one,
- 7-chloro-10-méthylamino-5H,10H-imidazo[1,2-a]indéno[1,2-e]pyrazine-4-one,
- 10-méthyl-10-(4-pyridylméthyl)-5H,10H-imidazo[1,2-a]indéno[1,2-e]pyrazine-4-one,
- 10-(4-pipéridylméthyl)-5H,10H-imidazo[1,2-a]indéno[1,2-e]pyrazine-4-one,
- 10-benzyl-7-chloro-5H,10H-imidazo[1,2-a]indéno[1,2-e]pyrazine-4-one,
- 5H,10H-imidazo[1,2-a]indéno[1,2-e]pyrazine-4,10-dione,
- 10-3-phényluréido)-5H,10H-imidazo[1,2-a]indéno[1,2-e]pyrazine-4-one,
- 7-chloro-10-(3-phényluréido)-5H,10H-imidazo[1,2-a]indéno[1,2-e]pyrazine-4-one,
- 10-méthyl-10-[2-(pyridine-4-yl)éthyl]-5H,10H-imidazo[1,2-a]indéno[1,2-e] pyrazine-4-one.

Sont paticulièrement intéressants vis-à-vis du site modulateur de la glycine les composés suivants :
- 10-hydroxy-5H,10H-imidazo[1,2-a]indéno[1,2-e]pyrazine-4-one,
- 10-acétamido-5H,10H-imidazo[1,2-a]indéno[1,2-e]pyrazine-4-one,
- 10-amino-5H,10H-imidazo[1,2-a]indéno[1,2-e]pyrazine-4-one,
- 10-(E-diméthylaminométhylène)-5H,10H-imidazo[1,2-a]indéno[1,2-e]pyrazine-4-one,
- 10-hydroxyméthylène-5H,10H-imidazo[1,2-a]indéno[1,2-e]pyrazine-4-one,
- 10,10-diméthyl-5H,10H-imidazo[1,2-a]indéno[1,2-e]pyrazine-4-one,
- spiro[5H,10H-imidazo[1,2-a]indéno[1,2-e]pyrazine-10:1'-cyclopropane]-4-one,
- spiro[5H,10H-imidazo[1,2-a]indéno[1,2-e]pyrazine-10:1'-cyclopentane]-4-one,
- 10-méthyl-5H,10H-imidazo[1,2-a]indéno[1,2-e]pyrazine-4-one,
- 10,10-dibenzyl-5H,10H-imidazo[1,2-a]indéno[1,2-e]pyrazine-4-one,
- 10-hydroxyméthyl-5H,10H-imidazo[1,2-a]indéno[1,2-e]pyrazine-4-one,
- 10-(2-furylméthyl)-5H,10H-imidazo[1,2-a]indéno[1,2-e]pyrazine-4-one,
- 10-(4-pyridylméthyl)-5H,10H-imidazo[1,2-a]indéno[1,2-e]pyrazine-4-one,
- 10-(phénylpropyl)-5H,10H-imidazo[1,2-a]indéno[1,2-e]pyrazine-4-one,
- 10-(3-pyridylméthyl)-5H,10H-imidazo[1,2-a]indéno[1,2-e]pyrazine-4-one,
- 10-(2-pyridylméthyl)-5H,10H-imidazo[1,2-a]indéno[1,2-e]pyrazine-4-one,
- 10-(2-imidazolylméthyl)-5H,10H-imidazo[1,2-a]indéno[1,2-e]pyrazine-4-one,
- 10-isobutyl-5H,10H-imidazo[1,2-a]indéno[1,2-e]pyrazine-4-one,
- acide (4-oxo-5H,10H-imidazo[1,2-a]indéno[1,2-e]pyrazine-10-ylidène)aminooxyacétique,
- 10-propionamido-5H,10H-imidazo[1,2-a]indéno[1,2-e]pyrazine-4-one,
- 10-(4-quinolinylméthylène)-5H,10H-imidazo[1,2-a]indéno[1,2-e]pyrazine-4-one,
- 10-(4-quinolinylméthyl)-5H,10H-imidazo[1,2-a]indéno[1,2-e]pyrazine-4-one,
- 10-(3-phényluréido)-5H,10H-imidazo[1,2-a]indéno[1,2-e]pyrazine-4-one,
- 10-(3-méthyluréido)-5H,10H-imidazo[1,2-a]indéno[1,2-e]pyrazine-4-one,
- 10-isobutyramido-5H,10H-imidazo[1,2-a]indéno[1,2-e]pyrazine-4-one,
- 10-amino-7-chloro-5H,10H-imidazo[1,2-a]indéno[1,2-e]pyrazine-4-one,
- 10-benzènesulfonylamido-5H,10H-imidazo[1,2-a]indéno[1,2-e]pyrazine-4-one,
- 10-méthylamino-5H,10H-imidazo[1,2-a]indéno[1,2-e]pyrazine-4-one,
- 10-(2-pyrazinylméthyl)-5H,10H-imidazo[1,2-a]indéno[1,2-e]pyrazine-4-one,
- 10-benzyl-7-chloro-5H,10H-imidazo[1,2-a]indéno[1,2-e]pyrazine-4-one,
- 7-chloro-10-(3-phényluréido)-5H,10H-imidazo[1,2-a]indéno[1,2-e]pyrazine-4-one,
- 7-chloro-10-méthylamino-5H,10H-imidazo[1,2-a]indéno[1,2-e]pyrazine-4-one,
- 10-benzyl-10-méthyl-5H,10H-imidazo[1,2-a]indéno[1,2-e]pyrazine-4-one,
- 10-méthyl-10-(4-pyridyiméthyl)-5H,10H-imidazo[1,2-a]indéno[1,2-e] pyrazine-4-one,
- 10-benzyl-5H,10H-imidazo[1,2-a]indéno[1,2-e]pyrazine-4-one,
- 10-hexyl-5H,10H-imidazo[1,2-a]indéno[1,2-e]pyrazine-4-one,
- 10-benzamido-5H,10H-imidazo[1,2-a]indéno[1,2-e]pyrazine-4-one,
- 10-(2-hydroxyéthyl)-5H,10H-imidazo[1,2-a]indéno[1,2-e]pyrazine-4-one,
- 10-méthyl-10-[2-(pyridine-4-yl)éthyl]-5H,10H-imidazo[1,2-a]indéno[1,2-e] pyrazine-4-one.

Les exemples suivants illustrent l'invention.

### EXEMPLE 1

0,4 g d'hydrure de sodium à 80% est ajouté à une suspension de 1,1 g de 5H,10H-imidazo[1,2-a]indéno[1,2-e]pyrazine-4-one dans 10 ml de diméthylsulfoxyde anhydre. Après 10 minutes d'agitation à une température voisine de 20°C, une solution de 0,7 g de nitrite d'isoamyle dans 2 ml de diméthylsulfoxyde anhydre est ajoutée goutte à goutte en 5 minutes puis le mélange est agité pendant 1 heure à la même température. 10 ml d'eau distillée sont ajoutés lentement et le mélange est ensuite versé sur 120 g d'eau et de glace, acidifié avec 1 ml d'acide acétique puis centrifugé. Après élimination de la solution surnageante, le solide est mis en suspension dans 25 ml d'eau distillée, filtré, lavé avec 10 ml d'acétone et séché sous pression réduite (15 mm Hg; 2 kPa) à 20°C. Le produit obtenu (1,5 g) est dissous dans 100 ml de diméthylformamide bouillant et la solution, additionnée de 0,1g de noir décolorant, est filtrée à chaud, refroidie, versée sur 800 ml d'eau distillée et centrifugée. Le solide est mis en suspension dans 20 ml d'eau distillée, filtré, lavé avec 20 ml d'acétone et séché sous pression réduite (15 mm Hg; 2 kPa) à 20°C. Le produit obtenu (0,9 g) est dissous dans 75 ml de diméthysulfoxyde à 20°C et la solution, additionnée de 0,1 g de noir décolorant est filtrée. Le filtre est lavé 2 fois avec 20 ml au total de diméthylsulfoxyde puis le filtrat et le lavage sont réunis, additionnés de 75 ml d'eau distillée et centrifugés. Le solide est mis en suspension dans 25 ml d'eau distillée, filtré, lavé 2 fois avec 50 ml au total d'acétone puis séché sous pression réduite (1 mm Hg; 0,13 kPa) à 60°C. On obtient ainsi 0,63 g de 10-(E-hydroxyimino)-5H,10H-imidazo[1,2-a]indéno[1,2-e]pyrazine-4-one se décomposant sans fondre au-dessus de 300°C [Spectre de R.M.N.: (200 MHz; DMSO d6; δ en ppm) : 7,40 et 7,48 (2t, J=7 Hz, 2H: -H7 et -H8); 7,60 et 8,00 (2s larges, 1H chacun: -H de l'imidazole); 7,82 et 8,20 (2d, J=7 Hz, 1H chacun: -H6 et -H9); 12,70 et 13,00 (2mf, 1H chacun: -NH- et -OH)].

La 5H,10H-imidazo[1,2-a]indéno[1,2-e]pyrazine-4-one peut être préparée de la manière suivante : une solution de 4,8 g de bromure de 3-méthyl-4-oxo-5H,10H-imidazo[1,2-a]indéno[1,2-e]pyrazinium dans 30 g d'imidazole est chauffée pendant 24 heures à 160°C, refroidie à 100°C puis versée sur un mélange agité de 75 g de glace et de 75 g d'eau distillée. L'insoluble est filtré, lavé 2 fois avec 20 ml au total d'eau distillée puis séché sous pression réduite (10 mm Hg; 1,3 kPa) à 50°C. Le produit ainsi obtenu (4 g) est dissous dans 80 ml de diméthylformamide et la solution additionnée de 20 g de silice est concentrée à sec sous pression réduite (15 mm Hg, 2 kPa) à 100°C. Le mélange est introduit dans une colonne de 4,2 cm de diamètre contenant 240 g de silice puis est élué par un mélange dichlorométhane-méthanol (97-3 en volumes) en recueillant des fractions de 60 ml . Les fractions 10 à 70 sont réunies, additionnées de 1,5 g de noir décolorant, filtrées et concentrées à sec sous pression réduite (15 mm Hg; 2 kPa) à 55°C. Le produit obtenu (1,7 g) est dissous dans 350 ml de méthanol bouillant et la solution additionnée de 0,1g de noir décolorant est filtrée à chaud, concentrée sous pression réduite (15 mm Hg; 2 kPa) à 40°C pour ramener son volume à environ 30 ml puis conservée à 5°C pendant 60 heures. Les cristaux sont séparés par filtration, lavés 2 fois avec 20 ml au total de méthanol glacé et séchés sous pression réduite (1 mm Hg; 0,13 kPa) à 60°C. On obtient ainsi 1,1 g de 5H,10H-imidazo[1,2-a]indéno[1,2-e]pyrazine-4-one se décomposant sans fondre à 350°C [Rf= 0,77, chromatographie sur couche mince de gel de silice, solvant : dichlorométhane-méthanol (8-2 en volumes)].

Le bromure de 3-méthyl-4-oxo-5H,10H-imidazo[1,2-a]indéno[1,2-e] pyrazi-nium peut être préparé de la manière suivante : une solution de 5 g de 1-méthyl-1H-imidazole-2-carboxamide et de 12 g de 2-bromoindanone à 85% dans 100 ml de diméthylformamide anhydre est agitée pendant 28 heures à 115°C puis refroidie à une température voisine de 20°C. L'insoluble est séparé par filtration, lavé 2 fois avec 20 ml au total de diméthylformamide glacé et séché sous pression réduite (10 mm Hg; 1,3 kPa). On obtient ainsi 4,8 g de bromure de 3-méthyl-4-oxo-5H,10H-imidazo[1,2-a]indéno[1,2-e]pyra zinium [Spectre de R.M.N.: (200 MHz; DMSO d6; δ en ppm) : 4,13 (s, 2H : -CH2 en 10); 4,34 (s, 3H : N⁺ -CH3; 7,47 (mt, 2H :- H7 et -H8); 7,68 et 7,96 (2d, J=7,5 Hz, 1H chacun : -H6 et -H9); 8,32 et 8,45 (2d, J = 1 Hz, 1H chacun : H de l'imidazole); 13,60 (mf, 1H: NH)].

Le 1-méthyl-1H-imidazole-2-carboxamide peut être préparée selon le procédé décrit par D.D. DAVEY, J. Org. Chem., 52, 4379 (1987).

### EXEMPLE 2

Une suspension de 1,5 g de 10-(hydroxyimino)-5H,10H-imidazo[1,2-a] indéno[1,2-e]pyrazine-4-one dans 90 ml d'une solution aqueuse d'acide chlorhydrique environ 5N est agitée à l'ébullition pendant 7 heures, refroidie puis concentrée à sec sous pression réduite (15 mm Hg; 2 kPa) à 70°C. Le produit obtenu (2 g) est dissous dans 50 ml de diméthylformamide et la solution, additionnée de 0,1 g de noir décolorant, est filtrée puis le filtre est lavé 3 fois avec 30 ml au total de diméthylformamide. Le filtrat et le lavage sont réunis, additionnés de 600 ml d'eau distillée et centrifugés. Le solide est mis en suspension dans 20 ml d'eau distillée, filtré, lavé avec 20 ml d'acétone et séché sous pression réduite (1 mm Hg; 0,13 kPa) à 100°C. Le produit obtenu (1,13g) est dissous dans 115 ml de diméthylsulfoxyde et la solution, additionnée de 0,1 g de noir décolorant, est filtrée puis le filtre est lavé 2 fois avec 30 ml au total de diméthylsulfoxyde. Le filtrat et le lavage sont réunis, additionnés de 115 ml d'eau distillée et centrifugés. Le solide est mis en suspension dans 20 ml d'eau distillée, filtré, lavé 2 fois avec 20 ml au total d'acétone et séché sous pression réduite (1 mm Hg; 0,13 kPa) à 100°C. On obtient ainsi 1 g de 5H,10H-imidazo[1,2-a]indéno[1,2-e]pyrazine-4,10-dione sous la forme d'un solide rouge orangé fondant à 360°C (décomposition) [Spectre de R.M.N.: (200 MHz; DMSO d6; d en ppm) : 7,42 et 7,55 (2t, J=7 Hz, 2H : -H7 et -H8); 7,52 et 7,72 (2d, J=7 Hz, 1H chacun : -H6 et -H9); 7,62 et 8,13 (2d, J=1 Hz, 1H chacun : -H de l'imidazole); 13,60 (mf, 1H: -NH-)].

### EXEMPLE 3

0,86 g de borohydrure de sodium sont ajoutés en 5 minutes à une suspension agitée de 2,4 g de 5H,10H-imidazo[1,2-a]indéno[1,2-e]pyrazine-4,10-dione dans 75 ml de méthanol à une température comprise entre 25°C et 35°C. Après 30 minutes d'agitation à 20°C, l'insoluble est isolé par filtration, lavé avec 10 ml de méthanol et séché sous pression réduite (15 mm Hg; 2kPa) à 20°C. Le produit obtenu (2,3g) est dissous dans 100 ml de diméthylformamide et la solution, additionnée de 0,1 g de noir décolorant, est filtrée puis le filtre est lavé avec 20 ml de diméthylformamide. Le filtrat et le lavage sont réunis et additionnés de 240 ml d'eau distillée. Le solide est filtré, lavé 2 fois avec 40 ml au total d'eau distillée et avec 20 ml d'acétone puis séché sous pression réduite (1 mm Hg; 0.13 kPa) à 100°C. On obtient ainsi 1,9 g de 10-hydroxy-5H,10H-imidazo[1,2-a]indéno[1,2-e]pyrazine-4-one fondant à 290°C (décomposition) [Spectre de R.M.N.: (200 MHz; DMSO d6; δ en ppm) : 5,65 (d, J=8,5 Hz, 1H: CH-O-); 6,17 (d, J=8.5 Hz, 1H: -OH); 7,33 et 7,41 (2dt, J=7,5 et 1 Hz, 1H chacun : -H7 et -H8); 7,57 et 7,77 (2dd, J=7,5 et 1 Hz, 1H chacun : -H6 et -H9); 7,58 et 7,93 (2s larges, 1H chacun : -H de l'imidazole); 12,40 (mf, 1H : -NH-)].

### EXEMPLE 4

Une suspension de 5,25 g de 10-(E-hydroxyimino)-5H,10H-imidazo[1,2-a]indéno[1,2-e]pyrazine-4-one et de 2,9 g de zinc en poudre dans 100 ml d'acide acétique est chauffée pendant 2 heures à une température comprise entre 80°C et 90°C. Après addition de 100 ml d'acide acétique, le mélange est filtré et le filtrat est concentré à sec sous pression réduite (10 mm Hg; 2 kPa) à 65°C. Le produit obtenu (3,8 g) est mis en suspension dans 100 ml d'eau distillée, filtré, lavé avec 10 ml d'eau distillée et avec 10 ml d'acétone puis séché sous pression réduite (15 mm Hg; 2 kPa) à 20°C. Le produit obtenu (2 g) est dissous dans 60 ml de diméthylformamide bouillant et la solution, additionnée de 0,1 g de noir décolorant est filtrée à chaud. Le filtre est lavé avec 10 ml de diméthylformamide bouillant puis le filtrat et le lavage réunis sont conservés pendant 4 heures à une température voisine de 20°C. Les cristaux apparus sont séparés par filtration, lavés successivement avec 10 ml de diméthylformamide, 10 ml d'eau distillée, 10 ml d'acétone et séchés à sec sous pression réduite (1 mm Hg; 0.13 kPa) à 100°C. On obtient ainsi 0,43 g de 10-acétamido-5H,10H-imidazo[1,2-a]indéno[1,2-e]pyrazine-4-one se décomposant sans fondre à 330°C [Spectre de R.M.N. : (200 MHz; DMSO d6; δ en ppm) : 2,00 (s, 3H : -CO-CH₃); 6,13 (d, J=8,5 Hz, 1H: CH-N); 7,35 et 7,48 (2t, J=7,5 Hz, 1H chacun : -H7 et -H8); 7,48 et 7,85 (2d, J=7,5 Hz, 1H chacun : -H6 et -H9); 7,58 et 7,65 (2s larges, 1H chacun : -H de l'imidazole); 8,58 (d, J=8,5 Hz, 1H: -NH-COCH₃); 12,50 (mf, 1H : -NH-)].

### EXEMPLE 5

Une solution de 12,9 g de 10-acétamido-5H,10H-imidazo[1,2-a]indéno[1,2-e] pyrazine-4-one dans 650 ml d'une solution aqueuse 2N d'acide chlorhydrique est chauffée à l'ébullition pendant 2 heures, refroidie puis concentrée à sec sous pression réduite (15 mm Hg; 2 kPa) à 80°C. 4 g (sur les 14,8 g obtenus au total) sont dissous dans 250 ml d'eau distillée et la solution est agitée pendant 16 heures à une température voisine de 20°C. Les critaux apparus sont séparés par filtration, lavés successivement avec 25 ml d'eau distillée et 25 ml de méthanol puis séchés à l'air à une température voisine de 20°C. Le produit obtenu (3,5 g) est agité en suspension pendant 10 minutes dans 100 ml de méthanol bouillant et, après refroidissement et conservation pendant 1 heure à 5°C, isolé par filtration, lavé avec 20 ml de méthanol glacé puis séché sous pression réduite (1 mm Hg; 0,13 kPa) à 100°C. On obtient ainsi 2,1 g de chlorhydrate de 10-amino-5H,10H-imidazo[1,2-a]indéno[1,2-e] pyrazine-4-one se décomposant sans fondre vers 240°C [Spectre de R.M.N.: (200 MHz; DMSO d6; δ en ppm) : 5,70 (s large, 1H: CH-N+-Cl-); 7,48 et 7,58 (2t, J=7,5 Hz, 1H chacun : -H7 et -H8); 7,72 et 8,76 (2s, 1H chacun : -H de l'imidazole); 7,98 et 8,09 (2d, J=7,5 Hz, 1H chacun : -H6 et -H9); 9,47 (mf, 3H : N⁺H3Cl-); 12,80 (mf, 1H : -NH-)].

### EXEMPLE 6

6,3 g de t-butoxy-bis(diméthylamino)méthane sont ajoutés goutte à goutte à une température voisine de 25°C en 5 minutes à une suspension de 5,5 g de 5H,10H-imidazo[1,2-a]indéno[1,2-e]pyrazine-4-one dans 100 ml de diméthylformamide. Après 30 minutes d'agitation à la même température, le mélange est versé sur 500 ml d'eau distillée et extrait 5 fois avec 1,5 litre au total de chloroforme. Les extraits organiques sont réunis, lavés avec 250 ml d'eau distillée, séchés sur du sulfate de magnésium anhydre et concentrés à sec sous pression réduite (15 mm Hg; 2 kPa) à 60°C. Le produit obtenu (4,5 g) est mis en suspension dans 25 ml de méthanol, filtré, lavé 2 fois avec 20 ml au total de méthanol et séché sous pression réduite (15 mm Hg; 2 kPa) à 20°C. Le produit obtenu (4,5 g) est dissous dans 45 ml de diméthylformamide bouillant et la solution, après refroidissement, est conservée pendant 4 heures à une température voisine de 5°C. Les cristaux sont séparés par filtration, lavés successivement avec 10 ml de diméthylformamide, 10 ml d'acétone et séchés à sec sous pression réduite (1 mm Hg; 0,13 kPa) à 100°C. On obtient ainsi 4 g de 10-(E-diméthylaminométhylène-5H,10H-imidazo[1,2-a]indéno[1,2-e]pyrazine-4-one fondant à 293°C [Spectre de R.M.N.: (200 MHz; DMSO d6; δ en ppm): 3,35 [s, 6H : -N(CH3)2]; 7,18 et 7,28 (2t, J=7,5 Hz, 2H : -H7 et -H8); 7,48 et 7,92 (2d, J=7,5 Hz, 1H chacun : -H6 et -H9); 7,63 et 8,50 (2s larges, 1H chacun : -H de l'imidazole); 8,09 (s, 1H : =CH-N); 12.30 (mf, 1H : -NH-)].

### EXEMPLE 7

Une solution de 1,4 g de 10-diméthylaminométhylène-5H,10H-imidazo[1,2-a]indéno[1,2-e]pyrazine-4-one dans 35 ml d'acide chlorhydrique 5N est agitée pendant 30 minutes à une température voisine de 25°C. Après addition de 60 ml d'eau distillée et neutralisation par 120 ml d'une solution aqueuse saturée d'hydrogénocarbonate de sodium, le solide apparu est séparé par filtration, lavé 2 fois par 60 ml au total d'eau distillée et séché à l'air. Le produit obtenu (1,1 g) est dissous dans 120 ml de diméthylsulfoxyde et, après addition de 120 ml d'eau distillée, le solide apparu est séparé par filtration, lavé 2 fois avec 10 ml au total d'eau distillée et 2 fois avec 10 ml au total d'acétone puis séché sous pression réduite (1 mm Hg; 0,13 kPa) à 100°C. On obtient ainsi 1 g de 10-hydroxy méthylène5H,10H-imidazo[1,2-a]indéno[1,2-e]pyrazine-4-one, mélange 60-40 des formes Z et E, se décomposant sans fondre à 290°C [Spectre de R.M.N.: (200 MHz; DMSO d6; δ en ppm) : on observe un mélange d'isomères 60/40 : de 7,20 à 7,40 (mt, 2H : -H7 et -H8); 7,56 et 7,64 - 8,29 et 8,79 (4s larges, 2fois 1H: -H de l'imidazole); de 7,80 à 8,15 (mt, 2H :-H6 et -H9); 8,21 et 8,24 (2s, 1H en totalité : =CH-O-); 12,43 (mf, 1H: -NH-)].

### EXEMPLE 8

On agite pendant 1 heure à une température voisine de 20°C une solution de 2 g de 5H,10H-imidazo[1,2-a]indéno[1,2-e]pyrazine-4-one dans 50 ml de diméthylsulfoxyde anhydre avec 4 g de soude perlée et 50 mg de bromure de tétrabutylammonium. On ajoute ensuite goutte à goutte 0,87 ml d'iodure de méthyle et on maintient l'agitation pendant 6 heures. Le mélange réactionnel est alors versé dans 300 g d'eau et de glace, acidifié à pH 1 avec de l'acide chlorhydrique 1 N et extrait avec 3 fois 100 ml de chloroforme. La phase organique est lavée avec 100 ml d'eau, filtrée, séchée sur sulfate de magnésium et concentrée à l'évaporateur rotatif. L'huile noire obtenue (2,8 g) est purifiée par chromatographie sur colonne de silice (250 g) avec un mélange d'acétate d'éthyle et de méthanol (95/5 en volumes) et le solide coloré obtenu (0,5 g) est trituré avec 20 ml d'acétone, filtré, lavé avec un mélange de 10 ml d'acétate d'éthyle et 1 ml d'éthanol et séché à 50°C pour donner 0,2 g de 5H,10H-10,10-diméthyl-imidazo[1,2-a]indéno[1,2-e] pyrazine-4-one sous forme de solide crème fondant au-dessus de 260°C (Analyse % calculé C : 71,70, H : 5,21, N : 16,72, O : 6,37, % trouvé C : 71,7, H : 5,3, N : 16,4, O : 6,4).

### EXEMPLE 9

On agite à une température voisine de 18°C une suspension de 1,3 g de 5H,10H-imidazo[1,2-a]indéno[1,2-e]pyrazine-4-one, 2 g de soude perlée et 32 mg de bromure de tétrabutylammonium dans 10 ml de diméthylsulfoxyde sec. On ajoute ensuite une solution de 0,4 ml de 1,2-dibromoéthane dans 5 ml de diméthylsulfoxyde et on maintient l'agitation durant la nuit à une température voisine de 20°C. Le mélange réactionnel est versé dans un mélange d'eau et de glace (250 ml), acidifié avec 8 ml d'acide acétique et maintenu sous agitation pendant 1 heure et 30 minutes. On filtre ensuite le solide formé et on le purifie par chromatographie sur colonne de silice (100 g) avec un mélange de dichlorométhane et de méthanol (95/5 en volumes). On obtient, après séchage à 60°C, 0,45 g de spiro[5H,10H-imidazo[1,2-a]indéno[1,2-e] pyrazine-10:1'-cyclopropane]-4-one sous forme de solide rose fondant au-dessus de 260°C (Analyse % calculé C : 72,28, H : 4,45, N : 16,86, O : 6,42, % trouvé C : 71,8, H : 4,4, N : 16,3).

### EXEMPLE 10

On opère comme à l'exemple 9 mais à partir de 1,1 g de 5H,10H-imidazo[1,2-a]indéno[1,2-e]pyrazine-4-one, 40 ml de diméthylsulfoxyde, 2 g de soude perlée, 32 mg de bromure de tétrabutylammonium et 0,6 ml de 1,4-dibromobutane. On obtient, après séchage à 80°C, 0,17 g de spiro[5H,10H-imidazo[1,2-a]indéno[1,2-e]pyrazine-10:1'-cyclopentane]-4-one sous forme de solide blanc fondant au-dessus de 260°C (Analyse % calculé C : 73,63, H : 5,45, N : 15,15, O : 5,77, % trouvé C : 73,8, H : 5,8, N : 15,1, O : 6,0).

### EXEMPLE 11

Un mélange de 1 g de 10-hydroxyméthylène-5H,10H-imidazo[1,2-a]indéno[1,2-e] pyrazine-4-one, 80 ml de diméthylformamide et 20 ml de méthanol est hydrogéné à une température voisine de 20°C et sous pression normale pendant 4 heures en présence de charbon palladié à 10%. Après filtration du catalyseur sous atmosphère inerte, on évapore les solvants et le solide beige obtenu (1,25 g) est purifié par chromatographie sur colonne de silice (100 g) avec un mélange de dichlorométhane et de méthanol (95/5 en volumes). On obtient, après séchage à 90°C, 0,35 g de 10-méthyl-5H,10Himidazo[1,2-a]indéno[1,2-e]pyrazine-4-one sous forme de solide crème fondant au-dessus de 260°C (Analyse % calculé C : 70,87, H : 4,67, N: 17,71, O : 6,74, % trouvé C : 70,5, H : 4,4, N : 17,4).

### EXEMPLE 12

A une solution de 0,45 g de 5H,10H-imidazo[1,2-a]indéno[1,2-e]pyrazine-4-one dans 20 ml de diméthylsulfoxyde sous azote, on ajoute à une température voisine de 20°C et sous agitation 60 mg d'hydrure de sodium. L'agitation est maintenue pendant 2 heures, puis on rajoute 66 mg d'hydrure de sodium.

Le milieu réactionnel est chauffé à 45°C pendant 30 minutes, refroidi vers 20°C et additionné de 0,25 ml de bromure de benzyle. On poursuit l'agitation pendant 3 heures, verse le mélange réactionnel dans 150 ml d'eau, acidifie à pH 4 avec de l'acide acétique et extrait avec trois fois 60 ml de chloroforme. Les phases organiques sont réunies, lavées avec deux fois 100 ml d'eau, séchées sur sulfate de magnésium, filtrées et concentrées à l'évaporateur rotatif. Le solide noir obtenu (0,78 g) est purifié par chromatographie sur colonne de silice (70 g) avec un mélange de dichlorométhane et de méthanol (95/5 en volumes) pour donner 0,1 g de 10,10-dibenzyl-5H,10H-imidazo[1,2-a]indéno[1,2-e]pyrazine-4-one sous forme de solide vert clair fondant au-dessus de 260°C [Spectre de RMN : (200MHz; DMSO d6; δ en ppm) : 3,63 et 3,85 (2d, J = 14 Hz, 2H chacun : -CH₂-Ar); 6,36 (d, J = 7 Hz, 4 H : -H en ortho des benzyles); de 6,75 à 6,95 (mt, 6 H :-H en méta et -H en para des benzyles); de 7,29 à 7,45 (2t, J = 7,5 Hz, 1H chacun : -H₇ et -H₈); 7,82 et 8,80 (2s larges, 1H chacun :-H de l'imidazole); 7,38 et 8,02 (2d, J = 7,5 Hz, 1H chacun : -H₆ et -H₉); 11,9 (mf, 1 H : -NH-)].

### EXEMPLE 13

Un mélange de 0,4 g 10-benzylidène-5H,10H-imidazo[1,2-a]indéno[1,2-e] pyrazine-4-one, 90 ml de diméthylformamide et 10 ml de méthanol est hydrogéné à une température voisine de 20°C sous une pression de 1,9 bar d'hydrogène pendant 3 heures en présence de charbon palladié à 10%. On filtre alors le catalyseur sous atmosphère inerte et on évapore les solvants. Le solide brun obtenu (0,37 g) est purifié par chromatographie sur colonne de silice (35 g) avec un mélange de dichlorométhane et de méthanol (90/10 en volumes). On obtient, après trituration avec 5 ml de dichlorométhane, filtration et séchage à 100°C, 0,15 g de 10-benzyl-5H,10H-imidazo[1,2-a] indéno[1,2-e]pyrazine-4-one sous forme de solide beige fondant au-dessus de 260°C (Analyse % calculé C : 76,66, H : 4,82, N : 13,41, O : 5,11, % trouvé C : 76,3, H : 4,6, N : 13,4).

### EXEMPLE 14

On agite à une température voisine de 20°C une suspension de 5,75 g de 5H,10H-imidazo[1,2-a]indéno[1,2-e]pyrazine-4-one, 10 g de soude perlée et 125 mg de bromure de tétrabutylammonium dans 200 ml de diméthylsulfoxyde. On ajoute ensuite 3,18 g de benzaldéhyde et maintient l'agitation pendant 18 heures à une température voisine de 20°C. Le mélange réactionnel est versé dans un mélange d'eau et de glace (250 ml) et acidifié avec 100 ml d'acide acétique. Le solide formé est filtré et le produit brut est purifié par deux chromatographies successives sur colonne de silice avec un mélange de dichlorométhane et de méthanol (90/10 en volumes). On obtient ainsi 0,13 g de 10-benzylidène-5H,10H-imidazo[1,2-a]indéno[1,2-e]pyrazine-4-one sous forme de solide jaune fondant au-dessus de 260°C (Analyse % calculé C : 77,16, H : 4,21, N : 13,50, O : 5,14, % trouvé C : 76,7, H : 3,7, N : 13,2).

### EXEMPLE 15

A une suspension de 3 g de 10-hydroxyméthylène-5H,10H-imidazo[1,2-a]indéno[1,2-e]pyrazine-4-one (mélange des formes Z et E) dans 240 ml de méthanol, on ajoute sous agitation en 10 minutes et par portions 1 g de borohydrure de sodium, et on poursuit l'agitation à une température voisine de 20°C pendant 1 heure et 30 minutes. On ajoute à nouveau 1 g de borohydrure de sodium par portions et on maintient l'agitation pendant 30 minutes. Le mélange réactionnel est alors filtré et le filtre est rincé avec 2 fois 30 ml de méthanol. L'insoluble est repris avec 50 ml d'eau et 4 ml d'acide chlorhydrique 1N, filtré à nouveau, lavé à l'eau jusqu'à neutralité et séché à l'air. Le produit brut (1 g) est purifié par dissolution à chaud dans 65 ml de diméthylformamide, filtration, addition au filtrat encore chaud de 80 ml de méthanol et cristallisation dans un bain d'eau et de glace. Les cristaux obtenus sont filtrés, lavés avec 2 fois 15 ml de méthanol et séchés à 80°C. On obtient ainsi 0,66 g de 10-hydroxyméthyl-5H,10H-imidazo[1,2-a] indéno[1,2-e]pyrazine-4-one sous forme de solide blanc fondant au-dessus de 260°C (Analyse % calculé C : 66,40, H : 4,38, N : 16,59, O : 12,63, %trouvé C : 66,4, H : 4,3, N : 16,6, O : 12,1).

### EXEMPLE 16

On hydrogène 2,8 g d'un mélange de 10-(2-méthyl-propène-1-yl)-5H,10H-imidazo[1,2]indéno[1,2-e]pyrazine-4-one et de 10-(2-méthyl-propylidène)-5H,10H-imidazo[1,2-a]indéno[1,2-e]pyrazine-4-one en présence de 180 ml de diméthylformamide, 20 ml de méthanol et de 0,5 g de charbon palladié à 10 % pendant 22 heures à 50°C sous une pression de 54 bar d'hydrogène. Après filtration du catalyseur sous atmosphère inerte, les solvants sont évaporés et le solide gris obtenu (2,1 g) est purifié par chromatographie sur colonne de silice (180 g partiellement désactivés avec 3 ml d'eau) en utilisant comme éluant un mélange de dichlorométhane et de méthanol (90/10 en volumes). On obtient, après trituration dans 20 ml d'éther éthylique, filtration et séchage à 100°C, 1,2 g de 10-isobutyl-5H,10H-imidazo[1,2-a]indéno[1,2-e] pyrazine-4-one sous forme de solide crème fondant au-dessus de 260°C (Analyse % calculé C : 73,10 H : 6,13 N : 15,04 O : 5,73, % trouvé C : 72,9 H : 5,6, N:14,8).

Le mélange de 10-(2-méthyl-propène-1-yl)-5H,10H-imidazo[1,2-a]indéno[1,2-e]pyrazine-4-one et de 10-(2-méthyl-propylidène)-5H, 10H-imidazo[1,2-a]indéno[1,2-e]pyrazine-4-one peut être préparé de la façon suivante : on opère comme à l'exemple 14 mais à partir de 6 g de 5H,10H-imidazo[1,2-a]indéno[1,2-e]pyrazine-4-one, 250 ml de diméthylsulfoxyde, 10 g de soude perlée, 125 mg de bromure de tétrabutylammonium et de 2,16 g d'isobutyraldéhyde. Le produit brut (6,26 g) est dissous dans 100 ml de diméthylsulfoxyde et décoloré à 60°C avec 0,6 g de noir 3S. Après filtration, le filtrat est additionné de 250 ml d'eau distillée et le précipité formé est essoré et séché à 80°C. On obtient ainsi 4,3 g d'un mélange de 10-(2-méthyl-propène-1-yl)-5H,10H-imidazo[1,2-a]indéno[1,2-e]pyrazine-4-one et de 10-(2-méthyl-propylidène)-5H,10H-imidazo[1,2-a]indéno[1,2-e] pyrazine-4-one sous forme d'un solide vert utilisé tel quel dans les synthèses ultérieures.

### EXEMPLE 17

Un mélange de 10-(2-furylméthylène)-5H,10H-imidazo[1,2-a]indéno[1,2-e] pyrazine-4-one, 100 ml de diméthylformamide et 0,15 g de charbon palladié à 10% est hydrogéné par balayage d'un courant d'hydrogène à une température voisine de 20°C pendant 2 heures. On filtre alors le catalyseur sous atmosphère inerte et on évapore le solvant. Le solide brun obtenu (2 g) est purifié par trituration dans 50 ml d'acétone, filtration et cristallisation de l'insoluble obtenu (1 g) dans 80 ml de méthanol. Après filtration et séchage à 80°C, on obtient 0,48 g de 10-(2-furylméthyl)-5H,10H-imidazo[1,2-a] indéno[1,2-e]pyrazine-4-one sous forme de solide brun clair fondant au-dessus de 260°C (Analyse % calculé C : 71,28, H : 4,32, N : 13,85, O : 10,55 % trouvé C : 71,5, H : 4,2, N : 14,0).

La 10-(2-furylméthylène)-5H,10H-imidazo[1,2-a]indéno[1,2-e]pyrazine-4-one peut être préparée de la manière suivante : à une solution refroidie à 18°C de 2 g de 5H,10H-imidazo[1,2-a]indéno[1,2-e]pyrazine-4-one dans 30 ml de diméthylsulfoxyde on ajoute sous agitation et par portions 0,64 g d'hydrure de sodium. L'agitation est maintenue pendant 35 minutes à une température voisine de 20°C et on ajoute ensuite, goutte à goutte, une solution de 0,83 ml de 2-furaldéhyde dans 10 ml de diméthylsulfoxyde. On maintient l'agitation pendant 2 heures. Le mélange réactionnel est alors versé dans un mélange d'eau et de glace (200 ml) et acidifié à pH 4 avec de l'acide acétique. La suspension colorée obtenue est centrifugée et le solide ainsi isolé est trituré dans 50 ml d'acétone, filtré et séché à l'air pour donner 2,17 g de 10-(2-furylméthylène)-5H,10H-imidazo[1,2-a]indéno[1,2-e]pyrazine-4-one fondant au-dessus de 260°C sous forme d'un solide ocre utilisé tel quel dans les synthèses ultérieures.

### EXEMPLE 18

On opère comme à l'exemple 13 mais à partir de 3,9 g de 10-(phényl propylidène)-5H,10H-imidazo[1,2-a]indéno[1,2-e]pyrazine-4-one, 180 ml de diméthylformamide, 20 ml de méthanol et 0,5 g de charbon palladié à 10%. Le produit brut (3,8 g) est purifié par chromatographie sur colonne de silice (380 g partiellement désactivés avec 10 ml d'eau) en éluant avec un mélange de dichlorométhane et de méthanol (90/10 en volumes). On obtient, après trituration dans 10 ml de dichlorométhane, filtration et séchage à 100°C, 1,1 g de 10-(phénylpropyl)-5H, 10H-imidazo[1,2-a]indéno[1,2-e]pyrazine-4one sous forme de solide beige fondant à 232°C.

La 10-(phénylpropylidène)-5H, 10H-imidazo[1,2-a]indéno[1,2-e]pyrazine-4one peut être préparée de la façon suivante : on opère comme à l'exemple 14 mais à partir de 6 g de 5H,10H-imidazo[1,2-a]indéno[1,2-e]pyrazine-4one, 240 ml de diméthylsulfoxyde, 10 g de soude perlée, 125 mg de bromure de tétrabutylammonium et 4,0 g de phénylpropionaldéhyde. Le produit brut (7,2g) est purifié par cristallisation dans 140 ml de diméthylsulfoxyde et 140 ml d'eau pour donner 5,5 g de 10-(phénylpropylidène)-5H,10H-imidazo[1,2-a]indéno[1,2-e]pyrazine-4-one sous forme de solide jaune utilisé tel quel dans les synthèses ultérieures.

### EXEMPLE 19

On hydrogène à une température voisine de 20°C sous une pression de 9,4 bar d'hydrogène, 1,3 g de 10-hexylidène-5H,10H-imidazo[1,2-a] indéno[1,2-e]pyrazine-4-one en présence de 120 ml de diméthylformamide, 20 ml de méthanol et 0,3 g de charbon palladié à 10% pendant 17 heures. Après filtration du catalyseur sous atmosphère inerte, les solvants sont évaporés et le produit brut (1,2 g) est purifié par chromatographie sur colonne de silice (120 g partiellement désactivés avec 2 ml d'eau) en éluant avec un mélange de dichlorométhane et de méthanol (90/10 en volumes). On obtient 0,47 g de 10-hexyl-5H,10H-imidazo[1,2-a]indéno[1,2-e]pyrazine-4-one sous forme de solide beige fondant vers 185°C (Analyse % calculé C : 74,24, H : 6,89, N : 13,67, O : 5,20, % trouvé C : 74,3, H : 7,0, N : 13,6).

La 10-hexylidène-5H,10H-imidazo[1,2-a]indéno[1,2-e]pyrazine-4-one peut être préparée de la manière suivante : on opère comme à l'exemple 17 pour la préparation de la 10-(2-furylméthylène)-5H,10H-imidazo[1,2-a] indéno[1,2-e]pyrazine-4-one mais à partir de 2,23 g de 5H,10H-imidazo[1,2-a]indéno[1,2-e]pyrazine-4-one, 50 ml de diméthylsulfoxyde, 0,72 g d'hydrure de sodium et 1,1 g d'hexanal. Le produit brut est purifié par cristallisation dans 120 ml de diméthylsulfoxyde et 120 ml d'eau pour donner 1,3 g de 10-hexylidène-5H,10H-imidazo[1,2-a]indéno[1,2-e]pyrazine-4-one sous forme de solide jaune utilisé tel quel dans les synthèses ultérieures.

### EXEMPLE 20

On opère comme à l'exemple 17 pour la préparation de la 10-(2-furylméthylène)-5H,10H-imidazo[1,2-a]indéno[1,2-e]pyrazine-4-one mais à partir de 2 g de 5H,10H-imidazo[1,2-a]indéno[1,2-e]pyrazine-4-one, 40 ml de diméthylsulfoxyde, 0,64 g d'hydrure de sodium et 1,07 g de 4-pyridine carboxaldéhyde. Le produt brut (1,5 g) est purifié par cristallisation dans 100 ml de diméthylformamide et 50 ml de méthanol pour donner 1,06 g de 10-(4-pyridylméthylène)-5H,10H-imidazo[1,2-a]indéno[1,2-e]pyrazine-4-one sous forme de solide orange fondant au-dessus de 260°C (Analyse % calculé C : 73,07, H : 3,87, N : 17,94, O : 5,12, % trouvé C : 72,9, H : 3,7, N : 18,2).

### EXEMPLE 21

On opère comme à l'exemple 13 mais à partir de 0,8 g de 10-(4-pyridylméthylène)-5H, 10H-imidazo[1,2-a]indéno[1,2-e]pyrazine-4-one, 250 ml de diméthylformamide et 80 mg de charbon palladié à 10% et en ajoutant en plus 10 ml d'acide acétique. On obtient 0,2 g de 10-(4-pyridylméthyl)-5H,10H-imidazo[1,2-a]indéno[1,2-e]pyrazine-4-one sous forme de solide jaune pâle fondant au-dessus de 260°C (Analyse % calculé C : 72,60, H : 4,49, N : 17,82, O : 5,09, % trouvé C : 72,3, H : 4,3, N : 17,8).

### EXEMPLE 22

On opère comme à l'exemple 17 pour la préparation de la 10-(2-furylméthylène)-5H,10H-imidazo[1,2-a]indéno[1,2-e]pyrazine-4-one mais à partir de 3 g de 5H,10H-imidazo[1,2-a]indéno[1,2-e]pyrazine-4-one, 60 ml de diméthylsulfoxyde, 0,96 g d'hydrure de sodium et 1,6 g de 3-pyridinecarboxaldéhyde. On obtient 1,38 g de 10-(3-pyridylméthylène)5H,10H-imidazo[1,2-a]indéno[1,2-e]pyrazine-4-one sous forme de solide jaune orangé fondant au-dessus de 260°C (Analyse % calculé C : 73,07, H : 3,87, N : 17,94, O : 5,12, % trouvé C : 73,1, H : 3,9, N : 17,9, O : 5,1).

### EXEMPLE 23

On opère comme à l'exemple 21 mais partir de 1 g de 10-(3-pyridylméthylène)-5H,10H-imidazo[1,2-a]indéno[1,2-e]pyrazine-4-one, 150 ml de diméthylformamide, 5 ml d'acide acétique et 0,1 g de charbon palladié à 10%. On obtient 0,38 g de 10-(3-pyridylméthyl)-5H,10H-imidazo[1,2-a]indéno[1,2-e] pyrazine-4-one sous forme de solide blanc cassé fondant au-dessus de 260°C (Analyse % calculé C : 72,60, H : 4,49, N : 17,82, O : 5,09, % trouvé C : 72,3, H : 4,8, N:17,8, 0:5,4).

### EXEMPLE 24

On opère comme à l'exemple 21 mais à partir de 0,7 g de 10-(2-pyridylméthylène)-5H,10H-imidazo[1,2-a]indéno[1,2-e]pyrazine-4-one, 200 ml de diméthylformamide, 5 ml d'acide acétique et 70 mg de charbon palladié à 10%. Le produit brut (1,1 g) est purifié par trituration dans un mélange de 10 ml de dichlorométhane et 40 ml d'éther éthylique, puis par cristallisation dans 25 ml de diméthylformamide et 40 ml d'eau. On obtient 0,35 g de 10-(2-pyridylméthyl)-5H,10H-imidazo[1,2-a]indéno[1,2-e]pyrazine-4-one sous forme de solide beige clair fondant au-dessus de 260°C (Analyse % calculé C : 72,60, H : 4,49, N : 17,82, O : 5,09, % trouvé C : 72,5, H : 4,6, N : 17,9, O : 5,5).

La 10-(2-pyridyîméthyléne)-5H, 10H-imidazo[1,2-a]indéno[1,2-e]pyrazine-4one peut être préparée de la façon suivante: on opère comme à l'exemple 17 pour la préparation de la 10-(2-furylméthylène)-5H,10H-imidazo[1,2-a] indéno[1,2-e]pyrazine-4-one mais à partir de 3 g de 5H,10H-imidazo[1,2-a] indéno[1,2-e]pyrazine-4-one, 60 ml de diméthylsulfoxyde, 0,96 g d'hydrure de sodium et 1,6 g de 2-pyridinecarboxaldéhyde. On obtient 0,7 g de 10-(2-pyridylméthylène)-5H,10H-imidazo[1,2-a]indéno[1,2-e]pyrazine-4-one sous forme de solide orange utilisé tel quel dans les synthèses ultérieures.

### EXEMPLE 25

On opère comme à l'exemple 21 mais à partir de 1,65 g de 10-(2-imidazolylméthylène)-5H, 10H-imidazo[1,2-a]indéno[1,2-e]pyrazine-4one, 300 ml de diméthylformamide, 5 ml d'acide acétique et 0,17 g de charbon palladié à 10%. On obtient 0,5 g de 10-(2-imidazolylméthyl)-5H,10H-imidazo[1,2-a]indéno[1,2-e]pyrazine-4-one sous forme de solide jaune pâle fondant au-dessus de 260°C (Analyse % calculé C : 67,32, H : 4,32, N : 23,09, O : 5,27, % trouvé C : 67,2, H : 4,3, N : 23,0).

La 10-(2-imidazolylméthylène)-5H,10H-imidazo[1,2-a]indéno[1,2-e]pyrazine-4-one peut être préparée de la manière suivante : on opère comme à l'exemple 17 pour la préparation de la 10-(2-furylméthylène)-5H,10H-imidazo[1,2-a]indéno[1,2-e]pyrazine-4-one mais à partir de 3 g de 5H,10H-imidazo[1,2-a]indéno[1,2-e]pyrazine-4-one, 70 ml de diméthylsulfoxyde, 1,4 g d'hydrure de sodium et 1,44 g de 2-imidazolecarboxaldéhyde. On obtient 1,65 g de 10-(2-imidazolylméthylène)-5H,10H-imidazo[1,2-a]indéno[1,2-e] pyrazine-4-one sous forme de solide vert foncé utilisé tel quel dans les synthèses ultérieures.

### EXEMPLE 26

1,8 g d'hydrure de sodium est ajouté à 20°C à une solution maintenue sous atmosphère d'azote de 7,4 g de 10-(hydroxyimino)-5H,10H-imidazo[1,2-a] indéno[1,2-e]pyrazine-4-one (mélange 85-15 des formes E et Z) (produit brut avant purification obtenu dans l'exemple 1) dans 125 ml de diméthylsulfoxyde anhydre. Après 30 minutes d'agitation, une solution de 5,4 g de bromoacétate de t-butyle dans 10 ml de diméthylsulfoxyde anhydre est ajoutée goutte à goutte en 5 minutes à la même température. Le mélange est agité pendant 1 heure, versé sur 1200 g au total de glace et d'eau distillée et centrifugé. Après élimination de la solution surnageante, l'insoluble est isolé par filtration, lavé avec 50 ml d'eau distillée et 2 fois avec 125 ml au total de méthanol puis séché à l'air. Le produit obtenu (6,4 g) est dissous à 20°C dans 800 ml de diméthylformamide et la solution, additionnée de noir décolorant, est filtrée. Le filtre est lavé avec 100 ml de diméthylformamide puis le filtrat et le lavage sont réunis et versés sur 1800 ml d'eau distillée. Après centrifugation, filtration de l'insoluble, lavage avec 20 ml d'eau distillée et 20 ml de méthanol puis séchage sous pression réduite (1 mm Hg; 0,13 kPa) à 100°C, on obtient 5 g de 10-(4-oxo-5H,10H-imidazo[1,2-a]indéno[1,2-e]pyrazi nylidène)aminooxyacétate de tert-butyle fondant à 305°C (décomposition) [Spectre de R.M.N. : (300 MHz; DMSO d6; δ en ppm) : mélange d'isomères : 85% d'isomère E, 15% d'isomère Z : 1,47 [s, 9H : -C(CH₃)₃]; 4,97 et 5,02 (2s, 2H en totalité, =N-OCH₂ de l'isomère E et Z respectivement); de 7,30 à 7,50 (mt, 2H : -H7 et -H8); 7,60 (s, 1H: -H de l'imidazole); de 7,70 à 7,90 et 8,12 [mt et (d, J=7,5 Hz), 1H chacun : -H6 et -H9]; 7,94 et 8,52 (2s, 1H en totalité : l'autre -H de l'imidazole respectivement de l'isomère E et Z); 12,77 (mf, 1H :-CO-NH-)].

### EXEMPLE 27

1,7 g de chlorure de benzoyle est ajouté en 1 minute à une solution de 3,1 g de chlorhydrate de 10-amino-5H,10H-imidazo[1,2-a]indéno[1,2-e]pyrazine-4-one dans 200 ml de soude 0,1N. Le mélange est agité pendant 2 heures à une température voisine de 20°C et l'insoluble est séparé par filtration, lavé 2 fois avec 200 ml au total d'eau distillée et séché à l'air. Le produit obtenu (3 g) est dissous dans 100 ml de diméthylsulfoxyde et la solution, additionnée de noir décolorant, est filtrée. Le filtre est lavé avec 20 ml de diméthylsulfoxyde puis le filtrat et le lavage sont réunis et additionnés à 20°C de 80 ml d'eau distillée. L'insoluble est séparé par filtration, lavé 2 fois avec 50 ml au total d'eau distillée et avec 25 ml d'acétone puis séché sous pression réduite (1 mm Hg; 0,13 kPa) à 100°C. On obtient ainsi 1,75 g de 10-benzamido-5H,10H-imidazo[1,2-a]indéno[1,2-e]pyrazine-4-one se décomposant sans fondre à 300°C [Spectre de R.M.N. : (200 MHz; DMSO d6; δ en ppm) : 6,40 (d, J=8,5 Hz, 1H: CH-N); de 7,30 à 7,70 (mt, 8H : -H6, -H7, -H8,-H9, -H aromatiques en para et méta du -CO-NH- et -H de l'imidazole ); de 7,80 à 8,00 (mt, 3H : -H aromatiques en ortho du -CO-NH- et -H de l'imidazole); 9,14 (d, J=8,5 Hz, 1H : -NH-); 12,55 (mf, 1H : -CO-NH- du cycle)].

### EXEMPLE 28

A une suspension de 4 g de 5H,10H-imidazo[1,2-a]indéno[1,2-e]pyrazine-4-one dans 70 ml de diméthylsulfoxyde on ajoute sous agitation et couverture d'azote 3,9 g de 2-pyrazinecarboxaldéhyde, refroidit à 19°C et ajoute par portions 1,6 g d'hydrure de sodium à 60% en maintenant le milieu réactionnel à une température comprise entre 15 et 21°C. L'agitation est poursuivie pendant 4 heures, puis on ajoute goutte à goutte à une température voisine de 20°C successivement 70 ml d'eau, 5 ml d'acide acétique et 50 ml de méthanol. La suspension obtenue est filtrée, le solide ainsi isolé est lavé avec du méthanol, séché à l'air et trituré dans un mélange de 80 ml de diméthylformamide et 25 ml de méthanol. Après séchage à 60°C sous vide (1 mmHg; 0,13 kPa) on obtient 3,3 g de 10-(2-pyrazinylméthylène)-5H,10H-imidazo[1,2-a]indéno[1,2-e]pyrazine-4-one sous forme de solide rouge orangé fondant au-dessus de 260°C (Analyse % calculé C : 69,00, H : 3,54, N : 22,35, O : 5,11, % trouvé C : 69,6, H : 3,6, N : 22,0, O : 4,9).

Le 2-pyrazinecarboxaldéhyde peut être préparé selon le procédé décrit par H. RUTNER et P.E. SPOERRI, J. Org. Chem., 28, 1898 (1963).

### EXEMPLE 29

Un mélange de 3,2 g de 10-(2-pyrazinylméthylène)-5H,10H-imidazo[1,2-a] indéno[1,2-e]pyrazine-4-one, 250 ml de diméthylformamide, 50 ml de méthanol et 5 ml d'acide acétique est hydrogéné à une température voisine de 20°C sous une pression de 1,8 bar d'hydrogène, en présence de charbon palladié à 10% pendant 18 heures. Le catalyseur filtré sous atmosphère inerte et le filtrat est concentré à l'évaporateur rotatif. Par addition de 200 ml de méthanol on forme un précipité qui, après trituration dans le dichlorométhane (100 ml), filtration et séchage à 60°C sous vide (1 mmHg; 0,13 kPa), fournit 1,4 g de 1.0-(2-pyrazinylméthyl)-5H,10H-imidazo[1,2-a]indéno[1,2-e] pyrazine-4-one sous forme de solide beige fondant au-dessus de 260°C (Analyse % calculé C : 68,56, H : 4,16, N : 22,21, O : 5,07, % trouvé C : 68,3, H : 3,9, N : 21,9).

### EXEMPLE 30

On opère comme à l'exemple 28 mais à partir de 3 g de 5H,10H-imidazo[1,2-a]indéno[1,2-g]pyrazine-4-one, 60 ml de diméthylsulfoxyde, 0,96 g d'hydrure de sodium à 80% et 3,18 g de 4-quinoléinecarboxaldéhyde. Après traitement à l'eau et à l'acide acétique, la suspension obtenue est filtrée et le solide ainsi isolé est lavé avec 2x30 ml d'eau, puis 4x30 ml d'acétone et séché à 100°C sous vide (2 mmHg; 0,26 kPa). On obtient 3,9 g de 10-(4-quinolinylméthylène)-5H,10H-imidazo[1 ,2-a]indéno[1,2-e]pyrazine-4-one sous forme de solide orange fondant au-dessus de 260°C (Analyse % calculé C : 76,23, H : 3,89, N : 15,46, O : 4,41, % trouvé C : 75,9, H : 4,2, N : 15,4).

### EXEMPLE 31

On opère comme à l'exemple 29 mais à partir de 3 g de 10-(4-quinolinylméthylène)-5H,10H-imidazo[1,2-a]indéno[1,2-e]pyrazine-4-one, 130 ml de diméthylformamide, 130 ml d'acide acétique et 0,3 g de charbon palladié à 10% sous une pression de 15 bar d'hydrogène pendant 22 heures. Après élimination du catalyseur et des solvants, on obtient un solide jaune brun (3,1 g) que l'on traite avec un mélange d'acide acétique et de diméthylformamide et filtre. Le filtrat est additionné de 7 g de silice, concentré à l'évaporateur rotatif et purifié par chromatographie sur colonne de silice (250 g partiellement désactivés avec 2% d'eau) en éluant avec un mélange de dichlorométhane et de méthanol (90/10 en volumes). Après séchage à 90°C sous vide (2 mmHg; 0,26 kPa), on obtient 0,97 g de 10-(4-quinolinylméthyl)-5H,10H-imidazo[1,2-a]indéno[1,2-e]pyrazine-4-one sous forme de solide jaune fondant au-dessus de 260°C (Analyse % calculé C:75,81, H : 4,43, N : 15,37, O : 4,39, % trouvé C : 75,4, H : 3,6, N : 15,2).

### EXEMPLE 32

Un mélange de 1,5 g de 10-(4-pyridylméthyl)-5H,10H-imidazo[1,2-a]indéno[1,2-e]pyrazine-4-one, 100 ml d'acide acétique, 5 ml d'acide chlorhydrique 1N et 0,19 g d'oxyde de platine est hydrogéné sous une pression de 56 bar pendant 18 heures à 50°C. Après élimination du catalyseur et du solvant on obtient un solide beige (1,5 g) auquel on ajoute 100 ml d'eau; la suspension obtenue est lavée avec 50 ml de dichlorométhane, alcalinisée à pH 8 avec une solution saturée d'hydrogénocarbonate de sodium, lavée de nouveau avec 2 fois 50 ml de dichlorométhane et concentrée à l'évaporateur rotatif. Au résidu obtenu on ajoute 250 ml de diméthylformamide, 20 ml d'eau et 3 g de silice et on concentre à l'évaporateur rotatif. Le résidu d'évaporation est purifié par chromatographie sur colonne de silice (150 g partiellement désactivés avec 3% d'eau) en éluant avec un mélange de chloroforme, de méthanol et d'ammoniaque à 28% (24/6/1 en volumes). On obtient 0,18 g de 10-(4-pipéridylméthyî)-5H 10H-imidazo[1,2-a]indéno[1,2-e] pyrazine-4-one sous forme de solide orange [Spectre de RMN: (300 MHz; (CD₃)₂SO d6 avec quelques gouttes de CD₃COOD d4; δ en ppm): de 1,10 à 1,50 (mt, 5H : CH₂ et CH de la pipéridine); 2,05 à 2,35 (mt, 2H : CH₂); 2,65 (mt, 2H : NCH₂ de la pipéridine H axiaux); 3,05 (d large, J= 12 Hz, 2H : NCH₂ de la pipéridine H équatoriaux); 4,30 (dd, J= 9 et 3 Hz, 1H: H 10); 7,30 et 7,40 (2t, J= 8,5 Hz, 2H : H 7 et H 8); 7,62 et 7,88 (2d larges, J= 8.5 Hz, 1H chacun : H 6 et H 9); 7,58 et 8,02 (2s larges, 1H chacun : -H de l'imidazole)].

### EXEMPLE 33

A une suspension refroidie à 0°C de 0,56 g de 10-carboxyméthyl-5H,10H-imidazo[1,2-a]indéno[1,2-e]pyrazine-4-one et de 20 ml de chloroforme on ajoute, sous agitation et sous couverture d'argon, 0,21 ml de chlorure d'oxalyle, puis un mélange de 5 ml de chloroforme et 0,5 ml de diméthylformamide. On maintient l'agitation pendant 4 heures à une température voisine de 20°C et on ajoute très lentement une solution de 0,38 g de borohydrure de sodium dans 20 ml de diméthylformamide en maintenant la température proche de 20°C. Le mélange réactionnel est versé dans 10 ml d'eau et laissé au repos pendant la nuit. La phase organique est décantée et évaporée pour donner 0,4 g de produit jaune (fraction A); la phase aqueuse est acidifiée à pH 1 avec de l'acide chlorhydrique 0,1N et le précipité formé est filtré, lavé à l'acétone et séché à l'air pour donner 0,2 g de produit blanc (fraction B). Les fractions A et B sont réunies, dissoutes dans un mélange de méthanol et de diméthylformamide et purifiées par chromatographie sur colonne de silice (30 g) en éluant avec un mélange de dichlorométhane et de méthanol (95/5 en volumes). Après séchage à 60°C sous vide (1 mmHg; 0,13 kPa), on obtient 0,1 g de 10-(2-hydroxyéthyl)-5H,10H-imidazo[1,2-a]indéno[1,2-e] pyrazine-4-one sous forme de solide blanc cassé fondant au-dessus de 260°C (Analyse % calculé C : 67,41, H : 4,90, N : 15,72, O : 11,97, % trouvé C : 67,6, H : 4,9, N:15,3).

La 10-carboxyméthyl-5H,10H-imidazo[1,2-a]indéno[1,2-e]pyrazine-4-one peut être préparée selon le procédé suivant : on agite pendant 107 heures à une température voisine de 20°C sous couverture d'argon un mélange de 1 g de 10-(tert-butoxycarbonylméthyl)-5H,10H-imidazo[1,2-a]indéno[1,2-e] pyrazine-4-one, 5 ml de diméthylsulfoxyde et 25 ml d'acide chlorhydrique 3N dans l'éther éthylique. Après concentration sous pression réduite le résidu est cristallisé dans 30 ml d'acétone. Le solide obtenu est traité avec 50 ml d'eau et 7 ml d'une solution saturée d'hydrogénocarbonate de sodium. On extrait au dichlorométhane (3 fois 50 ml), filtre la phase aqueuse et acidifie la solution aqueuse avec 8 ml d'acide chlorhydrique 1N. Le précipité formé est filtré, lavé à l'eau et séché à 80°C sous vide (1 mmHg; 0,13 kPa) pour donner 0,35 g de 10-(carboxyméthyl)-5H,10H-imidazo[1,2-a]indéno [1,2-e]pyrazine-4-one sous forme de solide verdâtre fondant au-dessus de 260°C (Analyse % calculé C : 64,05, H :3,94, N : 14,94, O : 17,06, % trouvé C : 63,7, H : 3,2, N:14,9).

La 10-(tert-butoxycarbonylméthyl)-5H, 10H-imidazo[1,2-a]indéno[1,2-e]pyrazine-4-one peut être préparée de la manière suivante : une suspension de 1,78 g de 10-(tert-butoxycarbonylméthylène)-5H,10H-imidazo[1,2-a]indéno[1,2-e]pyrazine-4-one dans 280 ml de diméthylformamide et 20 ml de méthanol est hydrogénée en présence de 0,18 g de charbon palladié à 10% pendant 20 heures sous 10 bar d'hydrogène à une température voisine de 20°C. Après évaporation des solvants le solide beige obtenu est purifié par chromatographie sur colonne de silice (180 g partiellement désactivés avec 2% d'eau) en éluant avec un mélange de dichlorométhane et de méthanol (95/5 en volumes). On obtient un solide blanc que l'on triture dans 10 ml de méthyltertiobutyléther, filtre et sèche à 80°C sous vide (2 mmHg; 0,26 kPa) pour donner 0,35 g de 10-(tert-butoxycarbonylméthyl)-5H,10H-imidazo[1,2-a]indéno[1,2-e]pyrazine-4-one (Analyse % calculé C : 67,64, H : 5,68, N : 12,45, O : 14,23, % trouvé C : 67,2, H : 6,0, N : 12,4, O : 14,5).

La 10-(tert-butoxycarbonylméthylène)-5H,10H-imidazo[1,2-a]indéno[1,2-e] pyrazine-4-one : sous couverture d'azote, à une solution refroidie à 19°C de 9 g de 5H,10H-imidazo[1,2-a]indéno[1,2-e]pyrazine-4-one dans 180 ml de diméthylsulfoxyde on ajoute sous agitation et par portions 4 g d'hydrure de sodium à 60%. L'agitation est maintenue pendant 35 minutes et on ajoute goutte à goutte 7,8 g de glyoxylate de tertiobutyle. Après 18 heures d'agitation le mélange réactionnel est traité avec 9 ml d'acide acétique et 150 ml d'eau. La suspension est filtrée, l'insoluble lavé à l'eau (2x200 ml), puis à l'acétone (3x100 ml) et séché à 80°C sous pression réduite. Le produit brut obtenu (5,85 g) est chauffé vers 90-10°C dans 300 ml de diméthylformamide, filtré aussitôt et le filtrat est additionné de 150 ml de méthanol et 200 ml d'eau et laissé à la glacière pendant une nuit. Les cristaux formés sont filtrés, lavés avec 20 ml d'eau et séchés à 80°C sous vide (2 mmHg; 0,26 kPa) pour donner 3,4 g de 10-(tert-butoxycarbonylméthylène)-5H,10H-imidazo[1,2-a]indéno[1,2-e]pyrazine-4-one sous forme de solide rouge fondant au-dessus de 260°C (Analyse % calculé C : 68,05, H : 5,11, N : 12,53, O : 14,31, % trouvé C : 68,0, H : 5,6, N : 12,5, O : 14,3).

Le glyoxylate de tertiobutyle peut être préparé selon le procédé décrit par L.A. CARPINO, J. Org. Chem., 29, 2820 (1964).

### EXEMPLE 34

A un mélange agité sous couverture d'argon de 0,48 g de 10-méthyl-5H,10H-imidazo[1,2-a]indéno[1,2-e]pyrazine-4-one, 5 ml de diméthylsulfoxyde et 0,14 g d'hydrure de sodium à 80% on ajoute 0,23 ml de chlorure de benzyle. L'agitation est maintenue pendant une heure, puis le mélange réactionnel est acidifié avec 0,5 ml d'acide acétique et versé sur 25 ml d'eau glacée. Un précipité rose pâle apparaît, qui est filtré, lavé avec 3x10 ml d'eau et séché à l'air. Le produit brut est purifié par chromatographie sur colonne de silice (25 g) en éluant avec un mélange de dichlorométhane et de méthanol (95/5 en volumes). Après séchahe à 60°C sous vide (1 mmHg; 0,13 kPa) on obtient 0,29 g de 10-benzyl-10-méthyl-5H,10H-imidazo[1,2-a] indéno[1,2-e]pyrazine-4-one sous forme de solide rose pâle fondant au-dessus de 260°C (Analyse % calculé C : 77,04, H : 5,23, N : 12,84, O : 4,89, % trouvé C : 76,8, H : 5,8, N : 12,8).

### EXEMPLE 35

A un mélange agité à une température voisine de 20°C de 0,48 g de 10-méthyl-5H,10H-imidazo[1,2-a]indéno[1,2-e]pyrazine-4-one, 25 ml de diméthylformamide et 0,33 g d'hydrure de sodium à 80% on ajoute sous couverture d'argon 0,3 ml de triméthylchlorosilane. L'agitation est maintenue pendant une heure, puis on ajoute 0,07 g d'hydrure de sodium à 80% et on continue à agiter pendant 30 minutes. On ajoute alors 0,36 g de chlorhydrate de 4-chlorométhylpyridine et on maintient l'agitation pendant 20 minutes. Le mélange réactionnel est additionné de 2 ml d'acide acétique, versé sur 150 ml d'eau glacée et concentré à l'évaporateur rotatif. Le résidu d'évaporation est traité avec 200 ml d'acétate d'éthyle, filtré et le filtrat évaporé. La pâte jaune orangée obtenue est purifiée par chromatographie sur colonne de silice (30 g) en éluant avec un mélange de dichlorométhane et de méthanol (95/5 en volumes). Après trituration dans 40 ml d'éther éthylique, filtration et séchage à 60°C sous vide (1 mmHg; 0,13 kPa), on obtient 0,18 g de 10-méthyl-10-(4-pyridylméthyl)-5H, 10H-imidazo[1,2-a]indéno[1,2-e]pyrazine-4-one sous forme de solide crème fondant au-dessus de 260°C (Analyse % calculé C : 73,15, H : 4,91, N : 17,06, O : 4,87, % trouvé C : 73,2, H : 5,1, N : 17,0).

### EXEMPLE 36

Une solution de 3,3 g de 10-(4-oxo-5H,10H-imidazo[1,2-a]indéno[1,2-e]pyrazinylidène)aminooxyacétate de tert-butyle dans 60 ml d'acide trifluoroacétique est maintenue à 60°C pendant 1 heure et 30 minutes, refoidie à 20°C et concentrée à sec sous pression réduite (15 mm Hg; 2 kPa) à 60°C. Le produit obtenu (5,7 g) est dissous dans 190 ml d'une solution aqueuse saturée d'hydrogénocarbonate de sodium et la solution est filtrée, extaite 2 fois avec 200 ml au total de dichlorométhane, acidifiée avec 15 ml d'acide acétique et agitée pendant 16 heures à une température voisine de 20°C. Le solide apparu est séparé par filtration, lavé avec 25 ml d'eau distillée et séché à l'air. Le produit obtenu (2,45 g) est dissous dans 300 ml de diméthylsulfoxyde et la solution, additionnée de noir décolorant, est filtrée. Le filtre est lavé 2 fois avec 100 ml au total de diméthylsulfoxyde puis le filtrat et le lavage sont réunis, additionnés de 400 ml d'eau distillée, conservés pendant 30 minutes à 5°C et centrifugés. La solution surnageante est éliminée puis le solide apparu est séparé par filtration, lavé avec 25 ml d'eau distillée et séché sous pression réduite (1 mm Hg; 0,13 kPa) à 100°C. On obtient ainsi 1,3 g d'acide 10-(4-oxo-5H,10H-imidazo[1,2-a]indéno[1,2-e]pyrazinylidène)aminooxyacétique se décomposant sans fondre au-dessus de 260°C [Spectre de R.M.N.: [300 MHz; (CD₃)₂SO d6; δ en ppm] : mélange d'isomères : 80% d'isomère E, 20% d'isomère Z, 5,05 et 5,08 (2s, 2H en totalité, =N-OCH₂-COO- respectivement de l'isomère E et Z ); de 7,35 à 7,60 (mt, 2,2 H: -H 7 et -H 8 de l'isomère E et Z et 1 des -H de l'imidazole pour l'isomère Z); 7,60 (s, 0,8 H: 1 des -H de l'imidazole pour l'isomère E); 7,64 et 7,85-7,93 et 8,15 (4d, J= 7,5 Hz, respectivement 0,2 H et 0,8 H: -H 6 et -H 9 respectivement de l'isomère Z et E); 8,04 et 8,65 (2s, 1H en totalité : l'autre -H de l'imidazole respectivement de l'isomère E et Z); 12,77 (mf, 1H: -CO-NH-).

### EXEMPLE 37

A une suspension de 0,6 g de chlorhydrate de 10-amino-5H,10H-imidazo[1,2-a]indéno[1,2-e]pyrazine-4-one dans 30 ml de diméthylformamide anhydre maintenue sous atmosphère d'azote à une température voisine de 0°C, on ajoute successivement 0,22 g de chlorure de propionyle et 0,4 g de triéthylamine. Le mélange est agité pendant 3 heures et 30 minutes et on ajoute 0,22 g de chlorure de propionyle. Le mélange est agité pendant 1 heure à la même température et pendant 16 heures à une température voisine de 20°C. L'insoluble apparu est éliminé par filtration et le filtrat est concentré à sec sous pression réduite (10 mm Hg; 1,3 kPa) à 100°C. Le produit obtenu (1,14 g) est dissous à 40°C dans 10 ml d'acide acétique et la solution, additionnée de 50 ml d'eau distillée, est agitée pendant 1 heure à une température voisine de 20°C. L'insoluble apparu est séparé par filtration, lavé 2 fois avec 4 ml au total d'eau distillée et séché sous pression réduite (1 mm Hg; 0,13 kPa) à 100°C. On obtient ainsi 0,26 g de 10-propionamido5H,10H-imidazo[1,2-a]indéno[1 ,2-e]pyrazine-4-one se décomposant sans fondre au-dessus de 260°C [Spectre de R.M.N.: [200 MHz;(CD₃)₂SO d6; δ en ppm] : 1,14 (t, J= 7,5 Hz, 3H : -CH₃ éthyl); 2,28 (q, J= 7,5 Hz, 2H : -COCH₂- éthyl); 6,18(d, J= 8,5 Hz, 1H: -CH- 10); 7,38 et 7,48 (2t larges, J= 8 Hz, 1H chacun : -H 7 et -H 8); 7,52 et 7,88 (2d larges, J= 8 Hz, 1H chacun : -H 6 et -H 9); 7,60 et 7,62 (2s, 1H chacun : -H de l'imidazole); 8,48 (d, J= 8,5 Hz, 1H : -NHCO- en 10); 12,43 (s large, 1H : -CONH- du cycle).

### EXEMPLE 38

A une suspension de 1 g de chlorhydrate de 10-amino-5H,10H-imidazo[1,2-a]indéno[1,2-e]pyrazine-4-one dans 50 ml de diméthylformamide anhydre maintenue sous atmosphère d'azote à une température voisine de -10°C, on ajoute successivement 0,9 g de chlorure d'isobutyryle et une solution de 0,4 g de triéthylamine dans 5 ml de diméthylformamide anhydre. Le mélange est agité pendant 1 heure à la même température et on ajoute 0,4 g de chlorure d'isobutyryle. Le mélange est agité pendant 30 minutes à la même température et pendant 1 heure en laissant remonter progressivement la température à 20°C. L'insoluble apparu est éliminé par filtration et le filtrat est concentré à sec sous pression réduite (15 mm Hg; 2 kPa) à 70°C. Le produit obtenu (1,63 g) est dissous dans 30 ml de méthanol et la solution, additionnée de noir décolorant, est filtrée, diluée avec 40 ml d'eau distillée et conservée pendant 45 minutes à une température voisine de 20°C. L'insoluble apparu est séparé par filtration, lavé avec 5 ml d'eau distillée et avec 5 ml d'acétone puis séché sous pression réduite (1 mm Hg; 0,13 kPa) à 100°C. On obtient ainsi 0,38 g de 10-isobutyramido-5H,10H-imidazo[1,2-a]indéno[1,2-e]pyrazine-4-one se décomposant sans fondre au-dessus de 260°C [Spectre de R.M.N.: [300 MHz; (CD₃)₂SO d6; δ en ppm] : 1,09 et 1,12 [2d, J= 7.5 Hz, 6H : -CH(CH₃)₂]; 2,48 [mt, 1H: -CH(CH₃)₂]; 6,15 (d, J= 8,5 Hz, 1H: -CH- 10); 7,35 et 7,45 (2t larges, J= 8 Hz, 1H chacun : -H 7 et -H 8); 7,47 et 7,85 (2d larges, J= 8 Hz, 1H chacun: -H 6 et -H 9); 7,52 et 7,58 (2s, 1H chacun: -H de l'imidazole); 8,46 (d, J= 8.5 Hz, 1H : -NHCO- en 10); 12,48 (s large, 1H: -CONH- du cycle).

### EXEMPLE 39

A une suspension de 0,6 g de chlorhydrate de 10-amino-5H,10H-imidazo[1,2-a]indéno[1,2-e]pyrazine-4-one dans 30 ml de diméthylformamide anhydre maintenue sous atmosphère d'azote à une température voisine de -10°C, on ajoute successivement 0,29 g de chlorure de benzènesulfonyle et une solution de 0,4 g de triéthylamine dans 2 ml de diméthylformamide anhydre et le mélange est agité pendant 1 heure et 30 minutes à la même température. On ajoute 0,29 g de chlorure de benzènesulfonyle et le mélange est agité pendant 2 heure 30 minutes en laissant remonter progressivement la température à 10°C. L'insoluble apparu est éliminé par filtration et le filtrat est concentré à sec sous pression réduite (10 mm Hg; 1,3 kPa) à 100°C. Le produit obtenu (1,72 g) est dissous dans 6 ml d'acide acétique à 40°C et la solution, additionnée de 40 ml d'eau distillée est agitée pendant 1 heure. L'insoluble apparu est séparé par filtration, lavé 2 fois avec 4 ml au total d'eau distillée et séché sous pression réduite (1 mm Hg; 0,13 kPa) à 100°C. On obtient ainsi 0,19 g de 10-benzènesulfonamido-5H,10H-imidazo[1,2-a]indéno[1,2-e]pyrazine-4-one fondant à 216°C [Spectre de R.M.N.: [300 MHz; (CD₃)₂SO d6; δ en ppm] : 5.71 (d, J= 8,5 Hz, 1H: -CH- 10); 6,34 et 7,77 (2d larges, J= 8 Hz, 1H chacun : -H 6 et -H 9); 7,08 et 7,36 (2t larges, J= 8 Hz, 1H chacun : -H 7 et -H 8); 7,56 et 7,74 (2s, 1H chacun : -H de l'imidazole); 7,68 (t, J= 7,5 Hz, 2H : -H en 3 et -H en 5 du phénylsulfonyl); 7,78 (t, J= 7,5 Hz, 1H : -H en 4 du phénylsulfonyl); 7,95 (d, J= 7,5 Hz, 2H : -H en 2 et -H en 6 du phénylsulfonyl); 8,62, (d, J= 8,5 Hz, 1H: -NHCO- en 10); 12,46 (s large, 1H: -CONH- du cycle).

### EXEMPLE 40

A une suspension de 0,92 g de chlorhydrate de 10-amino-5H,10H-imidazo[1,2-a]indéno[1,2-e]pyrazine-4-one dans 20 ml de diméthylformamide anhydre maintenue sous atmosphère d'azote à une température voisine de 20°C, on ajoute 1,1 g de triéthylamine puis, goutte à goutte en 10 minutes, une solution de 1,25 g d'isocyanate de phényle dans 2 ml de diméthylformamide anhydre et le mélange est agité pendant 2 heures à la même température. L'insoluble apparu est séparé par filtration, lavé 2 fois avec 2 ml au total de diméthylformamide et séché sous pression réduite (5 mm Hg; 0,65 kPa) à 100°C. Le produit obtenu (0,9 g) est dissous dans 15 ml de diméthylsulfoxyde et la solution, additionnée de 15 ml d'eau distillée, est agitée pendant 2 heures à une température voisine de 20°C. L'insoluble apparu est séparé par filtration, lavé 2 fois avec 2 ml au total d'eau distillée et séché sous pression réduite (1 mm Hg; 0,13 kPa) à 100°C. On obtient ainsi 0,53 g de 10-(3-phényluréido)-5H,10H-imidazo[1,2-a]indéno[1,2-e]pyrazine-4-one se décomposant sans fondre au-dessus de 260°C [Spectre de R.M.N.: [200 MHz; (CD₃)₂SO d6; δ en ppm] : 6,10 (d, J= 8,5 Hz, 1H: -CH-10); 6,91 (d, J= 8,5 Hz, 1H: -NHCO- en 10); 7,00 (t, J= 7,5 Hz, 1H: -H en 4 du phénylcarbamoyle); 7,31 (t, J=7,5 Hz, 2H : -H en 3 et -H en 5 du phénylcarbamoyle); 7,38 et 7,48 (2t larges, J= 8 Hz, 1H chacun : -H 7 et -H 8); 7,52 (d, J=7,5 Hz, 2H : -H en 2 et -H en 6 du phénylcarbamoyle); 7,60 et 7,85 (2s, 1H chacun : -H de l'imidazole); 7,62 et 7,88 (2d larges, J= 8 Hz, 1H chacun- : -H 6 et -H 9); 8,68 (s, 1H: Ar-NH-CO); 12,50 (s large, 1H: -CONH- du cycle).

### EXEMPLE 41

A une suspension de 0,92 g de chlorhydrate de 10-amino-5H,10H-imidazo[1,2-a]indéno[1,2-e]pyrazine-4-one dans 20 ml de diméthylformamide anhydre maintenue sous atmosphère d'azote à une température voisine de 20°C, on ajoute 1,1 g de triéthylamine puis, goutte à goutte en 5 minutes, une solution de 0,6 g d'isocyanate de méthyle dans 2 ml de diméthylformamide anhydre et le mélange est agité pendant 2 heures à la même température. L'insoluble apparu est séparé par filtration, lavé 2 fois avec 2 ml au total de diméthylformamide, 2 fois avec 2 ml au total d'acétone et séché sous pression réduite (5 mm Hg; 0,65 kPa) à 100°C. Le produit obtenu (0,35 g) est agité en suspension pendant 5 minutes dans 3 ml d'acétone bouillant et, après refroidissement et conservation pendant 45 minutes à 5°C, l'insoluble est séparé par filtration, lavé 2 fois avec 2 ml au total de méthanol et séché sous pression réduite (1 mm Hg; 0,13 kPa) à 100°C. On obtient ainsi 0,29 g de 10-(3-méthyluréido)-5H, 10H-imidazo[1,2-a]indéno[1,2-e]pyrazine-4-one se décomposant sans fondre au-dessus de 260°C [Spectre de R.M.N.: [200 MHz; (CD₃)₂SO d6; δ en ppm] : 2,70 (d, J= 4,5 Hz, 3H : -CH₃); 6,02 (d, J= 8,5 Hz, 1H: -CH-10); 6,04 (d, J= 4,5 Hz, 2H : -CONH-); 6,70 (d, J= 8,5 Hz, 1H : -NHCO- en 10); 7,37 et 7,55 (2t larges, J= 8 Hz, 1H chacun : -H 7 et -H 8); 7,53 et 7,85 (2d larges, J= 8 Hz, 1H chacun : -H 6 et -H 9); 7,60 et 7,78 (2s, 1H chacun : -H de l'imidazole); 12,46 (s large, 1H: -CONH-du cycle).

### EXEMPLE 42

A une solution de 0,48 g d'acétate d'ammonium dans 120 ml d'une solution aqueuse d'ammoniaque à 28%, on ajoute successivement 3,25 g de 8-fluoro-10-hydroxyimino-5H,10H-imidazo[1,2-a]indéno[1,2-e]pyrazine-4-one, 120 ml d'éthanol et 3,55 g de zinc en poudre. Le mélange est agité pendant 2 heures à l'ébullition et pendant 16 heures à une température voisine de 20°C puis on ajoute goutte à goutte 60 ml d'une solution aqueuse d'acide chlorhydrique 5N et le mélange est agité pendant 16 heures à la même température. L'insoluble apparu est éliminé par filtration et le filtrat est concentré à sec sous pression réduite (10 mm Hg; 1,3 kPa) à 50°C. Le produit obtenu (13,4 g) est agité en suspension dans 30 ml d'eau distillée et l'insoluble est séparé par filtration, lavé 2 fois avec 4 ml au total d'eau distillée et séché sous pression réduite (10 mm Hg; 1,3 kPa) à 50°C. Le produit obtenu (1,35 g) est mis en suspension pendant 10 minutes dans 10 ml de méthanol bouillant. Après refroidissement et conservation pendant 16 heures à 5°C, l'insoluble est séparé par filtration, lavé avec 1 ml de méthanol et remis en suspension pendant 10 minutes dans 5 ml de méthanol bouillant. Après refroidissement et conservation pendant 6 heures à 5°C, l'insoluble est séparé par filtration, lavé avec 1 ml de méthanol et séché sous pression réduite (1 mm Hg; 0,13 kPa) à 100°C. On obtient ainsi 0,48 g de dichlorhydrate de 10-amino-8-fluoro-5H,10H-imidazo[1,2-a]indéno [1,2-e]pyrazine-4-one se décomposant sans fondre au-dessus de 260°C [Spectre de R.M.N.: [200 MHz; (CD₃)₂SO d6; δ en ppm] : 5,69 (s large, 1H: -CH- 10); 7,44 (dt, J= 8,5 et 2 Hz, 1H : -H7); 7,68 et 8,54 (2s, 1H chacun : -H de l'imidazole); 7,93 (dd, J=8,5 et 2 Hz, 1H : -H 9); 7,94 (dd, J= 8,5 et 6 Hz, 1H : -H 6); 9,30 (mf, 3H : -NH₃⁺Cl⁻); 12,72 (mf étalé, 1H : -CONH-).

La 8-fluoro-10-hydroxyimino-5H,10H-imidazo[1,2-a]indéno[1,2-e]pyrazine-4-one peut être préparée de la façon suivante : 1,8 g d'hydrure de sodium à 80% est ajouté en 15 minutes à une suspension maintenue à 20°C sous atmosphère d'azote de 4,72 g de chlorhydrate de 8-fluoro-5H,10H-imidazo[1,2-a]indéno[1,2-e]pyrazine-4-one dans 70 ml de diméthylsulfoxyde anhydre. Après 25 minutes d'agitation, une solution de 2,3 g de nitrite d'isoamyle dans 7 ml de diméthylsulfoxyde anhydre est ajoutée goutte à goutte en 20 minutes puis le mélange est agité pendant 1 heure à la même température. 35 ml d'eau distillée sont ajoutés lentement et le mélange est ensuite versé sur un mélange de 100 g de glace et de 340 ml d'eau distillée, acidifié avec 4,1 ml d'acide acétique puis centrifugé. Après élimination de la solution surnageante, le solide est mis en suspension dans 25 ml d'acétone, filtré, lavé 2 fois avec 20 ml au total d'acétone et séché sous pression réduite (5 mm Hg; 0,65 kPa) à 100°C. On obtient ainsi 4,45 g de 8-fluoro-10-hydroxyimino-5H,10H-imidazo[1,2-a]indéno[1,2-e]pyrazine-4-one, mélange des formes E et Z.

La 8-fluoro-5H,10H-imidazo[1,2-a]indéno[1,2-e]pyrazine-4-one peut être obtenue de la manière suivante:1,8 g de 1-[2-(5-fluoro-1-oxo-indanyl)]imidazole-2-carboxamide est dissous dans 90 ml de méthanol bouillant et la solution additionnée de 0,1 g de noir décolorant est filtrée. Le filtre est lavé avec 20 ml de méthanol bouillant puis le filtrat et le lavage sont réunis, additionnés de 27 ml de solution aqueuse d'acide chlorhydrique 12N et conservés pendant 3 heures à 5°C. Les cristaux sont séparés par filtration, lavés 2 fois par 20 ml au total de méthanol glacé et séchés sous pression réduite (1 mm Hg; 0,13 kPa) à 60°C. On obtient ainsi 1 g de chlorhydrate de 8-fluoro-5H,10H-imidazo[1,2-a]indéno[1,2-e]pyrazine-4-one se décomposant sans fondre au-dessus de 300°C [Spectre de R.M.N. : (200 MHz; DMSO d6; δ en ppm) : 4,11 (s, 2H : -CH2- en 10); 7,32 (ddd, J=9,5 - 8,5 et 2 Hz, 1H : -H7); 7,57 (dd, J=9,5 et 2 Hz, 1H : -H9); 7,97 (dd, J=8,5 et 5 Hz, 1H: -H6); 7,99 et 8,28 (2d, J=1,5 Hz, 1H chacun : -H de l'imidazole); 13,13 (mf, 1H: -CONH-)].

Le 1-[2-(5-fluoro-1-oxo-indanyl)]imidazole-2-carboxamide peut être préparé de la façon suivante : 2,2 g de 1-[2-(5-fiuoro-1-oxo-indanyl)]imidazole-2-carboxylate d'éthyle sont dissous dans 80 ml d'une solution 2,5 N de méthanol ammoniacal et la solution est conservée pendant 20 heures à une température voisine de 20°C puis concentrée à sec sous pression réduite (15 mm Hg; 2 kPa) à 35°C. Le produit obtenu est mis en suspension dans 50 ml d'oxyde d'isopropyle, filtré, lavé 2 fois par 20 ml au total d'oxyde d'isopropyle puis séché sous pression réduite (15 mm Hg; 2 kPa) à une température voisine de 20°C. On obtient ainsi 1,85 g de 1-[2-(5-fluoro-1-oxo-indanyl)]imidazole-2-carboxamide sous forme d'un solide fondant à 221°C.

Le 1-[2-(5-fluoro-1-oxo-indanyl)]imidazole-2-carboxylate d'éthyle peut être préparé de la façon suivante : une solution de 13,3 g d'imidazole-2-carboxylate d'éthyle dans 145 ml de diméthylformamide anhydre est additionnée goutte à goutte en 20 minutes à une température comprise entre 20°C et 25°C à une suspension de 3,4 g d'hydrure de sodium à 80% dans 45 ml de diméthylformamide anhydre maintenue sous atmosphère d'azote. Après 15 minutes d'agitation, une solution de 26 g de 2-bromo-5 fluoro-1-indanone dans 190 ml de diméthylformamide anhydre est ajoutée goutte à goutte en 10 minutes à la même température. Le mélange est agité pendant 1 heure 30 minutes puis, après addition lente de 100 ml d'eau, versé sur 3800 ml d'eau distillée et extrait 4 fois par 3800 ml au total de chloroforme. Les extraits organiques sont réunis, lavés avec 950 ml d'eau distillée, séchés sur du sulfate de sodium anhydre et concentrés à sec sous pression réduite (15 mm Hg; 2 kPa) à 40°C. Le produit obtenu (27 g) est chromatographié sur 1490 g de gel de silice neutre (0,020-0,045 mm) contenus dans une colonne de 9,6 cm de diamètre en éluant sous pression avec un mélange dichlorométhane-acétate d'éthyle (70-30 en volumes) et en recueillant des fractions de 80 ml. Les fractions 13 à 70 sont réunies et concentrées à sec sous pression réduite (15 mm Hg; 2 kPa) à 40°C. On obtient ainsi 13,4 g de 1-[2-(5-fluoro-1-oxo-indanyl)]imidazole-2-carboxylate d'éthyle fondant à 127°C.

La 2-bromo-5-fluoro-1-indanone peut être préparée de la façon suivante : une solution de 20,6 g de brome dans 90 ml d'acide acétique est ajoutée goutte à goutte en 1 heure à 20°C à une solution de 20 g de 5-fluoro-1-indanone et de 0,1 ml d'une solution aqueuse d'acide bromhydrique à 47% dans 260 ml d'acide acétique. Après 2 heures d'agitation, le mélange est versé sur 850 ml d'eau distillée et extrait 3 fois par 850 ml au total de chlorure de méthylène. Les extraits organiques sont réunis, lavés avec 200 ml d'eau distillée, séchés sur du sulfate de sodium anhydre et concentrés à sec sous pression réduite (15 mm Hg; 2 kPa) à 30°C. Le produit obtenu (31 g) est chromatographié sur 1860 g de gel de silice neutre (0,020-0.045 mm) contenus dans une colonne de 9,8 cm de diamètre en éluant avec un mélange cyclohexane-acétate d'éthyle (70-30 en volumes) et en recueillant une fraction de 10,5 litres qui est éliminée puis une fraction de 25 litres qui est concentrée sous pression réduite (15 mm Hg; 2 kPa) à 40°C. On obtient ainsi 28,4 g de 2-bromo-5 fluoro-1-indanone sous forme d'une huile jaune [Rf=0,7; chromatographie sur couche mince de gel de silice; solvant : cyclohexane-acétate d'éthyle (70-30 en volumes)].

### EXEMPLE 43

A une suspension de 7 g de 10-formamido-5H,10H-imidazo[1,2-a]indéno [1,2-e]pyrazine-4-one dans 225 ml de tétrahydrofuranne anhydre maintenue à 0°C, on ajoute goutte à goutte en 10 minutes 62,5 ml d'une solution 2M dans le tétrahydrofuranne du complexe borane-sulfure de méthyle. Le mélange est agité pendant 10 minutes à la même température puis pendant 18 heures à une température voisine de 20°C. Après addition de 100 ml de méthanol et agitation pendant 1 heure, l'insoluble est séparé par filtration et lavé avec 25 ml de méthanol. Le filtrat et le lavage sont réunis et concentrés à sec sous pression réduite (15 mm Hg; 2 kPa) à 35°C. Le produit obtenu (3,9 g) est chromatographié sur 390 g de gel de silice neutre (0,020-0,045 mm) contenus dans une colonne de 5,3 cm de diamètre en éluant avec un mélange chloroforme- méthanol- ammoniaque à 28% (82- 15-3 en volumes) et en recueillant des fractions de 35 ml. Les fractions 25 à 31 sont réunies et concentrées à sec sous pression réduite (1 mm Hg; 0,13 kPa) à 90°C. On obtient ainsi 0,59 g de 10-méthylamino-5H,10H-imidazo[1,2-a]indé no[1,2-e] pyrazine-4-one fondant à 184°C [Spectre de R.M.N.: [200 MHz; (CD₃)₂SO d6; δ en ppm] :1,85 (s, 3H : -CH₃); 5,13 (s, 1H : -CH- 10); 7,36 et 7,43 (2t larges, J= 8 Hz, 1H chacun : -H 7 et -H 8); 7,61 et 8,17 (2s larges, 1H chacun : -H de l'imidazole); 7,62 et 7,85 (2d larges, J= 8 Hz, 1H chacun : -H 6 et -H 9); 12,35 (s large, 1H: -CONH- du cycle).

La 10-formamido-5H,10H-imidazo[1,2-a]indéno[1,2-e]pyrazine-4-one peut être préparée de la façon suivante : un mélange de 21,4 g d'anhydride acétique et de 11,5 g d'acide formique est chauffé pendant 2 heures à une température comprise entre 50°C et 60°C et refroidi à 20°C. On ajoute ensuite 0,9 g d'acétate de sodium anhydre puis, après dissolution, 2,75 g de chlorhydrate de 10-amino-5H, 10H-imidazo[1,2-a]indéno[1,2-e]pyrazine-4-one. Le mélange est agité pendant 1 heure à 20°C et concentré à sec sous pression réduite (15 mm Hg; 2 kPa) à 50°C. Le produit obtenu (4,3 g) est dissous dans 50 ml de diméthylformamide et la solution, additionnée de noir décolorant, est filtrée. On ajoute ensuite au filtrat 200 ml d'eau distillée et le mélange est conservé pendant 1 heure à une température voisine de 5°C. L'insoluble apparu est séparé par filtration, lavé avec 10 ml d'eau distillée et séché sous pression réduite (1 mm Hg; 0,13 kPa) à 100°C. On obtient ainsi 1,2g de 10-formamido-5H,10H-imidazo[1,2-a]indéno[1,2-e]pyrazine-4-one fondant à 280°C (décomposition) [Spectre de R.M.N.: [200 MHz; (CD₃)₂SO d6; δ en ppm] : 6,22 (d, J= 8,5 Hz, 1H : -CH- 10); 7,36 et 7,45 (2t larges, J= 8 Hz, 2H : -H 7 et -H 8); 7,51 et 7,87 (2d larges, J= 8 Hz, 2H : -H 6 et -H 9); 7,58 et 7,64 (2s, 1H chacun : -H de l'imidazole); 8,44 (s, 1H: -CH=O); 8,78 (d, J= 8,5 Hz, 1H: -CONH en 10); 12,50 (s large, 1H,: -CONH- du cycle).

### EXEMPLE 44

A une suspension de 1,05 g de 7-chloro-10-hydroxyimino-5H,10H-imidazo[1,2-a]indéno[1,2-e]pyrazine-4-one dans 35 ml d'éthanol en présence de 0,12 g d'acétate d'ammonium et de 35 ml d'ammoniaque à 28%, on ajoute progressivement 1,07 g de zinc en poudre. Le mélange réactionnel est porté à reflux pendant 5 heures. Après refroidissement à une température voisine de 20°C, 45 ml d'acide chlorhydrique 6N sont ajoutés goutte à goutte au milieu réactionnel et l'agitation poursuivie 24 heures à la même température. Le précipité formé est filtré, lavé à l'eau et séché sous pression réduite (1 mm Hg; 0,13 kPa) à 50°C. On obtient ainsi 0,35 g de dichlorhydrate et trihydrate de 10-amino-7-chloro-5H,10H-imidazo[1,2-a]indéno[1,2-e] pyrazine-4-one sous forme de poudre beige se décomposant à 250°C [Spectre de R.M.N.: (250 MHz; DMSO d6; δ en ppm) : 5,70 (s large, 1H : CH en 10); 7,55 (dd, J= 8 et 2 Hz, 1H: H-8); 7,67 et 8,57 (2d, J= 1 Hz, 1H chacun : H de l'imidazole); 8,01 (d, J= 2 Hz, 1H: H-6); 8,01 (d, J= 8 Hz, 1H : H-9); 9,30 (mf, 1H: NH2), 12,70 (mf, 1H: CONH)].

La 7-chloro-10-hydroxyimino-5H,10H-im idazo[1,2-a]indéno[1,2-e]pyrazine-4-one peut être obtenue selon le protocole suivant : 0,97 g d'hydrure de sodium à 60% sont ajoutés à une suspension de 2,5 g de 7-chloro-5H,10H-imidazo[1,2-a]indéno[1,2-e]pyrazine-4-one dans 30 ml de diméthylsulfoxyde anhydre. Après 30 minutes d'agitation à une température voisine de 20°C, une solution de 1,3 ml de nitrite d'isoamyle dans 10 ml de diméthylsulfoxyde est ajoutée goutte à goutte puis le mélange agité pendant 3 heures à la même température. 20 ml d'eau distillée sont ajoutés lentement et le mélange est ensuite versé sur de l'eau glacée, acidifié avec de l'acide acétique puis centrifugé. Après élimination de la solution surnageante, le solide est repris dans 20 ml d'acétone et l'insoluble filtré, lavé avec du méthanol et séché. Le produit obtenu (1,3 g) est mis en suspension dans 30 ml de diméthylsulfoxyde et l'insoluble filtré, lavé à l'eau et séché. Le filtrat est traité par 100 ml d'eau et le précipité formé filtré, lavé à l'eau et séché. Les deux insolubles ainsi isolés sont réunis, lavés à l'eau et séchés sous pression réduite (1 mm Hg; 0,13 kPa) à 50°C. On obtient 1,05 g de produit attendu sous forme de solide vert dont le point de fusion est supérieur à 260°C.

La 7-chloro-5H,10H-imidazo[1,2-a]indéno[1,2-e]pyrazine-4-one peut être obtenue de la manière suivante : 1 g de 1-[2-(6-chloro-1-oxo-indanyl)]imidazole-2-carboxamide est dissous dans 50 ml de méthanol bouillant et la solution additionnée de 0,1 g de noir décolorant est filtrée. Le filtre est lavé avec 25 ml de méthanol bouillant puis le filtrat et le lavage sont réunis, additionnés de 15 ml de solution aqueuse d'acide chlorhydrique 12N et conservés pendant 3 heures à 5°C. Les cristaux sont séparés par filtration, lavés 2 fois par 20 ml au total de méthanol glacé et séchés sous pression réduite (1 mm Hg; 0,13 kPa) à 60°C. On obtient ainsi 0,4 g de chlorhydrate de 7-chloro-5H,10H-imidazo[1,2-a]indéno[1,2-e]pyrazine-4-one se décomposant sans fondre au-dessus de 300°C [Spectre de R.M.N.: (300 MHz; DMSO d6; δ en ppm) : 4,12 (s, 2H : -CH2- en 10); 7,44 (dd, J=8 et 1 Hz 1H: -H8); 7,66 (d, J=8 Hz, 1H : -H9); 7,95 et 8,25 (2s larges, 1H chacun : -H de l'imidazole); 8,05 (d, J=1 Hz, 1H : -H6); 12,97 (mf, 1H : -CONH-)].

Le 1-[2-(6-chloro-1-oxo-indanyl)]imidazole-2-carboxamide peut être préparé de la façon suivante : 1,2 g de 1-[2-(6-chloro-1-oxo-indanyl)]imidazole-2-carboxylate d'éthyle sont dissous dans 45 ml d'une solution 2,5 N de méthanol ammoniacal et la solution est conservée pendant 20 heures à une température voisine de 20°C puis concentrée à sec sous pression réduite (15 mm Hg; 2 kPa) à 35°C. Le produit obtenu est mis en suspension dans 50 ml d'oxyde d'isopropyle, filtré, lavé 2 fois par 20 ml au total d'oxyde d'isopropyle puis séché sous pression réduite (15 mm Hg; 2 kPa) à une température voisine de 20°C. On obtient ainsi 1 g de 1-[2-(6-chloro-1-oxo-indanyl)]imidazole-2-carboxamide sous forme d'un solide fondant à 190°C.

Le 1-[2-(6-chloro-1-oxo-indanyl)]imidazole-2-carboxylate d'éthyle peut être préparé de la façon suivante : une solution de 2,5 g d'imidazole-2-carboxylate d'éthyle dans 30 ml de diméthylformamide anhydre est additionnée goutte à goutte en 20 minutes à une température comprise entre 20°C et 25°C à une suspension de 0,7 g d'hydrure de sodium à 80% dans 30 ml de diméthylformamide anhydre maintenue sous atmosphère d'azote. Après 15 minutes d'agitation, une solution de 5,4 g de 2-bromo-6-chloro-1-indanone dans 10 ml de diméthylformamide anhydre est ajoutée goutte à goutte en 10 minutes à la même température. Le mélange est agité pendant 1 heure 30 minutes puis, après addition lente de 100 ml d'eau, versé sur 3800 ml d'eau distillée et extrait 4 fois par 3800 ml au total de chloroforme. Les extraits organiques sont réunis, lavés avec 950 ml d'eau distillée, séchés sur du sulfate de sodium anhydre et concentrés à sec sous pression réduite (15 mm Hg; 2 kPa) à 40°C. Après chromatographie sur gel de silice avec un mélange dichlorométhane-acétate d'éthyle (70-30 en volumes), on obtient 1 g de 1-[2-(6-chloro-1-oxo-indanyl)]imidazole-2-carboxylate d'éthyle fondant à 180°C.

La 2-bromo-6-chloro-1-indanone peut être préparée comme décrit dans le brevet allemand 2 640 358.

### EXEMPLE 45

A 0,5 g de dichlorhydrate de 10-amino-7-chloro-5H,10H-imidazo[1,2-a]indéno[1,2-e]pyrazine-4-one en suspension dans 8 ml de diméthylsulfoxyde, on ajoute goutte à goutte 0,45 ml de triéthylamine, puis une solution de 0,9 ml de phénylisocyanate dans 2 ml de diméthylformamide. Le milieu réactionnel est agité 2 heures à une température voisine de 20°C et le précipité formé est filtré, lavé à l'eau et séché sous pression réduite (1 mm Hg; 0.13 kPa) à 50°C. On obtient ainsi 0,15 g de 7-chloro-10-(3-phényluréido)-5H,10H-imidazo[1,2-a]indéno[1,2-e]pyrazine-4-one sous forme de solide blanc dont le point de fusion est supérieur à 260°C (Analyse C20H14ClN5O2; 0,6 DMF; 0,8 H20 % calculé C: 61,31; H : 3,60; Cl: 9,05; N : 17,87; % trouvé C : 61,1; H : 3,5; Cl: 9,4; N : 17,7).

### EXEMPLE 46

A 0,8 g de 7-chloro-10-formamido-5H,10H-imidazo[1,2-a]indéno [1,2-e]pyrazine-4-one en suspension dans 25 ml de dioxanne anhydre à 10°C, on ajoute 6,7 ml d'une solution 2M de borane diméthylsulfure dans le tétrahydrofuranne en environ 1 heure. Le mélange réactionnel est agité 15 heures à une température voisine de 20°C, puis traité par 20 ml de méthanol et laissé 1 heure supplémentaire à la même température. Après concentration à sec sous pression réduite (15 mm Hg; 2 kPa), le résidu obtenu est purifié par flash-chromatographie sur colonne de silice en utilisant un mélange de dichlorométhane et de méthanol (95/5 en volumes) comme éluant. Le produit obtenu (0,43 g) en solution dans un mélange d'éthanol et de méthanol (1/1 en volumes) est acidifié à l'aide d'éther chlorhydrique pour conduire à 0,24 g de dichlorhydrate de 7-chloro-10-méthylamino-5H,10H-imidazo[1,2-a]indéno[1,2-e]pyrazine-4-one sous forme de poudre blanc verdâtre dont le point de fusion est supérieur à 260°C (Analyse C20H14CIN502; 0,24 EtOH; 0,15 H20 % calculé C : 44,53; H : 4,00; Cl 28,16; N : 14,84; % trouvé C : 44,5; H : 3,9; Cl : 28,1; N : 14,7).

La 7-chloro-10-formamido-5H,10H-imidazo[1,2-a]indéno[1,2-e]pyrazine-4-one peut être obtenue de la façon suivante : 4 ml d'acide formique sont ajoutés lentement à 8 ml d'anhydride acétique et le mélange chauffé à 50°C pendant 2 heures. Après refroidissement à une température voisine de 20°C, 0,4 g d'acétate de sodium sont ajoutés au mélange puis 1,25 g de chlorhydrate de 10-amino-7-chloro-5H,10H-imidazo[1,2-a]indéno[1,2-e] pyrazine-4-one par petites portions. La suspension est agitée 1 heure à la même température. Le précipité est filtré, lavé à l'eau et séché sous pression réduite (1 mm Hg; 0.13 kPa) à 50°C pour conduire à 0,88 g de produit attendu sous forme de solide bleuté dont le point de fusion est supérieur à 260°C.

### EXEMPLE 47

2,1 g de 10-benzylidéne-7-chloro-5H,10H-imidazo[1,2-a]indéno[1,2-e] pyrazine-4-one en solution dans 20 ml d'un mélange de méthanol et de diméthylformamide (1/1 en volumes) sont hydrogénés à une température voisine de 20°C sous une pression de 9,6 bar d'hydrogène pendant 24 heures en présence de charbon palladié à 10%. On filtre alors le catalyseur sous atmosphère inerte et on évapore les solvants. L'huile brune obtenue est reprise dans 25 ml de méthanol et un précipité rose formé après addition de 50 ml d'eau distillée. Le solide ainsi obtenu (1,3 g) est recristallisé dans 150 ml d'un mélange de méthanol et d'eau (1/2 en volumes). On obtient 0,6 g de 10-benzyl-7-chloro-5H, 10H-imidazo[1,2-a]indéno[1,2-e]pyrazine-4-one sous forme de solide beige dont le point de fusion est supérieur à 260°C (Analyse C20H14CIN30; 0,41 H20 % calculé C : 69,07; H : 4,06; Cl : 10,19; N : 12,08; % trouvé C : 69,0; H : 3,9; Cl : 10,5; N : 12,4).

La 10-benzylidéne-7-chloro-5H,10H-imidazo[1,2-a]indéno[1,2-e]pyrazine-4-one peut être préparée de la manière suivante: à un mélange de 2 g de 7-chloro-5H,10H-imidazo[1,2-a]indéno[1,2-e]pyrazine-4-one et de 1,2 ml de benzaldéhyde dans 60 ml de diméthylsulfoxyde, on ajoute progressivement 0,78 g d'hydrure de sodium à 60%. La réaction est poursuivie 5 heures à une température voisine de 20°C. Après refroidissement à 10°C, 5 ml d'acide acétique sont ajoutés goutte à goutte, puis 200 ml d'eau distillée. Le précipité formé est filtré, lavé à l'eau et séché pour conduire à 2,4 g de produit attendu sous forme de solide brun dont le point de fusion est supérieur à 260°C.

### EXEMPLE 48

A un mélange agité à une température voisine de 20°C de 0,48 g de 10-méthyl-5H,10H-imidazo[1,2-a]indéno[1,2-e]pyrazine-4-one et de 25 ml de diméthylformamide on ajoute, sous couverture d'argon, 0,21 g d'hydrure de sodium à 80% et on maintient l'agitation pendant 15 minutes. On ajoute ensuite 0,12 g d'hydrure de sodium à 80% et après 15 minutes d'agitation 0,28 ml de triméthylchlorosilane. L'agitation est maintenue pendant 15 minutes puis on ajoute 0,24 ml de 4-vinylpyridine et on poursuit l'agitation pendant 90 minutes. Le mélange réactionnel est traité avec 20 g de glace et 0,7 ml d'acide acétique, filtré et le filtrat est concentré à l'évaporateur rotatif.

Le résidu d'évaporation est trituré avec 40 ml de propanol-2, filtré et le filtrat est concentré à nouveau pour donner une huile rouge. Cette huile est acidifiée avec 5 ml d'acide chlorhydrique 1N, lavée avec du dichlorométhane (2x25 ml) et alcalinisée avec une solution d'hydrogénocarbonate de sodium. Sur cette phase alcaline on effectue deux extractions au dichlorométhane (2x25 ml) et on concentre l'extrait organique à l'évaporateur rotatif. Le résidu d'évaporation est purifié par chromatographie sur colonne de silice (60 g) en éluant avec un mélange de dichlorométhane-méthanol (95-5 en volumes) pour donner une laque incolore. Cette laque est triturée avec 5 ml d'acétate d'éthyle, puis 2x10 ml d'éther isopropylique et 25 ml d'éther de pétrole (40-65°C) et fournit, après séchage à 60°C sous vide (1 mmHg; 0,13 kPa), 0,21 g de 10-méthyl-10-[2-(pyridine-4-yl)éthyl]-5H,10H-imidazo[1,2-a]indéno [1,2-e]pyrazine-4-one sous forme de solide blanc cassé fondant aux environs de 200°C (Analyse % calculé C: 73,67, H: 5,30, N: 16,36, O: 4,67 % trouvé C: 73,8, H: 5,7, N: 16,3).

Les médicaments selon l'invention sont constitués par un composé de formule (I) ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention des conditions qui requièrent l'administration d'un antagoniste du récepteur AMPA ou d'un antagoniste du récepteur NMDA. Ces composés sont notamment utiles pour traiter ou prévenir toutes les ischémies et en particulier l'ischémie cérébrale, les effets dus à une anoxie, l'évolution de maladies neurodégénératives, de la chorée d'HUNTINGTON, de la maladie d'ALZHEIMER, de la sclérose latérale amyotrophique, de l'atrophie olivo-pontocérébelleuse et de la maladie de PARKINSON, vis-à-vis des manifestations épileptogènes et/ou convulsives, pour le traitement des traumatismes cérébraux et spinaux, des traumatismes liés à le dégénérescence de l'oreille interne ou de la rétine, de l'anxiété, de la dépression, de la schizophrénie, du syndrome de TOURETTE, de l'encéphalopathie hépatique, en tant qu'analgésiques, antiinflammatoires, antianorexiques, antimigraineux, antiémétiques et pour traiter les empoisonnements par des neurotoxines ou d'autres substances agonistes du récepteur NMDA, ainsi que les troubles neurologiques associés aux maladies virales telles que le sida, la rage, la rougeole et le tétanos. Ces composés sont aussi utiles pour la prévention des symptomes d'abstinence aux drogues et à l'alcool et de l'inhibition de l'accoutumance et de la dépendance aux opiacés ainsi que pour le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, l'hydroxybutirique-aminoacidurie, l'encéphalopathie de LEAD et la déficience en sulfite oxydase.

Les doses dépendent de l'effet recherché, de la durée du traitement et de la voie d'administration utilisée; elles sont généralement comprises entre 10 mg et 100 mg par jour par voie orale pour un adulte avec des doses unitaires allant de 5 mg à 50 mg de substance active.

D'une façon générale, le médecin déterminera la posologie appropriée en fonction de l'age, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau.q.s.p. 4 ml

## Revendications

1. Composés de formule : 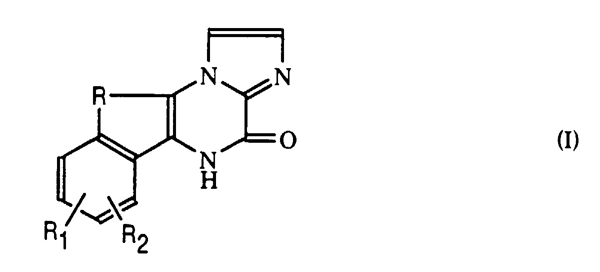 dans laquelle
soit R représente un radical C=R₃, C(R₄)R₅ ou CH-R₆,
- R₁ et R₂, identiques ou différents, représentent des atomes d'hydrogène ou d'halogène ou des radicaux alkyle, alcoxy, amino, acylamino, -NH-CO-NH-Ar, -N=CH-N(alk)alk', nitro, cyano, phényle, imidazolyle ou SO₃H,
- R₃ représente un atome d'oxygène ou un radical NOH, NO-alk-COOX ou CH-R₇,
- R₄ représente un radical alkyle, -alk-Het ou -alk-Ar,
- R₅ représente un radical alkyle (1-11C en chaîne droite ou ramifiée), -alk-Het ou -alk-Ar,
ou bien R₄ et R₅ forment ensemble avec l'atome de carbone auquel ils sont rattachés un radical cycloalkyle,
- R₆ représente un radical hydroxy, alkyle (1-11C en chaîne droite ou ramifiée), -NR₈R₉, -alk-OH, -alk-NR₈R₉, -alk-Ar ou -alk-Het,
- R₇ représente un radical hydroxy, alkyle, phényle, -alk-Ar, -alk-Het, -NR₁₀R₁₁ ou un hétérocycle mono ou polycyclique saturé ou insaturé contenant 4 à 9 atomes de carbone et un ou plusieurs hétéroatomes (O, S, N),
- R₈ et R₉, identiques ou différents, représentent chacun un radical alkyle, ou bien R₈ représente un atome d'hydrogène et R₉ représente un atome d'hydrogène ou un radical alkyle, -COR₁₂, -CSR₃₀ ou -SO₂R₁₃,
- R₁₀ et R₁₁, identiques ou différents, représentent chacun un radical alkyle ou cycloalkyle,
- R₁₂ représente un radical alkyle, cycloalkyle, phényle, -COO-alk, -CH₂-COOX, -CH₂-NH₂, -NH-alk, -NH₂, -NH-Ar ou -NH-Het,
- R₁₃ représente un radical alkyle ou phényle,
- R₃₀ représente un radical -NH-alk, -NH-Ar, -NH₂ ou -NH-Het,
- alk représente un radical alkyle ou alkylène,
- alk' représente un radical alkyle,
- X représente un atome d'hydrogène ou un radical alkyle,
- Ar représente un radical phényle et
- Het représente un hétérocycle mono ou polycyclique saturé ou insaturé contenant 4 à 9 atomes de carbone et un ou plusieurs hétéroatomes (O, S, N);
soit R représente un radical CH-R₆ et R₆ représente un radical 2-imidazolylméthyle et R₁ et R₂ représentent chacun un atome d'hydrogène,
étant entendu que les radicaux et portions alkyle et alkylène contiennent 1 à 4 atomes de carbone en chaîne droite ou ramifiée, les portions acyle contiennent 2 à 4 atomes de carbone, les radicaux cycloalkyle contiennent 3 à 6 atomes de carbone, ainsi que les isomères des composés pour lesquels R₃ représente un radical NOH, NO-alk-COOX ou CH-R₇, les racémiques et les énantiomères des composés de formule (I) pour lesquels R représente un radical C(R₄)R₅ dans lequel R₄ est différent de R₅ ou CH-R₆, et leurs sels

2. Composés de formule (I) selon la revendication 1 pour lesquels soit R représente un radical C=R₃, C(R₄)R₅ ou CH-R₆, R₁ et R₂, représentent des atomes d'hydrogène ou d'halogène, R₃ représente un atome d'oxygène ou un radical NO-alk-COOX ou CH-R₇, R₄ représente un radical alkyle ou -alk- Ar, R₅ représente un radical alkyle (1-11C en chaîne droite ou ramifiée), -alk-Het ou -alk-Ar ou bien R₄ et R₅ forment ensemble avec l'atome de carbone auquel ils sont rattachés un radical cycloalkyle, R₆ représente un radical hydroxy, alkyle (1-11C en chaîne droite ou ramifiée), -NR₈R₉, -alk-NR₈R_{9,} -alk-OH, -alk-Ar ou -alk-Het, R₇ représente un radical hydroxy, -NR₁₀R₁₁ ou un hétérocycle mono ou polycyclique saturé ou insaturé contenant 4 à 9 atomes de carbone et un ou plusieurs hétéroatomes (O, S, N), R₈ représente un atome d'hydrogène et R₉ représente un atome d'hydrogène ou un radical alkyle, -COR₁₂ ou -SO₂R₁₃, R₁₀ et R₁₁, identiques ou différents, représentent chacun un radical alkyle, R₁₂ représente un radical alkyle, -NH-Ar, -NH-alk ou phényle, R₁₃ représente un radical alkyle ou phényle, alk représente un radical alkyle ou alkylène, X représente un radical alkyle et Het représente un hétérocycle mono ou polycyclique saturé ou insaturé contenant 4 à 9 atomes de carbone et un ou plusieurs hétéroatomes (O, S, N) ou bien R représente un radical CH-R₆ et R₆ représente un radical 2-imidazolylméthyle et R₁, R₂ sont des atomes d'hydrogène ainsi que les isomères des composés pour lesquels R₃ représente un radical NO-alk-COOX ou CH-R₇, les racémiques et les énantiomères des composés de formule (I) pour lesquels R représente un radical C(R₄)R₅ dans lequel R₄ est différent de R₅ ou CH-R₆, et leurs sels.

3. Composés de formule (I) selon la revendication 1 choisis parmi les composés suivants :
- 10-hydroxy-5H,10H-imidazo[1 ,2-a]indéno[1 ,2-e]pyrazine-4-one,
- 10-acétamido-5H,10H-imidazo[1,2-a]indéno[1,2-e]pyrazine-4-one,
- 10-amino-5H,10H-imidazo[1,2-a]indéno[1,2-e]pyrazine-4-one,
- 10-(E-diméthylaminométhylène)-5H,10H-imidazo[1,2-a]indéno[1,2-e]pyrazine-4-one,
- 10-hydroxyméthylène-5H,10H-imidazo[1,2-a]indéno[1,2-e]pyrazine-4-one,
- 10,10-diméthyl-5H,10H-imidazo[1,2-a]indéno[1,2-e]pyrazine-4-one,
- spiro[5H,10H-imidazo[1,2-a]indéno[1,2-e]pyrazine- 10:1'-cyclopropane]-4-one,
spiro[5H,10H-im idazo[1,2-alindéno[1,2-e]pyrazine-10:1'-cyclopentane]-4-one,
- 10-méthyl-5H,10H-imidazo[1,2-a]indéno[1,2-e]pyrazine-4-one,
- 10,10-dibenzyl-5H,10H-imidazo[1,2-a]indéno[1,2-e]pyrazine-4-one,
- 10-hydroxyméthyl-5H,10H-imidazo[1,2-a]indéno[1,2-e]pyrazine-4-one,
- 10-(2-furylméthyl)-5H,10H-imidazo[1,2-a]indéno[1,2-e]pyrazine-4-one,
- 10-(4-pyridylméthylène)-5H,10H-imidazo[1,2-a]indéno[1,2-e]pyrazine-4-one,
- 10-(4-pyridylméthyl)-5H,10H-imidazo[1,2-a]indéno[1,2-e]pyrazine-4-one,
- 10-(phénylpropyl)-5H,10H-imidazo[1,2-a]indéno[1,2-e]pyrazine-4-one,
- 10-(3-pyridylméthylène)-5H,10H-imidazo[1,2-a]indéno[1,2-e]pyrazine-4-one,
- 10-(3-pyridylméthyl)-5H,10H-imidazo[1,2-a]indéno[1,2-e]pyrazine-4-one,
- 10-(2-pyridylméthyl)-5H,10H-imidazo[1,2-a]indéno[1,2-e]pyrazine-4-one,
- 10-(2-imidazolylméthyl)-5H,10H-imidazo[1,2-a]indéno[1,2-e]pyrazine-4-one,
- (4-oxo-5H,10H-imidazo[1,2-a]indéno[1,2-e]pyrazine-10-ylidène)amino oxyacétate de tert-butyle
- 10-isobutyl-5H,10H-imidazo[1,2-a]indéno[1,2-e]pyrazine-4-one,
- acide (4-oxo-5H,10H-imidazo[1,2-a]indéno[1,2-e]pyrazine-10-ylidène)amino oxyacétique,
- 10-propionamido-5H,10H-imidazo[1,2-a]indéno[1,2-e]pyrazine-4-one,
- 10-amino-8-fluoro-5H,10H-imidazo[1,2-a]indéno[1,2-e]pyrazine-4-one,
- 10-(4-quinolinylméthylène)-5H,10H-imidazo[1,2-a]indéno[1,2-e]pyrazine-4-one,
- 10-(4-quinolinylméthyl)-5H,10H-imidazo[1,2-a]indéno[1,2-e]pyrazine-4-one,
- 10-(3-phényluréido)-5H,10H-imidazo[1,2-a]indéno[1,2-e]pyrazine-4-one,
- 10-(3-méthyluréido)-5H,10H-imidazo[1,2-a]indéno[1,2-e]pyrazine-4-one,
- 10-isobutyramido-5H,10H-imidazo[1,2-a]indéno[1,2-e]pyrazine-4-one,
- 10-amino-7-chloro-5H,10H-imidazo[1,2-a]indéno[1,2-e]pyrazine-4-one,
- 10-benzènesulfonylamido-5H,10H-imidazo[1,2-a]indéno[1,2-e]pyrazine-4-one,
- 10-méthylamino-5H,10H-imidazo[1,2-a]indéno[1,2-e]pyrazine-4-one,
- 10-(2-pyrazinylméthylène)-5H,10H-imidazo[1,2-a]indéno[1,2-e]pyrazine-4-one,
- 10-(2-pyrazinylméthyl)-5H,10H-imidazo[1,2-a]indéno[1,2-e]pyrazine-4-one,
- 10-benzyl-7-chloro-5H,10H-imidazo[1,2-a]indéno[1,2-e]pyrazine-4-one,
- 7-chloro-10-(3-phényluréido)-5H,10H-imidazo[1,2-a]indéno[1,2-e]pyrazine-4-one,
- 10-(2-hydroxyéthyl)-5H,10H-imidazo[1,2-a]indéno[1,2-e]pyrazine-4-one,
- 7-chloro-10-méthylamino-5H,10H-imidazo[1,2-a]indéno[1,2-e]pyrazine-4-one,
- 10-benzyl-10-méthyl-5H,10H-imidazo[1,2-a]indéno[1,2-e]pyrazine-4-one,
- 10-méthyl-10-(4-pyridylméthyl)-5H,10H-imidazo[1,2-a]indéno[1,2-e]pyrazine-4-one,
- 10-(4-pipéridylméthyl)-5H,10H-imidazo[1,2-a]indéno[1,2-e]pyrazi ne-4-one,
- 5H,10H-imidazo[1,2-a]indéno[1,2-e]pyrazine-4,10-dione,
- 10-benzyl-5H,10H-imidazo[1,2-a]indéno[1,2-e]pyrazine-4-one,
- 10-hexyl-5H,10H-imidazo[1,2-a]indéno[1,2-e]pyrazine-4-one,
- 10-benzamido-5H,10H-imidazo[1,2-a]indéno[1,2-e]pyrazine-4-one,
- 10-méthyl-10-[2-(pyridine-4-yl)éthyl]-5H,10H-imidazo[1,2-a]indéno[1,2-e] pyrazine-4-one et leurs sels.

4. Procédé de préparation des composés de formule (I) selon la revendication 1 pour lesquels R représente un radical C=R₃ dans lequel R₃ représente un atome d'oxygène caractérisé en ce que l'on hydrolyse un composé de formule (I) correspondant pour lequel R représente un radical C=R₃ et R₃ représente un radical NOH, isole le produit et le transforme éventuellement en sel.

5. Procédé de préparation des composés de formule (I) selon la revendication 1 pour lesquels R représente un radical C=R₃ et R₃ représente un radical NOH caractérisé en ce que l'on fait réagir un nitrite d'alkyle sur un dérivé de formule : 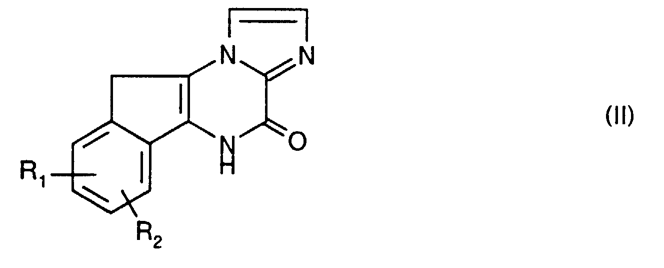 dans laquelle R₁ et R₂ ont les mêmes significations que dans la revendication 1, isole le produit et le transforme éventuellement en sel.

6. Procédé de préparation des composés de formule (I) selon la revendication 1 pour lesquels R représente un radical C=R₃ et R₃ représente un radical NO-alk-COOX caractérisé en ce que l'on fait réagir un composé de formule (I) correspondant pour lequel R représente un radical C=R₃ et R₃ représente un radical NOH sur un halogénure Hal-alk-COOX pour lequel Hal représente un atome d'halogène, alk représente un radical alkyle et X représente un radical alkyle, suivie éventuellement de la libération de la fonction carboxy par action de l'acide trifluoroacétique, isole le produit et le transforme éventuellement en sel.

7. Procédé de préparation des composés de formule (I) selon la revendication 1 pour lesquels R représente un radical C=R₃ et R₃ représente un radical CH-R₇ dans lequel R₇ représente un radical hydroxy caractérisé en ce que l'on hydrolyse un composésde formule (I) correspondant pour lequel R₇ représente un radical -NR₁₀R₁₁, isole le produit et le transforme éventuellement en sel.

8. Procédé de préparation des composés de formule (I) selon la revendication 1 pour lesquels R représente un radical C=R₃, R₃ représente un radical CH-R₇ et R₇ représente un radical -NR₁₀R₁₁ caractérisé en ce que l'on fait réagir un dérivé de formule : 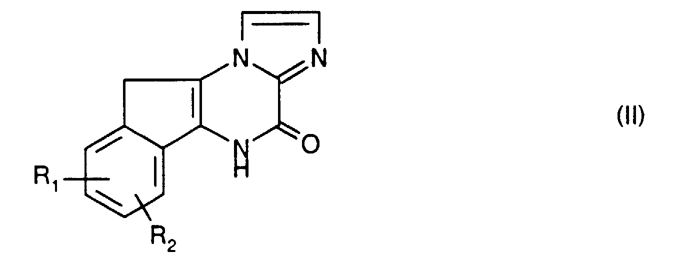 dans laquelle R₁ et R₂ ont les mêmes significations que dans la revendication 1 sur un dérivé de formule : 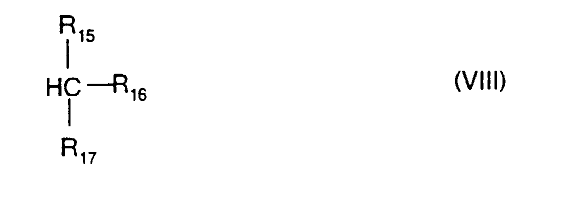 dans laquelle soit R₁₅ et R₁₇, identiques ou différents, représentent chacun un radical -NR₁₀R₁₁ et R₁₆ représente un radical alcoxy tel que tert-butoxy, soit R₁₅, R₁₆ et R₁₇, identiques, représentent chacun un radical -NR₁₀R₁₁, isole le produit et le transforme éventuellement en sel.

9. Procédé de préparation des composés de formule (I) selon la revendication 1 pour lesquels R représente un radical C=R₃, R₃ représente un radical CH-R₇ et R₇ représente un radical alkyle, phényle, -alk-Het, -alk-Ar ou un hétérocycle caractérisé en ce que l'on fait réagir un dérivé de formule : 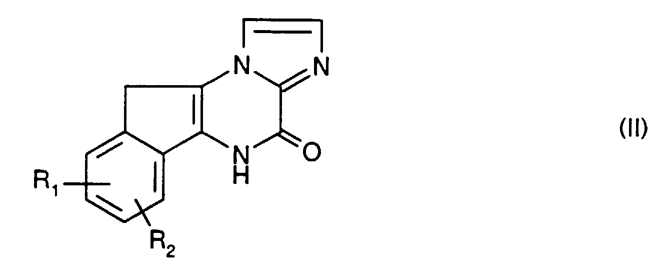 dans laquelle R₁ et R₂ ont les mêmes significations que dans la revendication 1 sur un aldéhyde de formule :
OHC-R₁₈ (IX)
dans laquelle R₁₈ représente un radical alkyle, phényle, -alk-Het, -alk-Ar ou Het, isole le produit et le transforme éventuellement en sel.

10. Procédé de préparation des composés de formule (I) selon la revendication 1 pour lesquels R représente un radical C(R₄)R₅, R₄ représente un radical alkyle, -alk-Het ou -alk-Ar et R₅ est identique à R₄ caractérisé en ce que l'on fait réagir un dérivé de formule : 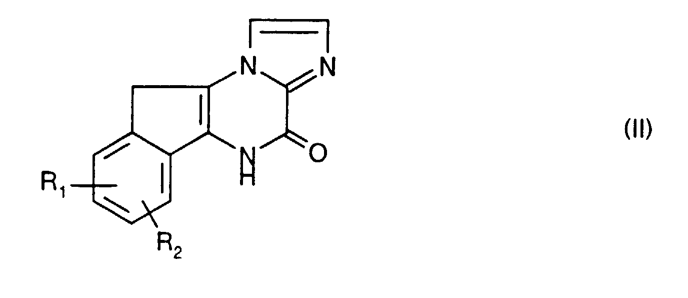 dans laquelle R₁ et R₂ ont les mêmes significations que dans la revendication 1 sur un halogénure de formule :
Hal-R₁₉ (X)
dans laquelle R₁₉ représente un radical alkyle, -alk-Het ou -alk-Ar, isole le produit et le transforme éventuellement en sel.

11. Procédé de préparation des composés de formule (I) selon la revendication 1 pour lesquels R représente un radical C(R₄)R₅, R₄ représente un radical alkyle, -alk-Het ou -alk-Ar et R₅ représente un radical alkyle (1-11C en chaîne droite ou ramifiée), -alk-Het ou -alk-Ar, caractérisé en ce que l'on fait réagir un dérivé de formule : 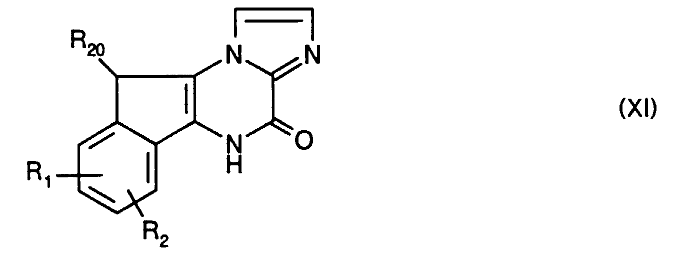 dans laquelle R₁ et R₂ ont les mêmes significations que dans la revendication 1 et R₂₀ représente un radical alkyle, -alk-Het ou -alk-Ar ou le dérivé silylé de ce dérivé sur un halogénure de formule :
Hal-R₂₁ (XII)
dans laquelle R₂₁ représente un radical alkyle (1-11C en chaîne droite ou ramifiée), -alk-Het ou -alk-Ar, isole le produit et le transforme éventuellement en sel.

12. Procédé de préparation des composés de formule (I) selon la revendication 1 pour lesquels R représente un radical C(R₄)R₅ et R₄ et R₅ forment avec l'atome de carbone auquel ils sont rattachés un radical cycloalkyle caractérisé en ce que l'on fait réagir un dérivé de formule : 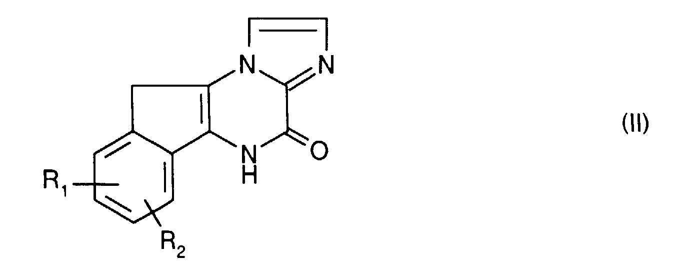 dans laquelle R₁ et R₂ ont les mêmes significations que dans la revendication 1 sur un dérivé de formule :
Hal-alk-Hal (XIII)
dans laquelle Hal représente un atome d'halogène et alk représente un radical alkyle (2-5C), isole le produit et le transforme éventuellement en sel.

13. Procédé de préparation des composés de formule (I) selon la revendication 1 pour lesquels R représente un radical CH-R₆ dans lequel R₆ représente un radical hydroxy caractérisé en ce que l'on réduit un composé de formule (I) correspondant pour lesquels R représente un radical C=R₃ et R₃ représente un atome d'oxygène, isole le produit et le transforme éventuellement en sel.

14. Procédé de préparation des composés de formule (I) selon la revendication 1 pour lesquels R représente un radical CH-R₆ dans lequel R₆ représente un radical alkyle (2-11C), -alk-Ar, -alk-Het ou 2-imidazolylméthyle caractérisé en ce que l'on hydrogène un dérivé de formule : 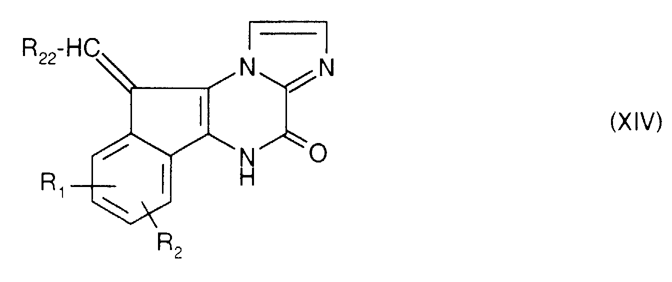 dans laquelle R₁ et R₂ ont les mêmes significations que dans la revendication 1, R₂₂ représente un radical 2-imidazolyle, alkyle en chaîne droite ou ramifiée contenant 1 à 10 atomes de carbone, phényle, -alk-Ar, -alk-Het dans lesquels la portion alkyle est en chaîne droite ou ramifiée et contient 1 à 3 atomes de carbone ou un hétérocycle mono ou polycyclique saturé ou insaturé contenant 4 à 9 atomes de carbone et un ou plusieurs hétéroatomes (O, S, N), isole le produit et le transforme éventuellement en sel.

15. Procédé de préparation des composés de formule (I) selon la revendication 1 pour lesquels R représente un radical CH-R₆ dans lequel R₆ représente un radical méthyle caractérisé en ce que l'on réduit un composé de formule (I) correspondant pour lequel R représente un radical C=R₃, R₃ représente un radical CH-R₇ et R₇ représente un radical hydroxy ou -NR₁₀R₁₁, isole le produit et le transforme éventuellement en sel.

16. Procédé de préparation des composés de formule (I) selon la revendication 1 pour lesquels R représente un radical CH-R₆ et R₆ représente un radical -alk(1C)-OH caractérisé en ce que l'on réduit un composé de formule (I) correspondant pour lequel R représente un radical C=R₃, R₃ représente un radical CH-R₇ et R₇ représente un radical hydroxy, isole le produit et le transforme éventuellement en sel.

17. Procédé de préparation des composés de formule (I) selon la revendication 1 pour lesquels R représente un radical CH-R₆ et R₆ représente un radical -alk(2-4C)-OH caractérisé en ce que l'on réduit un dérivé de formule : 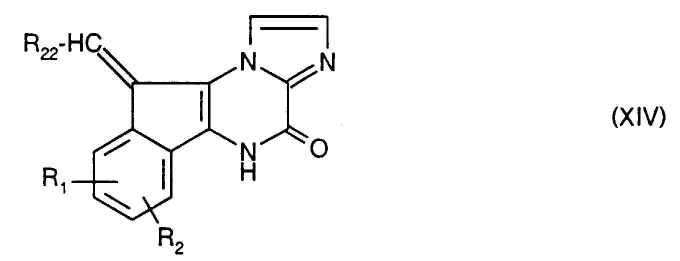 pour lequel R₁ et R₂ ont les mêmes significations que dans la revendication 1 et R₂₂ représente un radical -alk(1-3C)-O-CH₂-Ar.

18. Procédé de préparation des composés de formule (I) selon la revendication 1 pour lesquels R représente un radical CH-R₆ et R₆ représente un radical -alk(2-4C)-OH caractérisé en ce que l'on fait réagir le (COCl)₂ sur un dérivé de formule : 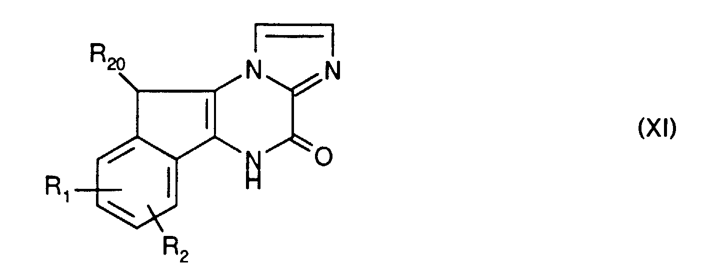 pour lequel R₁ et R₂ ont les mêmes significations que dans la revendication 1 et R₂₀ représente un radical -alk-COOH suivie d'une réduction, isole le produit et le transforme éventuellement en sel.

19. Procédé de préparation des composés de formule (I) selon la revendication 1 pour lesquels R représente un radical CH-R₆ dans lequel R₆ représente un radical -NR₈R₉, R₈ et R₉ représentent chacun un atome d'hydrogène caractérisé en ce que l'on hydrolyse un composé de formule (I) correspondant pour lequel R représente un radical CH-R₆ dans lequel R₆ représente un radical -NR₈R₉, R₈ représente un atome d'hydrogène et R₉ représente un radical -COR₁₂ et R₁₂ représente un radical alkyle, isole le produit et le transforme éventuellement en sel.

20. Procédé de préparation des composés de formule (I) selon la revendication 1 pour lesquels R représente un radical CH-R₆ dans lequel R₆ représente un radical -NR₈R₉, R₈ et R₉ représentent chacun un atome d'hydrogène caractérisé en ce que l'on réduit un composé de formule (I) correspondant pour lequel R représente un radical C=R₃ et R₃ représente un radical NOH, isole le produit et le transforme éventuellement en sel.

21. Procédé de préparation des composés de formule (I) selon la revendication 1 pour lesquels R représente un radical CH-R₆ dans lequel R₆ représente un radical -NR₈R₉, R₈ représente un atome d'hydrogène et R₉ représente un radical -COR₁₂ et R₁₂ représente un radical alkyle caractérisé en ce que l'on fait réagir un acide de formule :
R₂₄-COOH (XV)
dans laquelle R₂₄ représente un radical alkyle (1-3C) sur un composé de formule (I) correspondant pour lequel R représente un radical C=R₃ et R₃ représente un radical NOH, en présence d'un agent réducteur, isole le produit et le transforme éventuellement en sel.

22. Procédé de préparation des composés de formule (I) selon la revendication 1 pour lesquels R représente un radical CH-R₆ et R₆ représente un radical -NR₈R₉ ou -alk-NR₈R₉, dans lesquels R₈ et R₉, identiques ou différents, représentent chacun un radical alkyle ou bien R₈ représente un atome d'hydrogène et R₉ représente un radical alkyle, -COR₁₂ ou -SO₂R₁₃ et R₁₂ représente un radical alkyle, cycloalkyle, phényle, -COO-alk ou -CH₂-COOX caractérisé en ce que l'on fait réagir un composé de formule (I) correspondant pour lequel R₈ et R₉ représentent chacun un atome d'hydrogène ou bien R₈ représente un atome d'hydrogène et R₉ représente un radical alkyle, sur un halogénure de formule :
Hal-R₂₇ (XVI)
dans laquelle R₂₇ représente un radical alkyle, -COR₁₂ ou -SO₂R₁₃ et R₁₂ représente un radical alkyle, cycloalkyle, phényle, -COO-alk ou -CH₂-COOX et R₁₃ a les mêmes significations que dans la revendication 1, isole le produit et le transforme éventuellement en sel.

23. Procédé de préparation des composés de formule (I) selon la revendication 1 pour lesquels R représente un radical CH-R₆ et R₆ représente un radical -NR₈R₉ ou -alk-NR₈R₉ dans lesquels R₈ représente un atome d'hydrogène, R₉ représente un radical -COR₁₂ ou -CSR₃₀ et R₁₂ et R₃₀ représentent un radical -NH-alk, -NH₂, -NH-Ar ou -NH-Het caractérisé en ce que l'on fait réagir un composé de formule (I) correspondant pour lequel R₈ et R₉ représentent chacun un atome d'hydrogène sur un dérivé R₂₈-N=C=R₃₁ pour lequel R₂₈ représente un radical triméthylsilyle, benzoyle, alkyle, phényle ou un hétérocycle mono ou polycyclique saturé ou insaturé contenant 4 à 9 atomes de carbone et un ou plusieurs hétéroatomes (O, S, N) et R₃₁ représente un atome d'oxygène ou de soufre, suivie éventuellement par une hydrolyse, isole le produit et le transforme éventuellement en sel.

24. Procédé de préparation des composés de formule (I) selon la revendication 1 pour lesquels R représente un radical CH-R₆ et R₆ représente un radical -NR₈R₉ ou -alk-NR₈R₉ dans lesquels R₈ représente un atome d'hydrogène, R₉ représente un radical -COR₁₂ et R₁₂ représente un radical -CH₂-NH₂ caractérisé en ce que l'on fait réagir un composé de formule (I) correspondant pour lequel R₈ et R₉ représentent chacun un atome d'hydrogène sur un acide HOOC-CH₂-NH-R₂₉ dans lequel R₂₉ représente un groupe protecteur de la fonction amine tel que tert-butoxycarbonyle suivie d'une hydrolyse, isole le produit et le transforme éventuellement en sel.

25. Procédé de préparation des composés de formule (I) selon la revendication 1 pour lesquels R représente un radical CH-R₆ dans lequel R₆ représente un radical -alk-Ar ou -alk-Het caractérisé en ce que l'on fait réagir un dérivé de formule : 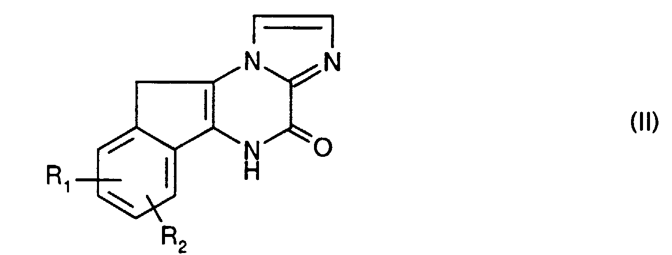 sur un halogénure de formule :
Hal-R₂₅ (XVII)
dans laquelle R₂₅ représente un radical -alk-Ar ou -alk-Het, isole le produit et le transforme éventuellement en sel.

26. Procédé de préparation des composés de formule (I) selon la revendication 1 pour lesquels R représente un radical CH-R₆, R₆ représente un radical -alk-NR₈R₉, R₈ et R₉ représentent chacun un atome d'hydrogène caractérisé en ce que l'on fait réagir du brome et dela soude sur un dérivé de formule : 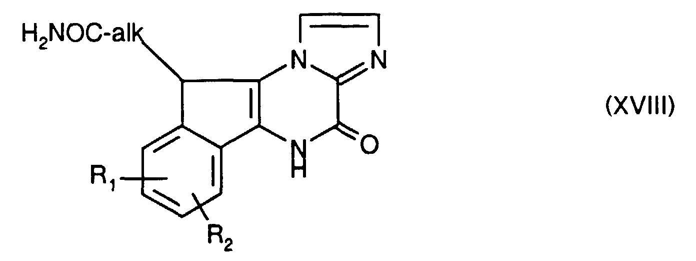 dans laquelle R₁ et R₂ ont les mêmes significations que dans la revendication 1 et alk représente un radical alkyle, isole le produit et le transforme éventuellement en sel.

27. Procédé de préparation des composés de formule (I) selon la revendication 1 pour lesquels R représente un radical CH-R₆, R₆ représente un radical -NR₈R₉ ou -alk-NR₈R₉, R₈ représente un atome d'hydrogène et R₉ représente un radical alkyle caractérisé en ce que l'on réduit un dérivé de formule : 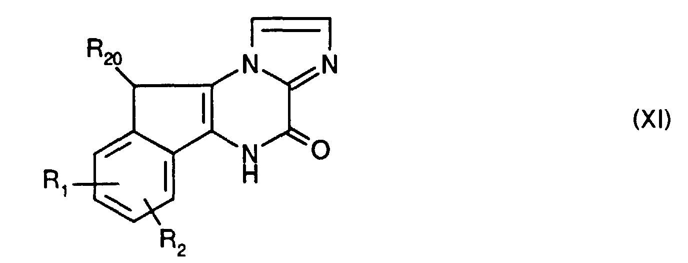 pour lequel R₁ et R₂ ont les mêmes significations que dans la revendication 1 et R₂₀ représente un radical -NR₈R₉ ou -alk-NR₈R₉, R₈ représente un atome d'hydrogène et R₉ représente un radical -CHO ou -COR₁₂ dans lequel R₁₂ représente un radical alkyle ou cycloalkyle, isole le produit et le transforme éventuellement en sel.

28. Procédé de préparation des composés de formule (I) selon la revendication 1 pour lesquels soit R représente un radical C(R₄)R₅ dans lequel R₄ et/ou R₅ représente un radical -alk-Het, soit R représente un radical CH-R₆, R₆ représente un radical -alk-Het dans lesquels Het représente un radical pipéridyle caractérisé en ce que l'on hydrogène un composé de formule (I) correspondant pour lequel soit R représente un radical C(R₄)R₅ dans lequel R₄ et/ou R₅ représente un radical -alk-Het, soit R représente un radical CH-R₆, R₆ représente un radical -alk-Het dans lesquels Het représente un radical pyridyle, isole le produit et le transforme éventuellement en sel.

29. Procédé de préparation des composés de formule (I) selon la revendication 1 pour lesquels R représente un radical C(R₄)R₅, R₄ représente un radical -alk-Het dans lequel alk est un radical éthyle et Het est un radical 4-pyridyl ou 2-pyridyl et R₅ représente un radical alkyle (1-11C en chaîne droite ou ramifiée), -alk-Het ou -alk-Ar caractérisé en ce que l'on fait réagir un dérivé de formule : 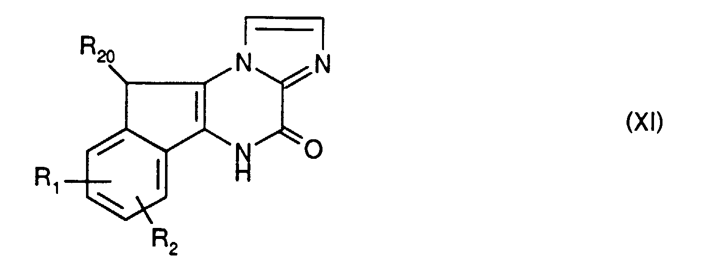 dans laquelle R₁ et R₂ ont les mêmes significations que dans la revendication 1 et R₂₀ représente un radical alkyle (1-11C en chaîne droite ou ramifiée), -alk-Het ou -alk-Ar sous sa forme silylée, sur la 2- ou 4-vinylpyridine, isole le produit et le transforme éventuellement en sel.

30. Médicaments contenant en tant que principe actif au moins un composé de formule (I) selon l'un des revendications 1 à 3.

31. Médicaments selon la revendication 30 utiles comme antagonistes du récepteur AMPA.

32. Médicaments selon la revendication 30 utiles comme antagonistes du récepteur NMDA.

## Patentansprüche

1. Verbindungen der Formel (I) 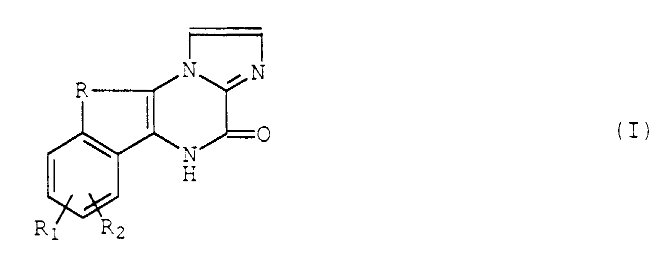 in der entweder
R einen Rest C=R₃, C(R₄)R₅ oder CH-R₆ darstellt,
- R₁ und R₂, gleich oder verschieden, Wasserstoffatome oder Halogenatome oder Reste Alkyl, Alkoxy, Amino, Acylamino, -NH-CO-NH-Ar, -N=CH-N(alk)alk', Nitro, Cyano, Phenyl, Imidazolyl oder SO₃H bedeuten,
- R₃ ein Sauerstoffatom oder ein Rest NOH, NO-alk-COOX oder CH-R₇ ist,
- R₄ einen Rest Alkyl, -alk-Het oder -alk-Ar darstellt,
- R₅ einen Rest Alkyl (1 bis 11 Kohlenstoffatome in gerader oder verzweigter Kette), -alk-Het oder -alk-Ar bedeutet,
oder R₄ und R₅ zusammen mit dem Kohlenstoffatom, an das sie gebunden sind, einen Rest Cycloalkyl bilden,
- R₆ einen Rest Hydroxy, Alkyl (1 bis 11 Kohlenstoffatome in gerader oder verzweigter Kette) -NR₈R₉, -alk-OH, -alk-NR₈R₉, -alk-Ar oder -alk-Het darstellt,
- R₇ einen Rest Hydroxy, Alkyl, Phenyl, -alk-Ar, -alk-Het, -NR₁₀R₁₁ oder einen gesättigten oder ungesättigten, monocyclischen oder polycyclischen Heterocyclus mit 4 bis 9 Kohlenstoffatomen und einem oder mehreren Heteroatomen (O,S,N) bedeutet,
- R₈ und R₉, gleich oder verschieden, jeweils einen Rest Alkyl darstellen oder auch R₈ ein Wasserstoffatom ist und R₉ ein Wasserstoffatom oder einen Rest Alkyl, -COR₁₂, -CSR₃₀ oder -SO₂R₁₃ bedeutet,
- R₁₀ und R₁₁, gleich oder verschieden, jeweils einen Rest Alkyl oder Cycloalkyl darstellen,
- R₁₂ einen Rest Alkyl, Cycloalkyl, Phenyl, -COO-alk, -CH₂-COOX, -CH₂NH₂, -NH-alk, -NH₂, -NH-Ar oder -NH-Het bedeutet,
- R₁₃ ein Rest Alkyl oder Phenyl ist,
- R₃₀ einen Rest -NH-alk, -NH-Ar, -NH₂ oder -NH-Het darstellt,
- alk einen Rest Alkyl oder Alkylen bedeutet,
- alk' ein Rest Alkyl ist,
- X ein Wasserstoffatom oder einen Rest Alkyl darstellt,
- Ar ein Rest Phenyl ist und
- Het einen gesättigten oder ungesättigten, monocyclischen oder polycyclischen Heterocyclus mit 4 bis 9 Kohlenstoffatomen und einem oder mehreren Heteroatomen (O,S,N) bedeutet;
oder auch
R einen Rest CH-R₆ darstellt und R₆ ein Rest 2-Imidazolylmethyl ist sowie R₁ und R₂ jeweils ein Wasserstoffatom bedeuten, mit der Maßgabe, daß die Reste und Teile Alkyl und Alkylen 1 bis 4 Kohlenstoffatome in gerader oder verzweigter Kette enthalten, die Teile Acyl 2 bis 4 Kohlenstoffatome enthalten, die Reste Cycloalkyl 3 bis 6 Kohlenstoffatome enthalten, sowie die Isomeren der Verbindungen, worin R₃ einen Rest NOH, NO-alk-COOX oder CH-R₇ darstellt, die Racemate und die Enantiomeren der Verbindungen der Formel (I), in der R einen Rest C(R₄)R₅ bedeutet, worin R₄ von R₅ verschieden ist oder CH-R₆ darstellt, und ihre Salze.

2. Verbindungen der Formel (I) nach Anspruch 1, in der entweder R einen Rest C=R₃, C(R₄)R₅ oder CH-R₆ darstellt, R₁ und R₂ Wasserstoffatome oder Halogenatome bedeuten, R₃ ein Sauerstoffatom oder ein Rest NO-alk-COOX oder CH-R₇ ist, R₄ einen Rest Alkyl oder -alk-Ar darstellt, R₅ einen Rest Alkyl (1 bis 11 Kohlenstoffatome in gerader oder verzweigter Kette), -alk-Het oder -alk-Ar bedeutet, oder R₄ und R₅ zusammen mit dem Kohlenstoffatom, an das sie gebunden sind, einen Rest Cycloalkyl bilden, R₆ einen Rest Hydroxy, Alkyl (1 bis 11 Kohlenstoffatome in gerader oder verzweigter Kette), -NR₈R₉, -alk-NR₈R₉, -alk-OH, -alk-Ar oder -alk-Het darstellt, R₇ einen Rest Hydroxy, -NR₁₀R₁₁ oder einen gesättigten oder ungesättigten, monocyclischen oder polycyclischen Heterocyclus mit 4 bis 9 Kohlenstoffatomen und einem oder mehreren Heteroatomen (O,S,N) bedeutet, R₈ ein Wasserstoffatom ist und R₉ ein Wasserstoffatom oder einen Rest Alkyl, -COR₁₂ oder -SO₂R₁₃ bedeutet, R₁₀ und R₁₁, gleich oder verschieden, jeweils einen Rest Alkyl darstellen, R₁₂ einen Rest Alkyl, -NH-Ar, -NH-alk oder Phenyl bedeutet, R₁₃ ein Rest Alkyl oder Phenyl ist, alk einen Rest Alkyl oder Alkylen bedeutet, X ein einen Rest Alkyl darstellt und Het einen gesättigten oder ungesättigten, monocyclischen oder polycyclischen Heterocyclus mit 4 bis 9 Kohlenstoffatomen und einem oder mehreren Heteroatomen (O,S,N) bedeutet; oder auch R einen Rest CH-R₆ darstellt und R₆ ein Rest 2-Imidazolylmethyl ist sowie R₁ und R₂ jeweils ein Wasserstoffatom bedeuten, sowie die Isomeren der Verbindungen, worin R₃ einen Rest NO-alk-COOX oder CH-R₇ darstellt, die Racemate und die Enantiomeren der Verbindungen der Formel (I), in der R einen Rest C(R₄)R₅ bedeutet, worin R₄ von R₅ verschieden ist oder CH-R₆ darstellt, und ihre Salze.

3. Verbindungen der Formel (I) nach Anspruch 1, ausgewählt unter den folgenden Verbindungen:
- 10-Hydroxy-5H,10H-imidazo[1,2-a]-indeno[1,2-e]-pyrazin-4-on,
- 10-Acetamido-5H,10H-imidazo[1,2-a]-indeno[1,2-e]-pyrazin-4-on,
- 10-Amino-5H,10H-imidazo[1,2-a]-indeno[1,2-e]-pyrazin-4-on,
- 10-(E-Dimethylaminomethylen)5H,10H-imidazo[1,2-a]-indeno-[1,2-e]-pyrazin-4-on,
- 10-Hydroxymethylen-5H,10H-imidazo[1,2-a]-indeno[1,2-e]-pyrazin-4-on,
- 10,10-Dimethyl-5H,10H-imidazo[1,2-a]-indeno[1,2-e]-pyrazin-4-on,
- Spiro-{5H,10H-imidazo[1,2-a]-indeno[1,2-e]-pyrazin-10:1'-cyclopropan}-4-on,
- Spiro-{5H,10H-imidazo[1,2-a]-indeno[1,2-e]-pyrazin-10:1'-cyclopentan}-4-on,
- 10-Methyl-5H,10H-imidazo[1,2-a]-indeno[1,2-e]-pyrazin-4-on,
- 10,10-Dibenzyl-5H,10H-imidazo[1,2-a]-indeno[1,2-e]-pyrazin-4-on,
- 10-Hydroxymethyl-5H,10H-imidazo[1,2-a]-indeno[1,2-e]-pyrazin-4-on,
- 10-(2-Furylmethyl)-5H,10H-imidazo[1,2-a]-indeno[1,2-e]-pyrazin-4-on,
- 10-(4-Pyridylmethylen)-5H,10H-imidazo[1,2-a]-indeno[1,2-e]-pyrazin-4-on,
- 10-(4-Pyridylmethyl)-5H,10H-imidazo[1,2-a]-indeno[1,2-e]-pyrazin-4-on,
- 10-(Phenylpropyl)-5H,10H-imidazo[1,2-a]-indeno[1,2-e]-pyrazin4-on,
- 10-(3-Pyridylmethylen)-5H,10H-imidazo[1,2-a]-indeno[1,2-e]-pyrazin-4-on,
- 10-(3-Pyridylmethyl)-5H,10H-imidazo[1,2-a]-indeno[1,2-e]-pyrazin-4-on,
- 10-(2-Pyridylmethyl)-5H,10H-imidazo[1,2-a]-indeno[1,2-e]-pyrazin-4-on,
- 10-(2-Imidazolylmethyl)-5H,10H-imidazo[1,2-a]-indeno[1,2-e]pyrazin-4-on,
- (4-Oxo-5H,10H-imidazo[1,2-a]-indeno[1,2-e]-pyrazin-10-yliden)amino-oxyessigsäure-tert.-butylester,
- 10-Isobutyl-5H,10H-imidazo[1,2-a]-indeno[1,2-e]-pyrazin-4-on, (4-Oxo-5H,10H-imidazo[1,2-a]-indeno[1,2-e]-pyrazin-10-yliden)-amino-oxyessigsäure,
- 10-Propionamido-5H,10H-imidazo[1,2-a]-indeno[1,2-e]-pyrazin-4-on,
- 10-Amino-8-fluor-5H,10H-imidazo[1,2-a]-indeno[1,2-e]-pyrazin-4-on,
- 10-(4-Chinolinylmethylen)-5H,10H-imidazo[1,2-a]-indeno[1,2-e]-pyrazin-4-on,
- 10-(4-Chinolinylmethyl)-5H,10H-imidazo[1,2-a]-indeno[1,2-e]-pyrazin-4-on,
- 10-(3-Phenylureido)-5H,10H-imidazo[1,2-a]-indeno[1,2-e]-pyrazin-4-on,
- 10-(3-Methylureido)-5H,10H-imidazo[1,2-a]-indeno[1,2-e]-pyrazin-4-on,
- 10-Isobutyramido-5H,10H-imidazo[1,2-a]-indeno[1,2-e]-pyrazin-4-on,
- 10-Amino-7-chlor-5H,10H-imidazo[1,2-a]-indeno[1,2-e]-pyrazin-4-on,
- 10-Benzolsulfonylamido-5H,10H-imidazo[1,2-a]-indeno[1,2-e]-pyrazin-4-on,
- 10-Methylamino-5H,10H-imidazo[1,2-a]-indeno[1,2-e]-pyrazin-4-on,
- 10-(2-Pyrazinylmethylen)-5H,10H-imidazo[1,2-a]-indeno[1,2-e]-pyrazin-4-on,
- 10-(2-Pyrazinylmethyl)-5H,10H-imidazo[1,2-a]-indeno[1,2-e]-pyrazin-4-on,
- 10-Benzyl-7-chlor-5H,10H-imidazo[1,2-a]-indeno[1,2-e]-pyrazin-4-on,
- 7-Chlor-10-(3-phenylureido)-5H,10H-imidazo[1,2-a]-indeno-[1,2-e]-pyrazin-4-on,
- 10-(2-Hydroxyethyl)-5H,10H-imidazo[1,2-a]-indeno[1,2-e]-pyrazin-4-on,
- 7-Chlor-10-methylamino-5H,10H-imidazo[1,2-a]-indeno[1,2-e]-pyrazin-4-on,
- 10-Benzyl-10-methyl-5H,10H-imidazo[1,2-a]-indeno[1,2-e]-pyrazin-4-on,
- 10-Methyl-10-(4-pyridylmethyl)-5H,10H-imidazo[1,2-a]-indeno-[1,2-e]-pyrazin-4-on,
- 10-(4-Piperidylmethyl)-5H,10H-imidazo[1,2-a]-indeno[1,2-e]-pyrazin-4-on,
- 5H,10H-Imidazo[1,2-a]-indeno[1,2-e]-pyrazin-4,10-dion,
- 10-Benzyl-5H,10H-imidazo[1,2-a]-indeno[1,2-e]-pyrazin-4-on,
- 10-Hexyl-5H,10H-imidazo[1,2-a]-indeno[1,2-e]-pyrazin-4-on,
- 10-Benzamido-5H,10H-imidazo[1,2-a]-indeno[1,2-e]-pyrazin-4-on,
- 10-Methyl-10-[2-(pyridin-4-yl)-ethyl]-5H,10H-imidazo[1,2-a]-indeno[1,2-e]-pyrazin-4-on und ihre Salze.

4. Verfahren zur Herstellung der Verbindungen der Formel (I) nach Anspruch 1, in der R einen Rest C=R₃ darstellt, worin R₃ ein Sauerstoffatom ist, dadurch gekennzeichnet, daß man eine entsprechende Verbindung der Formel (I), in der R einen Rest C=R₃ darstellt, worin R₃ ein Rest NOH ist, hydrolysiert, das Produkt isoliert und gegebenenfalls in Salz umwandelt.

5. Verfahren zur Herstellung der Verbindungen der Formel (I) nach Anspruch 1, in der R einen Rest C=R₃ darstellt, worin R₃ ein Rest NOH ist, dadurch gekennzeichnet, daß man ein Alkylnitrit mit einem Derivat der Formel (II) 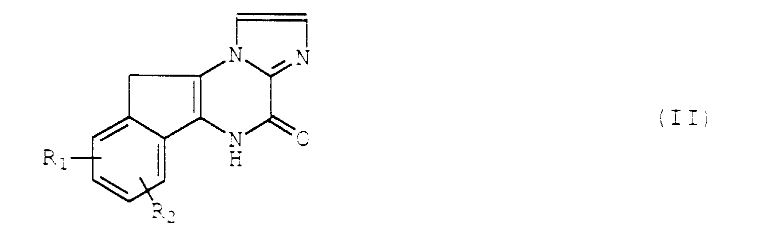 in der R₁ und R₂ die gleichen Bedeutungen wie in Anspruch 1 besitzen, zur Reaktion bringt, das Produkt isoliert und gegebenenfalls in Salz umwandelt.

6. Verfahren zur Herstellung der Verbindungen der Formel (I) nach Anspruch 1, in der R einen Rest C=R₃ darstellt, worin R₃ ein Rest NO-alk-COOX ist, dadurch gekennzeichnet, daß man eine entsprechende Verbindung der Formel (I), in der R einen Rest C=R₃ darstellt, worin R₃ ein Rest NOH ist, mit einem Halogenid Hal-alk-COOX, worin Hal ein Halogenatom ist, alk einen Rest Alkyl darstellt und X einen Rest Alkyl darstellt, zur Reaktion bringt, gegebenenfalls gefolgt von der Freisetzung der Funktion Carboxy durch Einwirkung von Trifluoressigsäure, das Produkt isoliert und gegebenenfalls in Salz umwandelt.

7. Verfahren zur Herstellung der Verbindungen der Formel (I) nach Anspruch 1, in der R einen Rest C=R₃ darstellt, worin R₃ ein Rest CH-R₇ ist und R₇ einen Rest Hydroxy bedeutet, dadurch gekennzeichnet, daß man eine entsprechende Verbindung der Formel (I), in der R₇ einen Rest -NR₁₀R₁₁ darstellt, hydrolysiert, das Produkt isoliert und gegebenenfalls in Salz umwandelt.

8. Verfahren zur Herstellung der Verbindungen der Formel (I) nach Anspruch 1, in der R einen Rest C=R₃ darstellt, worin R₃ ein Rest CH-R₇ ist und R₇ einen Rest -NR₁₀R₁₁ bedeutet, dadurch gekennzeichnet, daß man ein Derivat der Formel (II) 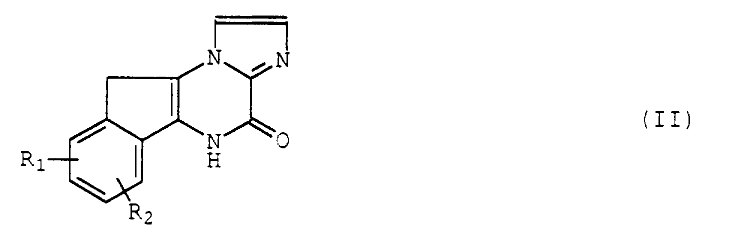 in der R₁ und R₂ die gleichen Bedeutungen wie in Anspruch 1 besitzen, mit einem Derivat der Formel (VIII) 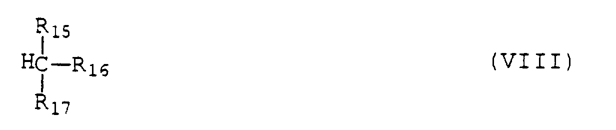 in der entweder R₁₅ und R₁₇, gleich oder verschieden, jeweils einen Rest -NR₁₀R₁₁ darstellen und R₁₆ ein Rest Alkoxy wie tert.-Butoxy ist, oder R₁₅, R₁₆ und R₁₇ gleich sind und jeweils einen Rest -NR₁₀R₁₁ bedeuten, zur Reaktion bringt, das Produkt isoliert und gegebenenfalls in Salz umwandelt.

9. Verfahren zur Herstellung der Verbindungen der Formel (I) nach Anspruch 1, in der R einen Rest C=R₃ darstellt, worin R₃ ein Rest CH-R₇ ist und R₇ einen Rest Alkyl, Phenyl, -alk-Het, -alk-Ar oder einen Heterocyclus bedeutet, dadurch gekennzeichnet, daß man ein Derivat der Formel (II) 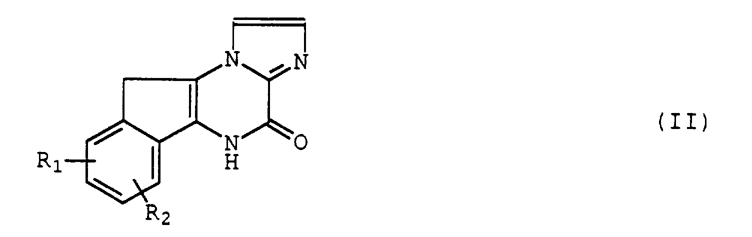 in der R₁ und R₂ die gleichen Bedeutungen wie in Anspruch 1 besitzen, mit einem Aldehyd der Formel (IX)
OHC-R₁₈ (IX)
in der R₁₈ einen Rest Alkyl, Phenyl, -alk-Het, -alk-Ar oder Het darstellt, zur Reaktion bringt, das Produkt isoliert und gegebenenfalls in Salz umwandelt.

10. Verfahren zur Herstellung der Verbindungen der Formel (I) nach Anspruch 1, in der R einen Rest C(R₄)R₅ darstellt, worin R₄ einen Rest Alkyl, -alk-Het oder -alk-Ar bedeutet und R₅ mit R₄ identisch ist, dadurch gekennzeichnet, daß man ein Derivat der Formel (II) 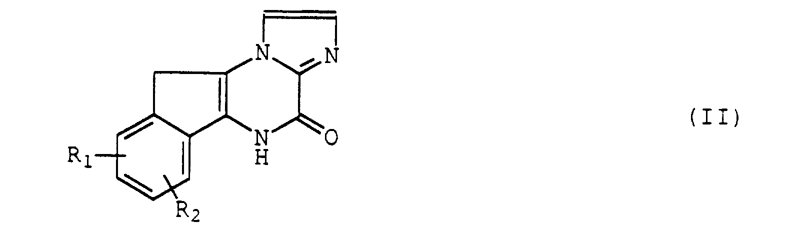 in der R₁ und R₂ die gleichen Bedeutungen wie in Anspruch 1 besitzen, mit einem Halogenid der Formel (X)
Hal-R₁₉ (X)
in der R₁₉ einen Rest Alkyl, -alk-Het oder -alk-Ar darstellt, zur Reaktion bringt, das Produkt isoliert und gegebenenfalls in Salz umwandelt.

11. Verfahren zur Herstellung der Verbindungen der Formel (I) nach Anspruch 1, in der R einen Rest C(R₄)R₅ darstellt, worin R₄ einen Rest Alkyl, -alk-Het oder -alk-Ar bedeutet und R₅ einen Rest Alkyl (1 bis 11 Kohlenstoffatome in gerader oder verzweigter Kette), -alk-Het oder -alk-Ar darstellt, dadurch gekennzeichnet, daß man ein Derivat der Formel (XI) 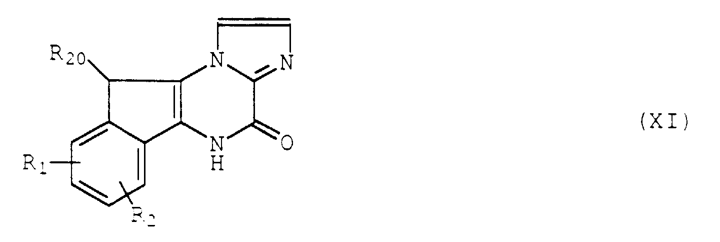 in der R₁ und R₂ die gleichen Bedeutungen wie in Anspruch 1 besitzen und R₂₀ ein Rest Alkyl, -alk-Het oder -alk-Ar ist, oder das silylierte Derivat dieses Derivates mit einem Halogenid der Formel (XII)
Hal-R₂₁ (XII)
in der R₂₁ einen Rest Alkyl (1 bis 11 Kohlenstoffatome in gerader oder verweigter Kette), -alk-Het oder -alk-Ar darstellt, zur Reaktion bringt, das Produkt isoliert und gegebenenfalls in Salz umwandelt.

12. Verfahren zur Herstellung der Verbindungen der Formel (I) nach Anspruch 1, in der R einen Rest C(R₄)R₅ darstellt und R₄ und R₅ zusammen mit dem Kohlenstoffatom, an das sie gebunden sind, einen Rest Cycloalkyl bilden dadurch gekennzeichnet, daß man ein Derivat der Formel (II) 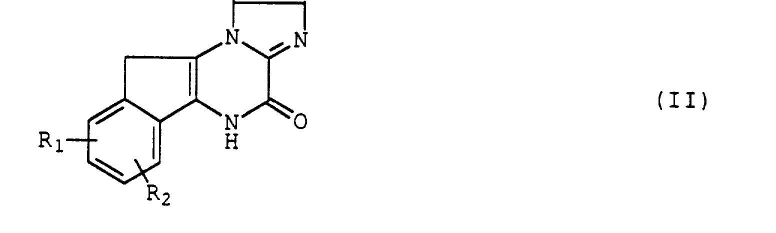 in der R₁ und R₂ die gleichen Bedeutungen wie in Anspruch 1 besitzen, mit einem Derivat der Formel (XIII)
Hal-alk-Hal (XIII)
in der Hal ein Halogenatom ist und alk einen Rest Alkyl (2 bis 5 Kohlenstoffatome) darstellt, zur Reaktion bringt, das Produkt isoliert und gegebenenfalls in Salz umwandelt.

13. Verfahren zur Herstellung der Verbindungen der Formel (I) nach Anspruch 1, in der R einen Rest CH-R₆ darstellt, worin R₆ ein Rest Hydroxy ist, dadurch gekennzeichnet, daß man eine entsprechende Verbindung der Formel (I), in der R einen Rest C=R₃ darstellt und R₃ ein Sauerstoffatom ist, reduziert, das Produkt isoliert und gegebenenfalls in Salz umwandelt.

14. Verfahren zur Herstellung der Verbindungen der Formel (I) nach Anspruch 1, in der R einen Rest CH-R₆ darstellt, worin R₆ einen Rest Alkyl (2 bis 11 Kohlenstoffatome), -alk-Ar, -alk-Het oder 2-Imidazolylmethyl bedeutet, dadurch gekennzeichnet, daß man ein Derivat der Formel (XIV) 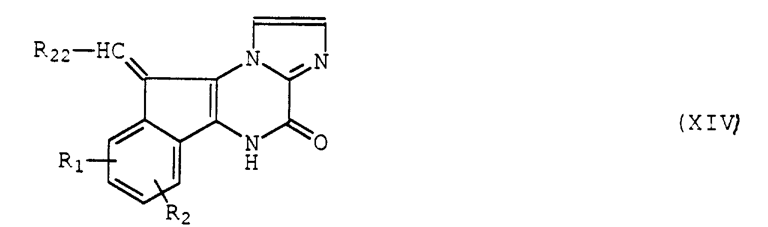 in der R₁ und R₂ die gleichen Bedeutungen wie in Anspruch 1 besitzen, R₂₂ einen Rest 2-Imidazolyl, Alkyl mit 1 bis 10 Kohlenstoffatomen in gerader oder verzweigter Kette, Phenyl, -alk-Ar, -alk-Het, in denen der Teil Alkyl in gerader oder verzweigter Kette vorliegt und 1 bis 3 Kohlenstoffatome enthält, oder einen gesättigten oder ungesättigten, monocyclischen oder polycyclischen Heterocyclus mit 4 bis 9 Kohlenstoffatomen und einem oder mehreren Heteroatomen (O,S,N) bedeutet, hydriert, das Produkt isoliert und gegebenenfalls in Salz umwandelt.

15. Verfahren zur Herstellung der Verbindungen der Formel (I) nach Anspruch 1, in der R einen Rest CH-R₆ darstellt, worin R₆ ein Rest Methyl ist, dadurch gekennzeichnet, daß man eine entsprechende Verbindung der Formel (I), in der R einen Rest C=R₃ darstellt, R₃ ein Rest CH-R₇ ist und R₇ einen Rest Hydroxy oder -NR₁₀R₁₁ bedeutet, reduziert, das Produkt isoliert und gegebenenfalls in Salz umwandelt.

16. Verfahren zur Herstellung der Verbindungen der Formel (I) nach Anspruch 1, in der R einen Rest CH-R₆ darstellt, worin R₆ ein Rest -alk(1C)-OH ist, dadurch gekennzeichnet, daß man eine entsprechende Verbindung der Formel (I), in der R einen Rest C=R₃ darstellt, R₃ ein Rest CH-R₇ ist und R₇ einen Rest Hydroxy bedeutet, reduziert, das Produkt isoliert und gegebenenfalls in Salz umwandelt.

17. Verfahren zur Herstellung der Verbindungen der Formel (I) nach Anspruch 1, in der R einen Rest CH-R₆ darstellt, worin R₆ ein Rest -alk(2-4C)-OH ist, dadurch gekennzeichnet, daß man ein Derivat der Formel (XIV) 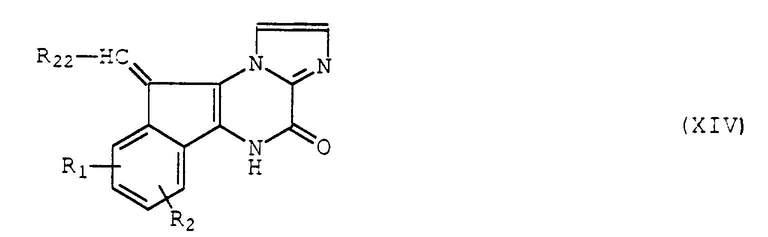 in der R₁ und R₂ die gleichen Bedeutungen wie in Anspruch 1 besitzen und R₂₂ einen Rest -alk(1-3C)-O-CH₂-Ar darstellt, reduziert.

18. Verfahren zur Herstellung der Verbindungen der Formel (I) nach Anspruch 1, in der R einen Rest CH-R₆ darstellt, worin R₆ ein Rest -alk(2-4C)-OH ist, dadurch gekennzeichnet, daß man (COCl)₂ mit einem Derivat der Formel (XI) 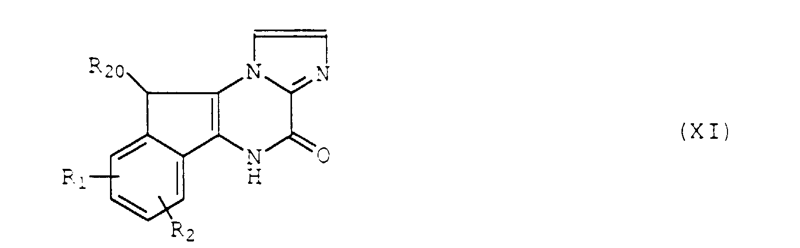 in der R₁ und R₂ die gleichen Bedeutungen wie in Anspruch 1 besitzen und R₂₀ einen Rest -alk-COOH bedeutet, zur Reaktion bringt, eine Reduktion anschließt, das Produkt isoliert und gegebenenfalls in Salz umwandelt.

19. Verfahren zur Herstellung der Verbindungen der Formel (I) nach Anspruch 1, in der R einen Rest CH-R₆ darstellt, worin R₆ ein Rest -NR₈R₉ ist und R₈ und R₉ jeweils ein Wasserstoffatom darstellen, dadurch gekennzeichnet, daß man eine entsprechende Verbindung der Formel (I), in der R ein Rest CH-R₆ ist, R₆ einen Rest -NR₃R₉darstellt, R₃ ein Wasserstoffatom ist, R₉ einen Rest -COR₁₂ bedeutet und R₁₂ ein Rest Alkyl ist, hydrolysiert, das Produkt isoliert und gegebenenfalls in Salz umwandelt.

20. Verfahren zur Herstellung der Verbindungen der Formel (I) nach Anspruch 1, in der R einen Rest CH-R₆ darstellt, worin R₆ ein Rest -NR₈R₉ ist, R₈ und R₉ jeweils ein Wasserstoffatom bedeuten, dadurch gekennzeichnet, daß man eine entsprechende Verbindung der Formel (I), in der R einen Rest C=R₃ darstellt, worin R₃ ein Rest NOH ist, reduziert, das Produkt isoliert und gegebenenfalls in Salz umwandelt.

21. Verfahren zur Herstellung der Verbindungen der Formel (I) nach Anspruch 1, in der R einen Rest CH-R₆ darstellt, worin R₆ einen Rest -NR₈R₉bedeutet, R₈ ein Wasserstoffatom ist, R₉ einen Rest -OOR₁₂ darstellt und R₁₂ ein Rest Alkyl ist, dadurch gekennzeichnet, daß man eine Säure der Formel (XV)
R₂₄-COOH (XV)
in der R₂₄ einen Rest Alkyl (1 bis 3 Kohlenstoffatome) bedeutet, mit einer entsprechenden Verbindung der Formel (I), in der R einen Rest C=R₃ darstellt, worin R₃ ein Rest NOH ist, in Anwesenheit eines Reduktionsmittels zur Reaktion bringt, das Produkt isoliert und gegebenenfalls in Salz umwandelt.

22. Verfahren zur Herstellung der Verbindungen der Formel (I) nach Anspruch 1, in der R einen Rest CH-R₆ darstellt und R₆ einen Rest -NR₈R₉ oder -alk-NR₈R₉ bedeutet, worin R₈ und R₉, gleich oder verschieden, jeweils einen Rest Alkyl darstellen oder R₈ ein Wasserstoffatom ist und R₉ einen Rest Alkyl, -COR₁₂ oder -SO₂R₁₃ bedeutet und R₁₂ ein Rest Alkyl, Cycloalkyl, Phenyl, -COO-alk oder -CH₂-COOX ist, dadurch gekennzeichnet, daß man eine entsprechende Verbindung der Formel (I), in der R₈ und R₉ jeweils ein Wasserstoffatom darstellen oder R₈ ein Wasserstoffatom ist und R₉ einen Rest Alkyl bedeutet, mit einem Halogenid der Formel (XVI)
Hal-R₂₇ (XVI)
in der R₂₇ einen Rest Alkyl, -COR₁₂ oder -SO₂R₁₃ bedeutet und R₁₂ ein Rest Alkyl, Cycloalkyl, Phenyl, -COO-alk oder -CH₂-COOX ist und R₁₃ die gleichen Bedeutungen wie in Anspruch 1 besitzt, zur Reaktion bringt, das Produkt isoliert und gegebenenfalls in Salz umwandelt.

23. Verfahren zur Herstellung der Verbindungen der Formel (I) nach Anspruch 1, in der R einen Rest CH-R₆ darstellt und R₆ einen Rest -NR₈R₉ oder -alk-NR₈R₉ bedeutet, worin R₈ ein Wasserstoffatom ist, R₉ einen Rest -COR₁₂ oder -CSR₃₀ bedeutet und R₁₂ und R₃₀ einen Rest -NH-alk, -NH₂, -NH-Ar oder -NH-Het darstellen, dadurch gekennzeichnet, daß man eine entsprechende Verbindung der Formel (I), in der R₈ und R₉ jeweils ein Wasserstoffatom darstellen, mit einem Derivat R₂₈-N=C=R₃₁, worin R₂₈ einen Rest Trimethylsilyl, Benzoyl, Alkyl, Phenyl oder einen gesättigten oder ungesättigten, monocyclischen oder polycyclischen Heterocyclus mit 4 bis 9 Kohlenstoffatomen und einem oder mehreren Heteroatomen (O,S,N) bedeutet und R₃₁ ein Sauerstoffatom oder Schwefelatom ist, zur Reaktion bringt, gegebenenfalls eine Hydrolyse anschließt, das Produkt isoliert und gegebenenfalls in Salz umwandelt.

24. Verfahren zur Herstellung der Verbindungen der Formel (I) nach Anspruch 1, in der R einen Rest CH-R₆ darstellt und R₆ einen Rest -NR₈R₉ oder -alk-NR₈R₉ bedeutet, worin R₈ ein Wasserstoffatom ist, R₉ einen Rest -OOR₁₂ bedeutet und R₁₂ ein Rest -CH₂-NH₂ ist, dadurch gekennzeichnet, daß man eine entsprechende Verbindung der Formel (I), in der R₈ und R₉ jeweils ein Wasserstoffatom darstellen, mit einer Säure HOOC-CH₂-NH-R₂₉, worin R₂₉ eine Schutzgruppe für die Aminfunktion wie tert.-Butoxycarbonyl ist, zur Reaktion bringt, gegebenenfalls eine Hydrolyse anschließt, das Produkt isoliert und gegebenenfalls in Salz umwandelt.

25. Verfahren zur Herstellung der Verbindungen der Formel (I) nach Anspruch 1, in der R einen Rest CH-R₆ darstellt und R₆ einen Rest -alk-Ar oder -alk-Het bedeutet, dadurch gekennzeichnet, daß man ein Derivat der Formel (II) 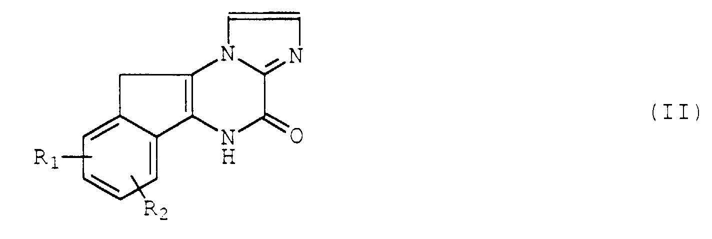 mit einem Halogenid der Formel (XVII)
Hal-R₂₅ (XVII)
in der R₂₅ einen Rest -alk-Ar oder -alk-Het darstellt, zur Reaktion bringt, das Produkt isoliert und gegebenenfalls in Salz umwandelt.

26. Verfahren zur Herstellung der Verbindungen der Formel (I) nach Anspruch 1, in der R einen Rest CH-R₆ darstellt, R₆ ein Rest -alk-NR₈R₉ ist, R₈ und R₉ jeweils ein Wasserstoffatom bedeuten, dadurch gekennzeichnet, daß man Brom und dann Natriumhydroxid mit einem Derivat der Formel (XVIII) 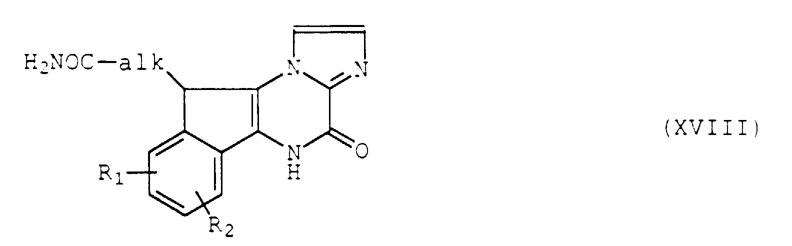 in der R₁ und R₂ die gleichen Bedeutungen wie in Anspruch 1 besitzen und alk ein Rest Alkyl ist, zur Reaktion bringt, das Produkt isoliert und gegebenenfalls in Salz umwandelt.

27. Verfahren zur Herstellung der Verbindungen der Formel (I) nach Anspruch 1, in der R einen Rest CH-R₆ darstellt, R₆ ein Rest -NR₈R₉ oder -alk-NR₈R₉ ist, R₈ ein Wasserstoffatom bedeutet und R₉ einen Rest Alkyl darstellt, dadurch gekennzeichnet, daß man ein Derivat der Formel (XI) 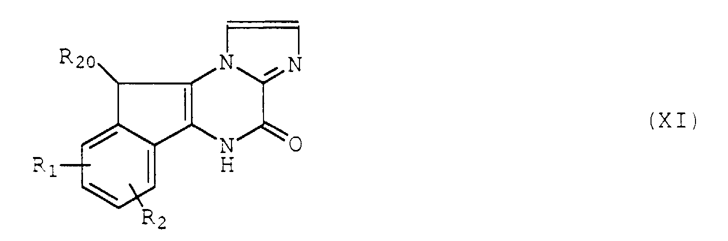 in der R₁ und R₂ die gleichen Bedeutungen wie in Anspruch 1 besitzen und R₂₀ einen Rest -NR₈R₉ oder -alk-NR₈R₉ bedeutet, R₈ ein Wasserstoffatom ist und R₉ einen Rest -CHO oder -COR₁₂ darstellt, worin R₁₂ ein Rest Alkyl oder Cycloalkyl ist, reduziert, das Produkt isoliert und gegebenenfalls in Salz umwandelt.

28. Verfahren zur Herstellung der Verbindungen der Formel (I) nach Anspruch 1, in der entweder R einen Rest C(R₄)R₅ darstellt, worin R₄ und/oder R₅ einen Rest -alk-Het bedeuten, oder R einen Rest CH-R₆ darstellt, R₆ ein Rest -alk-Het ist, worin Het einen Rest Piperidyl bedeutet, dadurch gekennzeichnet, daß man eine entsprechende Verbindung der Formel (I), in der entweder R einen Rest C(R₄)R₅ darstellt, worin R₄ und/oder R₅ einen Rest -alk-Het bedeuten, oder R einen Rest CH-R₆ darstellt, R₆ ein Rest -alk-Het ist, worin Het einen Rest Pyridyl bedeutet, hydriert, das Produkt isoliert und gegebenenfalls in Salz umwandelt.

29. Verfahren zur Herstellung der Verbindungen der Formel (I) nach Anspruch 1, in der R einen Rest C(R₄)R₅ darstellt, worin R₄ ein Rest -alk-Het ist, alk einen Rest Ethyl bedeutet und Het einen Rest 4-Pyridyl oder 2-Pyridyl darstellt, und R₅ einen Rest Alkyl (1 bis 11 Kohlenstoffatome in gerader oder verzweigter Kette), -alk-Het oder -alk-Ar bedeutet, dadurch gekennzeichnet, daß man ein Derivat der Formel (XI) 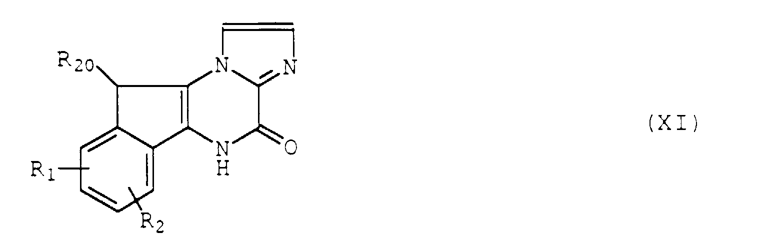 in der R₁ und R₂ die gleichen Bedeutungen wie in Anspruch 1 besitzen und R₂₀ einen Rest Alkyl (1 bis 11 Kohlenstoffatome in gerader oder verzweigter Kette), -alk-Het oder -alk-Ar bedeutet, in seiner silylierten Form mit 2- oder 4-Vinylpyridin zur Reaktion bringt, das Produkt isoliert und gegebenenfalls in Salz umwandelt.

30. Arzneimittel, enthaltend als Wirkstoff mindestens eine Verbindung der Formel (I) nach einem der Ansprüche 1 bis 3.

31. Arzneimittel nach Anspruch 30, die als Antagonisten des Rezeptors AMPA nützlich sind.

32. Arzneimittel nach Anspruch 30, die als Antagonisten des Rezeptors NMDA nützlich sind.

## Claims

1. Compounds of formula: 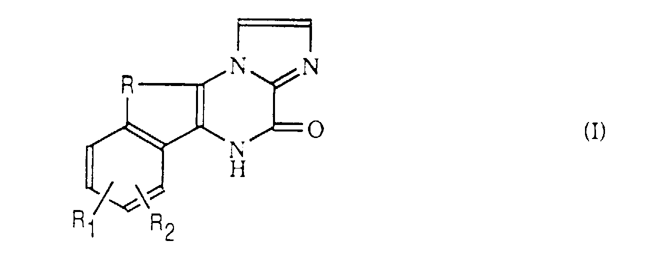 in which
either R represents a radical C=R₃, C(R₄)R₅ or CH-R₆,
- R₁ and R₂, which may be identical or different, represent hydrogen or halogen atoms or alkyl, alkoxy, amino, acylamino, -NH-CO-NH-Ar, -N=CH-N(alk)alk', nitro, cyano, phenyl, imidazolyl or SO₃H radicals,
- R₃ represents an oxygen atom or an NOH, NO-alk-COOX or CH-R₇ radical,
- R₄ represents an alkyl, -alk-Het or -alk-Ar radical,
- R₅ represents an alkyl (1-11C in a straight or branched chain), -alk-Het or -alk-Ar radical,
or alternatively R₄ and R₅ form, together with the carbon atom to which they are attached, a cycloalkyl radical,
- R₆ represents a hydroxyl, alkyl (1-11C in a straight or branched chain), -NR₈R₉, -alk-OH, -alk-NR₈R₉, -alk-Ar or -alk-Het radical,
- R₇ represents a hydroxyl, alkyl, phenyl, -alk-Ar, -alk-Het or -NR₁₀R₁₁ radical or a saturated or unsaturated mono- or polycyclic heterocycle containing 4 to 9 carbon atoms and one or more hetero atoms (O, S, N),
- R₈ and R₉, which may be identical or different, each represent an alkyl radical, or alternatively R₈ represents a hydrogen atom and R₉ represents a hydrogen atom or an alkyl, -COR₁₂, -CSR₃₀ or -SO₂R₁₃ radical,
- R₁₀ and R₁₁, which may be identical or different, each represent an alkyl or cycloalkyl radical,
- R₁₂ represents an alkyl, cycloalkyl, phenyl, -COO-alk, -CH₂-COOX, -CH₂-NH₂, -NH-alk, -NH₂, -NH-Ar or -NH-Het radical,
- R₁₃ represents an alkyl or phenyl radical,
- R₃₀ represents an -NH-alk, -NH-Ar, -NH₂ or -NH-Het radical,
- alk represents an alkyl or alkylene radical,
- alk' represents an alkyl radical,
- X represents a hydrogen atom or an alkyl radical,
- Ar represents a phenyl radical and
- Het represents a saturated or unsaturated mono- or polycyclic heterocycle containing 4 to 9 carbon atoms and one or more hetero atoms (O, S, N);
or R represents a radical CH-R₆, R₆ represents a 2-imidazolylmethyl radical and R₁ and R₂ each represent a hydrogen atom,
it being understood that the alkyl and alkylene radicals and portions contain 1 to 4 carbon atoms in a straight or branched chain, the acyl portions contain 2 to 4 carbon atoms, the cycloalkyl radicals contain 3 to 6 carbon atoms, as well as the isomers of the compounds for which R₃ represents an NOH, NO-alk-COOX or CH-R₇ radical, the racemic mixtures and the enantiomers of the compounds of formula (I) for which R represents a radical C(R₄)R₅ in which R₄ is different from R₅ or CH-R₆, and their salts.

2. Compounds of formula (I) according to claim 1, for which either R represents a radical C=R₃, C(R₄)R₅ or CH-R₆, R₁ and R₂ represent hydrogen or halogen atoms, R₃ represents an oxygen atom or a radical NO-alk-COOX or CH-R₇, R₄ represents an alkyl, or -alk-Ar radical, R₅ represents an alkyl (1-11C in a straight or branched chain), -alk-Het or -alk-Ar radical or alternatively R₄ and R₅ form, together with the carbon atom to which they are attached, a cycloalkyl radical, R₆ represents a hydroxyl, alkyl (1-11C in a straight or branched chain), -NR₈R₉, -alk-NR₈R₉, -alk-OH, -alk-Ar or -alk-Het radical, R₇ represents a hydroxyl or -NR₁₀R₁₁ radical or a saturated or unsaturated mono- or polycyclic heterocycle containing 4 to 9 carbon atoms and one or more hetero atoms (O, S, N), R₈ represents a hydrogen atom and R₉ represents a hydrogen atom or an alkyl, -COR₁₂ or -SO₂R₁₃ radical, R₁₀ and R₁₁, which may be identical or different, each represent an alkyl radical, R₁₂ represents an alkyl, -NH-Ar, -NH-alk or phenyl radical, R₁₃ represents an alkyl or phenyl radical, alk represents an alkyl or alkylene radical, X represents an alkyl radical, and Het represents a saturated or unsaturated mono- or polycyclic heterocycle containing 4 to 9 carbon atoms and one or more hetero atoms (O, S, N) or alternatively R represents a radical CH-R₆ and R₆ represents a 2-imidazolylmethyl radical and R₁ and R₂ are hydrogen atoms, as well as the isomers of the compounds for which R₃ represents an NO-alk-COOX or CH-R₇ radical, the racemic mixtures and the enantiomers of the compounds of formula (I) for which R represents a radical C(R₄)R₅ in which R₄ is different from R₅ or CH-R₆, and their salts.

3. Compounds of formula (I) according to claim 1, chosen from the following compounds:
- 10-hydroxy-5H,10H-imidazo[1,2-a]indeno[1,2-e]pyrazin-4-one,
- 10-acetamido-5H,10H-imidazo[1,2-a]indeno[1,2-e]pyrazin-4-one,
- 10-amino-5H,10H-imidazo[1,2-a]indeno[1,2-e]pyrazin-4-one,
- 10-(E-dimethylaminomethylene)-5H,10H-imidazo[1,2-a]-indeno[1,2-e]pyrazin-4-one,
- 10-hydroxymethylene-5H,10H-imidazo[1,2-a]indeno-[1,2-e]pyrazin-4-one,
- 10,10-dimethyl-5H,10H-imidazo[1,2-a]indeno[1,2-e]-pyrazin-4-one,
- spiro[5H,10H-imidazo[1,2-a]indeno[1,2-e]pyrazine-10:1'-cyclopropane]-4-one,
- spiro[5H,10H-imidazo[1,2-a]indeno[1,2-e]pyrazine-10:1'-cyclopentane]-4-one,
- 10-methyl-5H,10H-imidazo[1,2-a]indeno[1,2-e]pyrazin-4-one,
- 10,10-dibenzyl-5H,10H-imidazo[1,2-a]indeno[1,2-e]pyrazin-4-one,
- 10-hydroxymethyl-5H,10H-imidazo[1,2-a]indeno[1,2-e]-pyrazin-4-one,
- 10-(2-furylmethyl)-5H,10H-imidazo[1,2-a]indeno-[1,2-e]pyrazin-4-one,
- 10-(4-pyridylmethylene)-5H,10H-imidazo[1,2-a]indeno-[1,2-e]pyrazin-4-one,
- 10-(4-pyridylmethyl)-5H,10H-imidazo[1,2-a]indeno-[1,2-e]pyrazin-4-one,
- 10-(phenylpropyl)-5H,10H-imidazo[1,2-a]indeno-[1,2-e]pyrazin-4-one,
- 10-(3-pyridylmethylene)-5H,10H-imidazo[1,2-a]indeno-[1,2-e]pyrazin-4-one,
- 10-(3-pyridylmethyl)-5H,10H-imidazo[1,2-a]indeno-[1,2-e]pyrazin-4-one,
- 10-(2-pyridylmethyl)-5H,10H-imidazo[1,2-a]indeno-[1,2-e]pyrazin-4-one,
- 10-(2-imidazoly1methyl)-5H,10H-imidazo[1,2-a]indeno-[1,2-e]pyrazin-4-one,
- tert-butyl (4-oxo-5H,10H-imidazo[1,2-a]indeno-[1,2-e]pyrazine-10-ylidene)aminooxyacetate
- 10-isobutyl-5H,10H-imidazo[1,2-a]indeno[1,2-e]pyrazin-4-one,
- (4-oxo-5H,10H-imidazo[1,2-a]indeno[1,2-e]pyrazine-10-ylidene)aminooxyacetic acid,
- 10-propionamido-5H,10H-imidazo[1,2-a]indeno[1,2-e]-pyrazin-4-one,
- 10-amino-8-fluoro-5H,10H-imidazo[1,2-a]indeno-[1,2-e]pyrazin-4-one,
- 10-(4-quinolylmethylene)-5H,10H-imidazo[1,2-a]indeno[1,2-e]pyrazin-4-one,
- 10-(4-quino1y1methyl)-5H,10H-imidazo[1,2-a]indeno-[1,2-e]pyrazin-4-one,
- 10-(3-phenylureido)-5H,10H-imidazo[1,2-a]indeno-[1,2-e]pyrazin-4-one,
- 10-(3-methylureido)-5H,10H-imidazo[1,2-a]indeno-[1,2-e]pyrazin-4-one,
- 10-isobutyramido-5H,10H-imidazo[1,2-a]indeno[1,2-e]-pyrazin-4-one,
- 10-amino-7-chloro-5H,10H-imidazo[1,2-a]indeno-[1,2-e]pyrazin-4-one,
- 10-benzenesulphonylamido-5H,10H-imidazo[1,2-a]indeno[1,2-e]pyrazin-4-one,
- 10-methylamino-5H,10H-imidazo[1,2-a]indeno[1,2-e]-pyrazin-4-one,
- 10-(2-pyrazinylmethylene)-5H,10H-imidazo[1,2-a]-indeno[1,2-e]pyrazin-4-one,
- 10-(2-pyrazinylmethyl)-5H,10H-imidazo[1,2-a]indeno-[1,2-e]pyrazin-4-one,
- 10-benzyl-7-chloro-5H,10H-imidazo[1,2-a]indeno-[1,2-e]pyrazin-4-one,
- 7-chloro-10-(3-phenylureido)-5H,10H-imidazo[1,2-a]-indeno[1,2-e]pyrazin-4-one,
- 10-(2-hydroxyethyl)-5H,10H-imidazo[1,2-a]indeno-[1,2-e]pyrazin-4-one,
- 7-chloro-10-methylamino-5H,10H-imidazo[1,2-a]indeno-[1,2-e]pyrazin-4-one,
- 10-benzyl-10-methyl-5H,10H-imidazo[1,2-a]indeno-[1,2-e]pyrazin-4-one,
- 10-methyl-10-(4-pyridylmethyl)-5H,10H-imidazo-[1,2-a]indeno[1,2-e]pyrazin-4-one,
- 10-(4-piperidylmethyl)-5H,10H-imidazo[1,2-a]indeno-[1,2-e]pyrazin-4-one,
- 5H,10H-imidazo[1,2-a]indeno[1,2-e]pyrazine-4,10-dione,
- 10-benzyl-5H,10H-imidazo[1,2-a]indeno[1,2-e]pyrazin-4-one,
- 10-hexyl-5H,10H-imidazo[1,2-a]indeno[1,2-e]pyrazin-4-one,
- 10-benzamido-5H,10H-imidazo[1,2-a]indeno[1,2-e]-pyrazin-4-one,
- 10-methyl-10-[2-(4-pyridyl)ethyl]-5H,10H-imidazo-[1,2-a]indeno[1,2-e]pyrazin-4-one and their salts.

4. Process for the preparation of the compounds of formula (I) according to claim 1 for which R represents a radical C=R₃ in which R₃ represents an oxygen atom, characterized in that a corresponding compound of formula (I) for which R represents a radical C=R₃ and R₃ represents a radical NOH is hydrolysed, the product is isolated and is optionally converted to a salt.

5. Process for the preparation of the compounds of formula (I) according to claim 1 for which R represents a radical C=R₃ and R₃ represents a radical NOH, characterized in that an alkyl nitrite is reacted with a derivative of formula: 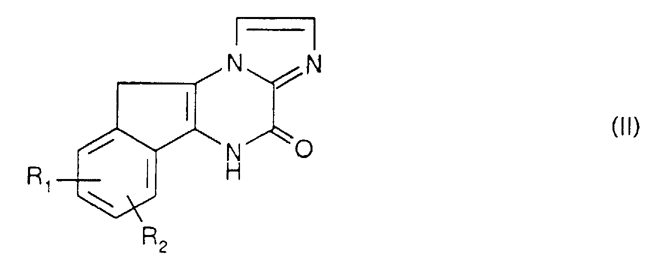 in which R₁ and R₂ have the same meanings as in claim 1, the product is isolated and is optionally converted to a salt.

6. Process for the preparation of the compounds of formula (I) according to claim 1 for which R represents a radical C=R₃ and R₃ represents a radical NO-alk-COOX, characterized in that a corresponding compound of formula (I) for which R represents a radical C=R₃ and R₃ represents a radical NOH is reacted with a halide Hal-alk-COOX for which Hal represents a halogen atom, alk represents an alkyl radical and X represents an alkyl radical, optionally followed by freeing of the carboxyl function by the action of trifluoroacetic acid, the product is isolated and is optionally converted to a salt.

7. Process for the preparation of the compounds of formula (I) according to claim 1 for which R represents a radical C=R₃ and R₃ represents a radical CH-R₇, in which R₇ represents a hydroxyl radical, characterized in that a corresponding compound of formula (I) for which R₇ represents a radical -NR₁₀R₁₁ is hydrolysed, the product is isolated and is optionally converted to a salt.

8. Process for the preparation of the compounds of formula (I) according to claim 1 for which R represents a radical C=R₃, R₃ represents a radical CH-R₇ and R₇ represents a radical -NR₁₀R₁₁, characterized in that a derivative of formula: 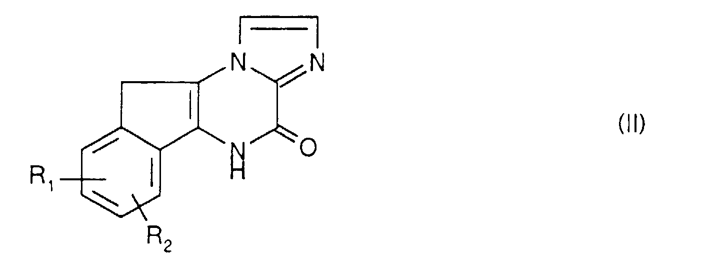 in which R₁ and R₂ have the same meanings as in claim 1 is reacted with a derivative of formula: 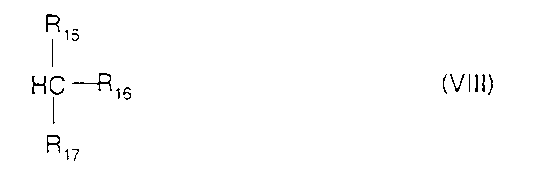 in which either R₁₅ and R₁₇, which may be identical or different, each represent a radical -NR₁₀R₁₁ and R₁₆ represents an alkoxy radical such as tert-butoxy, or R₁₅, R₁₆ and R₁₇, which are identical, each represent a radical -NR₁₀R₁₁, the product is isolated and is optionally converted to a salt.

9. Process for the preparation of the compounds of formula (I) according to claim 1 for which R represents a radical C=R₃, R₃ represents a radical CH-R₇ and R₇ represents an alkyl, phenyl, -alk-Het or -alk-Ar radical or a heterocycle, characterized in that a derivative of formula: 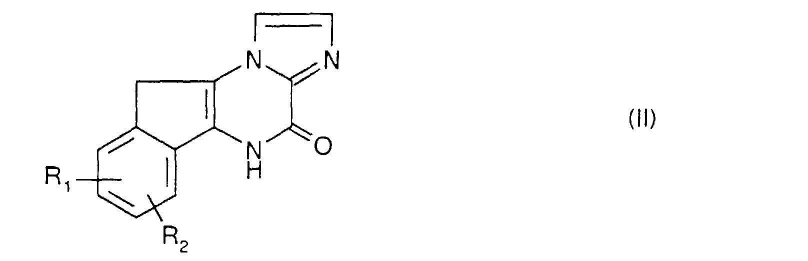 in which R₁ and R₂ have the same meanings as in claim 1 is reacted with an aldehyde of formula:
OHC-R₁₈ (IX)
in which R₁₈ represents an alkyl, phenyl, -alk-Het, -alk-Ar or Het radical, the product is isolated and is optionally converted to a salt.

10. Process for the preparation of the compounds of formula (I) according to claim 1 for which R represents a radical C(R₄)R₅, R₄ represents an alkyl, -alk-Het or -alk-Ar radical and R₅ is identical to R₄, characterized in that a derivative of formula: 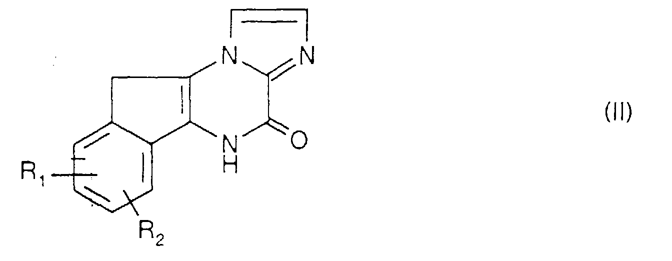 in which R₁ and R₂ have the same meanings as in claim 1 is reacted with a halide of formula:
Hal-R₁₉ (X)
in which R₁₉ represents an alkyl, -alk-Het or -alk-Ar radical, the product is isolated and is optionally converted to a salt.

11. Process for the preparation of the compounds of formula (I) according to claim 1 for which R represents a radical C(R₄)R₅, R₄ represents an alkyl, -alk-Het or -alk-Ar radical and R₅ represents an alkyl (1-11C in a straight or branched chain), -alk-Het or -alk-Ar radical, characterized in that a derivative of formula: 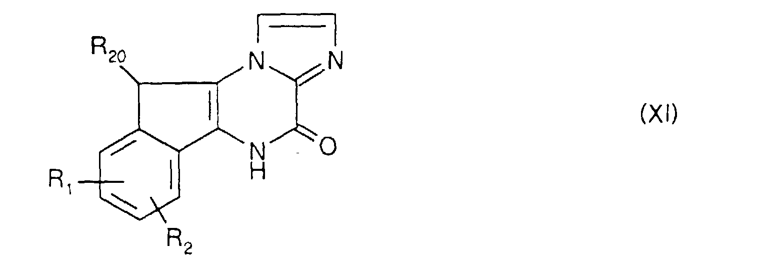 in which R₁ and R₂ have the same meanings as in claim 1 and R₂₀ represents an alkyl, -alk-Het or -alk-Ar radical or the silyl derivative of this derivative is reacted with a halide of formula:
Hal-R₂₁ (XII)
in which R₂₁ represents an alkyl (1-11C in a straight or branched chain), -alk-Het or -alk-Ar radical, the product is isolated and is optionally converted to a salt.

12. Process for the preparation of the compounds of formula (I) according to claim 1 for which R represents a radical C(R₄)R₅ and R₄ and R₅ form, together with the carbon atom to which they are attached, a cycloalkyl radical, characterized in that a derivative of formula: 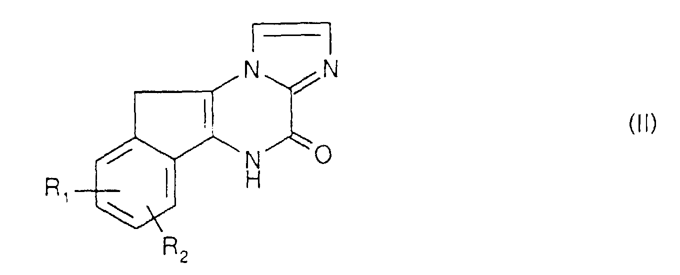 in which R₁ and R₂ have the same meanings as in claim 1 is reacted with a derivative of formula:
Hal-alk-Hal (XIII)
in which Hal represents a halogen atom and alk represents a (2-5C) alkyl radical, the product is isolated and is optionally converted to a salt.

13. Process for the preparation of the compounds of formula (I) according to claim 1 for whcih R represents a radical CH-R₆, in which R₆ represents a hydroxyl radical, characterized in that a corresponding compound of formula (I) for which R represents a radical C=R₃ and R₃ represents an oxygen atom is reduced, the product is isolated and is optionally converted to a salt.

14. Process for the preparation of the compounds of formula (I) according to claim 1 for which R represents a radical CH-R₆, in which R₆ represents a (2-11C) alkyl, -alk-Ar, -alk-Het or 2-imidazolylmethyl radical, characterized in that a derivative of formula: 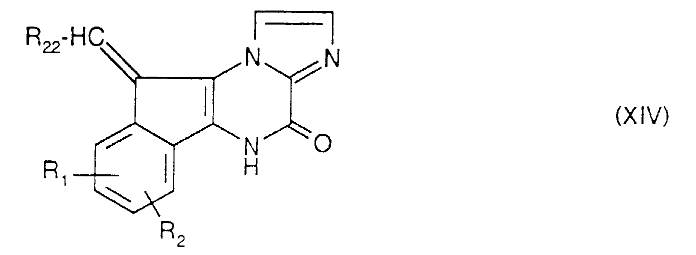 in which R₁ and R₂ have the same meanings as in claim 1, R₂₂ represents a 2-imidazolyl, alkyl in a straight or branched chain containing 1 to 10 carbon atoms, phenyl, -alk-Ar, -alk-Het in which the alkyl portion is in a straight or branched chain and contains 1 to 3 carbon atoms or a saturated or unsaturated mono- or polycyclic heterocycle containing 4 to 9 carbon atoms and one or more hetero atoms (O, S, N) is hydrogenated, the product is isolated and is optionally converted to a salt.

15. Process for the preparation of the compounds of formula (I) according to claim 1 for which R represents a radical CH-R₆, in which R₆ represents a methyl radical, characterized in that a corresponding compound of formula (I) for which R represents a radical C=R₃, R₃ represents a radical CH-R₇ and R₇ represents a hydroxyl or -NR₁₀R₁₁ radical is reduced, the product is isolated and is optionally converted to a salt.

16. Process for the preparation of the compounds of formula (I) according to claim 1 for which R represents a radical CH-R₆ and R₆ represents an -alk(lC)-OH radical, characterized in that a corresponding compound of formula (I) for which R represents a radical C=R₃, R₃ represents a radical CH-R₇ and R₇ represents a hydroxyl radical is reduced, the product is isolated and is optionally converted to a salt.

17. Process for the preparation of the compounds of formula (I) according to claim 1 for which R represents a radical CH-R₆ and R₆ represents an -alk(2-4C)-OH radical, characterized in that a derivative of formula: 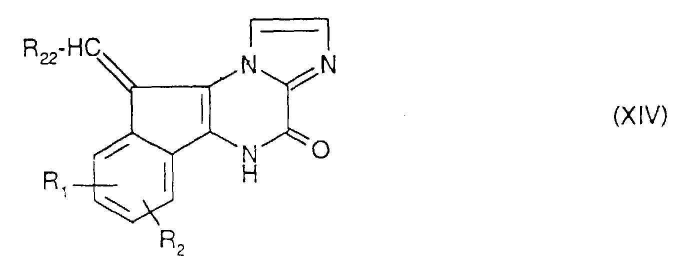 for which R₁ and R₂ have the same meanings as in claim 1 and R₂₂ represents a radical -alk(1-3C)-O-CH₂-Ar is reduced.

18. Process for the preparation of the compounds of formula (I) according to claim 1 for which R represents a radical CH-R₆ and R₆ represents an -alk(2-4C)-OH radical, characterized in that (COCl)₂ is reacted with a derivative of formula: 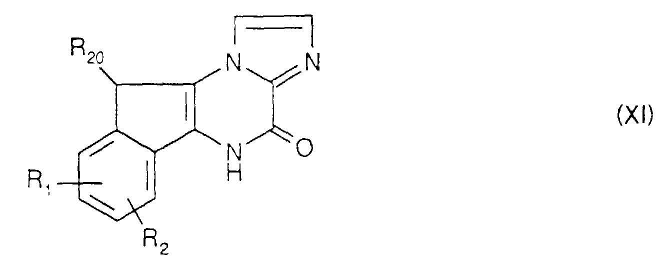 for which R₁ and R₂ have the same meanings as in claim 1 and R₂₀ represents an -alk-COOH radical, followed by a reduction, the product is isolated and is optionally converted to a salt.

19. Process for the preparation of the compounds of formula (I) according to claim 1 for which R represents a radical CH-R₆, in which R₆ represents a radical -NR₈R₉, R₈ and R₉ each representing a hydrogen atom, characterized in that a corresponding compound of formula (I) for which R represents a radical CH-R₆, in which R₆ represents a radical -NR₈R₉, R₈ represents a hydrogen atom and R₉ represents a radical -COR₁₂ and R₁₂ represents an alkyl radical, is hydrolysed, the product is isolated and is optionally converted to a salt.

20. Process for the preparation of the compounds of formula (I) according to claim 1 for which R represents a radical CH-R₆, in which R₆ represents a radical -NR₈R₉, R₈ and R₉ each representing a hydrogen atom, characterized in that a corresponding compound of formula (I) for which R represents a radical C=R₃ and R₃ represents a radical NOH is reduced, the product is isolated and is optionally converted to a salt.

21. Process for the preparation of the compounds of formula (I) according to claim 1 for which R represents a radical CH-R₆, in which R₆ represents a radical -NR₈R₉, R₈ represents a hydrogen atom and R₉ represents a radical -COR₁₂ and R₁₂ represents an alkyl radical, characterized in that an acid of formula:
R₂₄-COOH (XV)
in which R₂₄ represents a (1-3C) alkyl is reacted with a corresponding compound of formula (I) for which R represents a radical C=R₃ and R₃ represents a radical NOH, in the presence of a reducing agent, the product is isolated and is optionally converted to a salt.

22. Process for the preparation of the compounds of formula (I) according to claim 1 for which R represents a radical CH-R₆ and R₆ represents a radical -NR₈R₉ or -alk-NR₈R₉, in which R₈ and R₉, which may be identical or different, each represent an alkyl radical or alternatively R₈ represents a hydrogen atom and R₉ represents an alkyl, -COR₁₂ or -SO₂R₁₃ radical and R₁₂ represents an alkyl, cycloalkyl, phenyl, -COO-alk or -CH₂-COOX radical, characterized in that a corresponding compound of formula (I), for which R₈ and R₉ each represent a hydrogen atom or alternatively R₈ represents a hydrogen atom and R₉ represents an alkyl radical, is reacted with a halide of formula:
Hal-R₂₇ (XVI)
in which R₂₇ represents an alkyl, -COR₁₂ or -SO₂R₁₃ radical and R₁₂ represents an alkyl, cycloalkyl, phenyl, -COO-alk or -CH₂-COOX radical and R₁₃ has the same meanings as in claim 1, the product is isolated and is optionally converted to a salt.

23. Process for the preparation of the compounds of formula (I) according to claim 1 for which R represents a radical CH-R₆ and R₆ represents a radical -NR₈R₉ or -alk-NR₈R₉, in which R₈ represents a hydrogen atom, R₉ represents a radical -COR₁₂ or -CSR₃₀ and R₁₂ and R₃₀ represent a radical -NH-alk, -NH₂, -NH-Ar or -NH-Het, characterized in that a corresponding compound of formula (I), for which R₈ and R₉ each represent a hydrogen atom, is reacted with a derivative R₂₈-N=C=R₃₁, for which R₂₈ represents a trimethylsilyl, benzoyl, alkyl, phenyl or a saturated or unsaturated mono- or polycyclic heterocycle containing 4 to 9 carbon atoms and one or more hetero atoms (O, S, N) and R₃₁ represents an oxygen or sulphur atom, optionally followed by a hydrolysis, the product is isolated and is optionally converted to a salt.

24. Process for the preparation of the compounds of formula (I) according to claim 1 for which R represents a radical CH-R₆ and R₆ represents a radical -NR₈R₉ or -alk-NR₈R₉, in which R₈ represents a hydrogen atom, R₉ represents a radical -COR₁₂ and R₁₂ represents a -CH₂-NH₂ radical, characterized in that a corresponding compound of formula (I), for which R₉ and R₉ each represent a hydrogen atom, is reacted with an acid HOOC-CH₂-NH-R₂₉, in which R₂₉ represents a protecting group for the amine function such as tert-butoxycarbonyl, followed by a hydrolysis, the product is isolated and is optionally converted to a salt.

25. Process for the preparation of the compounds of formula (I) according to claim 1 for which R represents a radical CH-R₆, in which R₆ represents a radical -alk-Ar or -alk-Het, characterized in that a derivative of formula: 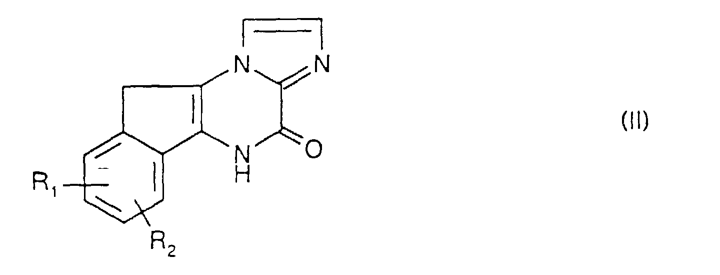 is reacted with a halide of formula:
Hal-R₂₅ (XVII)
in which R₂₅ represents a radical -alk-Ar or -alk-Het, the product is isolated and is optionally converted to a salt.

26. Process for the preparation of the compounds of formula (I) according to claim 1, for which R represents a radical CH-R₆, R₆ represents a radical -alk-NR₈R₉, R₈ and R₉ each representing a hydrogen atom, characterized in that bromine and sodium hydroxide are reacted with a derivative of formula: 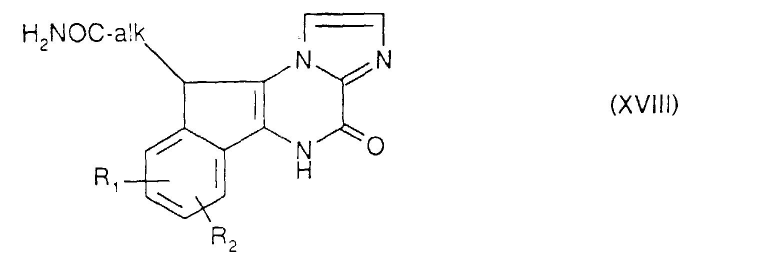 in which R₁ and R₂ have the same meanings as in claim 1 and alk represents an alkyl radical, the product is isolated and is optionally converted to a salt.

27. Process for the preparation of the compounds of formula (I) according to claim 1 for which R represents a radical CH-R₆, R₆ represents a radical -NR₈R₉ or -alk-NR₈R₉, R₈ represents a hydrogen atom and R₉ represents an alkyl radical, characterized in that a derivative of formula: 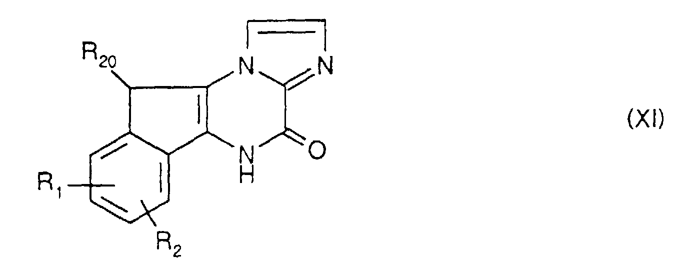 for which R₁ and R₂ have the same meanings as in claim 1 and R₂₀ represents a radical -NR₈R₉ or -alk-NR₈R₉, R₈ represents a hydrogen atom and R₉ represents a radical -CHO or -COR₁₂ in which R₁₂ represents an alkyl or cycloalkyl radical is reduced, the product is isolated and is optionally converted to a salt.

28. Process for the preparation of the compounds of formula (I) according to claim 1 for which either R represents a radical C(R₄)R₅, in which R₄ and/or R₅ represents a radical -alk-Het, or R represents a radical CH-R₆, R₆ represents a radical -alk-Het, in which Het is a piperidyl radical, characterized in that a corresponding compound of formula (I) for which either R represents a radical C(R₄)R₅, in which R₄ and/or R₅ represents a radical -alk-Het, or R represents a radical CH-R₆, R₆ represents a radical -alk-Het, in which Het represents a pyridyl radical, is hydrogenated, the product is isolated and is optionally converted to a salt.

29. Process for the preparation of the compounds of formula (I) according to claim 1 for which R represents a radical C(R₄)R₅, R₄ represents a radical -alk-Het, in which alk is an ethyl radical and Het is a 4-pyridyl or 2-pyridyl radical and R₅ represents an alkyl (1-11C in a straight or branched chain), -alk-Het or -alk-Ar radical, characterized in that a derivative of formula: 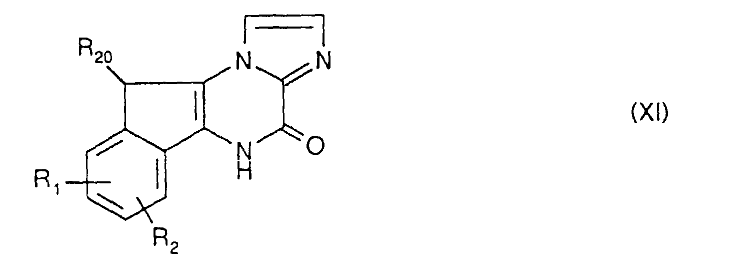 in which R₁ and R₂ have the same meanings as in claim 1 and R₂₀ represents an alkyl (1-11C in a straight or branched chain), -alk-Het or -alk-Ar radical in its silyl form, is reacted with 2- or 4-vinylpyridine, the product is isolated and is optionally converted to a salt.

30. Medicaments containing at least one compound of formula (I) according to one of claims 1 to 3 as active principle.

31. Medicaments according to claim 30, which are useful as antagonists of the AMPA receptor.

32. Medicaments according to claim 30, which are useful as antagonists of the NMDA receptor.
